# EUROPÄISCHE PATENTSCHRIFT

(11) **EP 2 303 814 B1**
(45) Veröffentlichungstag und Bekanntmachung des Hinweises auf die Patenterteilung: **12.12.2018**
(21) Anmeldenummer: 09776634.9
(22) Anmeldetag: 20.05.2009
(51) Int. Cl.: C07C 13/62, C09K 11/02, C09K 11/06, H01L 51/00

(54) **VERBINDUNGEN FÜR ELEKTRONISCHE VORRICHTUNGEN**
COMPOUNDS FOR ELECTRONIC DEVICES
COMPOSÉS POUR DISPOSITIFS ÉLECTRONIQUES

(30) Priorität: 29.07.2008 DE 102008035413
(43) Veröffentlichungstag der Anmeldung: 06.04.2011
(73) Patentinhaber: Merck Patent GmbH, 64293 Darmstadt (DE)
(72) Erfinder: BUESING, Arne, 65929 Frankfurt am Main (DE); HEIL, Holger, 60389 Frankfurt (DE); STOESSEL, Philipp, 60487 Frankfurt am Main (DE)
(86) Internationale Anmeldenummer: PCT/EP2009/003602
(87) Internationale Veröffentlichungsnummer: WO 2010/012328

(56) Entgegenhaltungen:
- WO-A-2004/041901
- WO-A-2007/140847
- SETAYESH S ET AL: "POLYFLUORENES WITH POLYPHENYLENE DENDRON SIDE CHAINS: TOWARD NON-AGGREGATING, LIGHT-EMITTING POLYMERS" JOURNAL OF THE AMERICAN CHEMICAL SOCIETY, AMERICAN CHEMICAL SOCIETY, WASHINGTON, DC. US, Bd. 123, Nr. 5, 1. Januar 2001 (2001-01-01), Seiten 946-953, XP001077861 ISSN: 0002-7863

## Beschreibung

Die vorliegende Erfindung beschreibt neue organische Verbindungen und deren Einsatz in elektronischen Vorrichtungen.

Der allgemeine Aufbau organischer Elektrolumineszenzvorrichtungen ist beispielsweise in US 4539507, US 5151629, EP 0676461 und WO 98/27136 beschrieben. Allerdings zeigen diese Vorrichtungen immer noch Probleme, die denen noch Verbesserungsbedarf besteht:
1. Bei der Effizienz gibt es gerade bei fluoreszierenden OLEDs immer noch Verbesserungsbedarf. Dies gilt insbesondere für tiefblau emittierende OLEDs.
2. Bei der operativen Lebensdauer ist insbesondere bei blauer Emission noch eine weitere Verbesserung wünschenswert.
3. Die Betriebsspannung ist gerade bei fluoreszierenden OLEDs recht hoch. Hier besteht daher noch Verbesserungsbedarf, um die Leistungseffizienz zu verbessern. Das ist insbesondere für mobile Anwendungen von großer Bedeutung.
4. Viele blau emittierende Materialien gemäß dem Stand der Technik sind nicht kompatibel zu häufig verwendeten Elektroneninjektions- und -transportmaterialien, wie beispielsweise Hydroxychinolinat-Metallkomplexen (z. B. Alq, Beq), Benzimidazolderivaten, Phenanthrolinderivaten (z. B. BCP) oder Anthracenderivaten, die mit Donatoren, wie Alkali- oder Erdalkalimetallen (z. B. Li, Na, K, Rb oder Cs), mit deren anorganischen Salzen (z. B. LiF oder Cs₂CO₃) oder mit deren organischen Salzen (z. B. Lithium-, Natrium-, Kalium-, Rubidium- oder Caesiumchinolinat) gemischt werden und so einen Elektronenüberschuss im Device produzieren. Diese Inkompatibilität führt nur zu ungenügenden Devicelebensdauern. Insbesondere treten die Probleme häufig auf, wenn als blau emittierendes Material ein Diarylaminoderivat eines kondensierten Aromaten verwendet wird. Derartige Emitter sind jedoch die häufigsten und bislang besten blauen Emitter. Hier sind daher weitere Verbesserungen wünschenswert.

Blau fluoreszierende Polymere auf Basis von Polyfluorenen sind aus dem Stand der Technik bekannt, z. B in Setayesh et al., J. Am. Chem. Soc. 2001, 123, 946-953.

Der nächstliegende Stand der Technik für blau fluoreszierende Emitter sind Dibenzoindenofluorenderivate gemäß WO 07/140847 und Monobenzoindenofluorenderivate gemäß WO 08/006449. Um aus diesen Grundstrukturen effiziente blaue Emitter zu erhalten, ist die Einführung von ein oder zwei Diarylaminogruppen erforderlich. Mit diesen Verbindungen werden bereits gute blau emitterende OLEDs erzielt. Jedoch sind auch hier noch weitere Verbesserungen in Bezug auf die Effizienz wünschenswert. Während diese Diarylamino-substituierten Verbindungen in Kombination mit einer undotierten Elektronentransportschicht auch sehr gute Lebensdauern zeigen, ist die Lebensdauer noch nicht ausreichend, wenn diese Verbindungen in Kombination mit einer dotierten Elektronentransportschicht, wie oben beschrieben, verwendet werden. Daher sind auch weitere Verbesserungen in Bezug auf die Lebensdauer erforderlich, insbesondere in Kombination mit dotierten Elektronentransportschichten, welche zu einem Elektronenüberschuss im Device führen.

Es wurde überraschend gefunden, dass Verbindungen, in denen drei Aryl- oder Heteroarylgruppen mit zwei Indenobrücken bzw. entsprechenden Heterobrücken überbrückt sind, insbesondere dann besonders gute Eigenschaften als blaue Emitter zeigen, wenn die Summe der π-Elektronen der drei aromatischen bzw. heteroaromatischen Gruppen mindestens 28 beträgt. Es ist nicht erforderlich, in diese Verbindungen Diarylaminosubstituenten einzuführen, da bereits die unsubstituierten Verbindungen hocheffiziente tiefblaue Emission zeigen. Weiterhin führen die Verbindungen in organischen Elektrolumineszenzvorrichtungen zu sehr guten Lebensdauern. Diese Verbindungen und deren Verwendung in organischen Elektrolumineszenzvorrichtungen sind daher der Gegenstand der vorliegenden Erfindung.

Gegenstand der Erfindung sind daher Verbindungen gemäß Formel (1), 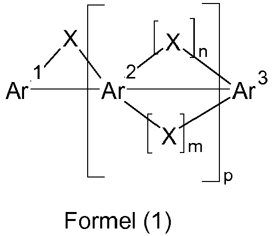 wobei für die verwendeten Symbole und Indizes gilt:
Ar¹, Ar², Ar³ ist bei jedem Auftreten gleich oder verschieden eine Aryl- oder Heteroarylgruppe mit 5 bis 22 aromatischen Ringatomen, ausgewählt aus Benzol, Naphthalin, Anthracen, Phenanthren, Fluoranthen, Naphthacen, Benzanthracen, Chrysen, Pyren, Benzfluoranthen, Triphenylen, Perylen, Dibenzanthracen, Benzpyren, Picen, Pentacen, Pentaphen, Pyridin, Pyrazin, Pyrimidin, Pyridazin, Chinolin, Isochinolin, Phenanthrolin und Acridin, die mit einem oder mehreren Resten R¹ substituiert sein kann, mit der Maßgabe, dass Ar² nicht für Anthracen, Naphthacen oder Pentacen steht;
X ist bei jedem Auftreten gleich oder verschieden eine Gruppe, ausgewählt aus C(R²)₂;
R¹ ist bei jedem Auftreten gleich oder verschieden eine Gruppe, ausgewählt aus H, D, F, geradkettigen Alkyl- oder Alkoxygruppen mit 1 bis 10 C-Atomen oder verzweigten oder cyclischen Alkyl- oder Alkoxygruppen mit 3 bis 10 C-Atomen, die jeweils mit einem oder mehreren Resten R³ substituiert sein kann, wobei jeweils eine oder mehrere nicht benachbarte CH₂-Gruppen durch R³C=CR³ oder O ersetzt sein können und wobei ein oder mehrere H-Atome durch F ersetzt sein können, oder aromatischen oder heteroaromatischen Ringsystemen mit 5 bis 24 aromatischen Ringatomen; dabei können zwei oder mehrere Substituenten R¹ auch miteinander ein mono- oder polycyclisches, aliphatisches oder aromatisches Ringsystem bilden;
R² ist bei jedem Auftreten gleich oder verschieden eine Gruppe, ausgewählt aus H, geradkettigen Alkylgruppen mit 1 bis 10 C-Atomen oder verzweigten oder cyclischen Alkylgruppen mit 3 bis 10 C-Atomen, wobei jeweils eine oder mehrere nicht benachbarte CH2-Gruppen durch -R²C=CR²- oder -O- ersetzt sein können und wobei ein oder mehrere H-Atome durch F ersetzt sein können, oder einer monovalenten Aryl- oder Heteroarylgruppe mit 5 bis 16 aromatischen Ringatomen, die mit einem oder mehreren nichtaromatischen Resten R² substituiert sein kann; dabei können auch zwei Reste R², die in derselben Gruppe X gebunden sind, miteinander ein Ringsystem bilden;
R³ ist bei jedem Auftreten gleich oder verschieden H oder ein aliphatischer oder aromatischer Kohlenwasserstoffrest mit 1 bis 20 C-Atomen;
m, n sind 0 oder 1, mit der Maßgabe, dass m + n = 1 ist;
p ist 1;
dabei bilden Ar¹, Ar² und X zusammen einen Fünfring oder einen Sechsring und Ar², Ar³ und X bilden zusammen einen Fünfring oder einen Sechsring, mit der Maßgabe, dass entweder alle Symbole X in der Verbindung gemäß Formel (1) in einem Fünfring gebunden sind oder dass alle Symbole X in der Verbindung gemäß Formel (1) in einem Sechsring gebunden sind;
dadurch gekennzeichnet, dass die Summe aller π-Elektronen der Gruppen Ar¹, Ar² und Ar³ mindestens 28 beträgt;
dabei sind die folgenden Verbindungen von der Erfindung ausgenommen:

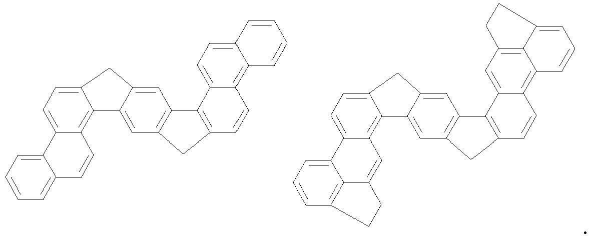

Dabei bedeutet n = 0 bzw. m = 0, dass die entsprechende Gruppe X nicht vorhanden ist und dass statt dessen an die entsprechenden Positionen von Ar² und Ar³ Wasserstoff bzw. ein Substituent R¹ gebunden ist.

Die Summe aller π-Elektronen der Gruppen Ar¹, Ar² und Ar³ zu ermitteln, ist für den Fachmann offensichtlich. So steht in einer Arylgruppe jede Doppelbindung (wobei es sich um delokalisierte Doppelbindungen handelt) für zwei π-Elektronen, so dass beispielsweise Benzol 6 π-Elektronen, Naphthalin 10 π-Elektronen, Anthracen und Phenanthren 14 π-Elektronen, Pyren 16 π-Elektronen, Naphthacen, Benzanthracen und Chrysen 18 π-Elektronen und Perylen 20 π-Elektronen aufweisen. In einer Arylgruppe stimmt die Zahl der π-Elektronen mit der Zahl der C-Atome im aromatischen Ringsystem überein. Auch in Heteroaromaten trägt jede Doppelbindung (auch hier handelt es sich um delokalisierte Doppelbindungen) zwei π-Elektronen bei, wobei diese delokalisierten Doppelbindungen entweder zwischen zwei Kohlenstoffatomen, zwischen Kohlenstoff und Stickstoff oder zwischen zwei Stickstoffatomen gebildet werden können. Weiterhin trägt in Fünfring-Heteroarylgruppen jeweils das Heteroatom, das formal nicht in einer Doppelbindung gebunden ist (also beispielsweise der Stickstoff im Pyrrol, der Sauerstoff im Furan oder der Schwefel im Thiophen) ebenfalls über das freie Elektronenpaar zwei π-Elektronen zum gesamten π-Elektronensystem bei. Daher weisen beispielsweise Pyridin, Pyrazin, Pyrimidin und Pyridazin jeweils 6 π-Elektronen, Chinolin und Isochinolin 10 π-Elektronen, Phenanthrolin 14 π-Elektronen, Pyrrol, Imidazol, Pyrazol, Thiophen, Thiazol und Furan jeweils 6 π-Elektronen, Indol, Benzimidazol, Benzothiophen und Benzofuran jeweils 10 π-Elektronen und Carbazol, Dibenzothiophen und Dibenzofuran jeweils 14 π-Elektronen auf.

Im Folgenden ist am Beispiel von Phenyl und Naphthalin als Gruppen Ar¹ und Ar² aufgezeigt, was unter der Bildung eines Fünfrings bzw. eines Sechsrings aus den Gruppen Ar¹, Ar² und X verstanden wird:

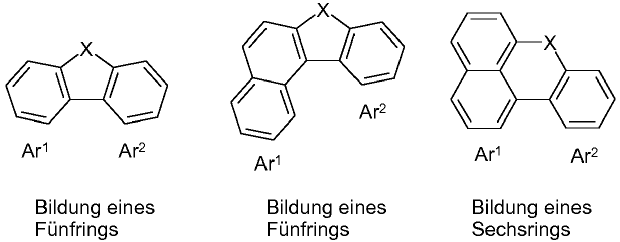

Mit einer einfachen, nicht-kondensierten Aryl- oder Heteroarylgruppe, beispielsweise mit Phenyl, ist immer nur die Bildung eines Fünfrings möglich. Mit einer kondensierten Aryl- oder Heteroarylgruppe, beispielsweise mit Naphthalin, ist je nach Verknüpfung die Bildung eines Fünfrings oder eines Sechsrings möglich. Dasselbe Verknüpfungsprinzip kann entsprechend auf andere kondensierte Arylgruppen oder auf kondensierte oder nicht-kondensierte Heteroarylgruppen angewandt werden. In einem Fünfring bildet also jeweils eine Kante der Aryl- oder Heteroarylgruppe Ar¹ bzw. Ar² bzw. Ar³ mit X einen Fünfring. In einem Sechsring bilden zwei Kanten einer kondensierten Aryl- oder Heteroarylgruppe Ar¹ bzw. Ar² bzw. Ar³ zusammen mit einer Kante einer weiteren Aryl- oder Heteroarylgruppe Ar¹ bzw. Ar² bzw. Ar³ und zusammen mit X einen Sechsring.

In einer bevorzugten Ausführungsform der Erfindung bilden Ar¹, Ar² und X einen Fünfring und Ar², Ar³ und X bilden einen Fünfring.

Unter einer Arylgruppe bzw. einer Heteroarylgruppe im Sinne dieser Erfindung wird eine aromatische Gruppe bzw. heteroaromatische Gruppe mit einem gemeinsamen aromatischen Elektronensystem verstanden, wobei eine Arylgruppe 6 bis 30 C-Atome und eine Heteroarylgruppe 2 bis 30 C-Atome und insgesamt mindestens 5 aromatische Ringatome umfasst. Die Heteroatome sind bevorzugt ausgewählt aus N, O und/oder S. Dies kann im Sinne dieser Erfindung ein einfacher Homo- oder Heterocyclus sein, beispielsweise Benzol, Pyridin, Thiophen, etc., oder es kann eine kondensierte Aryl- oder Heteroarylgruppe sein, in der mindestens zwei aromatische oder heteroaromatische Ringe, beispielsweise Benzolringe, miteinander "verschmolzen", d. h. durch Anellierung einander ankondensiert sind, also mindestens eine gemeinsame Kante und dadurch auch ein gemeinsames aromatisches System aufweisen. Diese Aryl- oder Heteroarylgruppe kann substituiert oder unsubstituiert sein; ebenso können gegebenenfalls vorhandene Substituenten weitere Ringsysteme bilden. So sind beispielsweise Systeme wie Naphthalin, Anthracen, Phenanthren, Pyren, etc. als Arylgruppen und Chinolin, Acridin, Benzothiophen, Carbazol, etc. als Heteroarylgruppen im Sinne dieser Erfindung zu sehen, während beispielsweise Biphenyl, Fluoren, Spirobifluoren, etc. keine Arylgruppen darstellen, da es sich hierbei um separate aromatische Elektronensysteme handelt.

Ein aromatisches Ringsystem im Sinne dieser Erfindung enthält 6 bis 60 C-Atome im Ringsystem. Ein heteroaromatisches Ringsystem im Sinne dieser Erfindung enthält 2 bis 60 C-Atome und mindestens ein Heteroatom im Ringsystem, mit der Maßgabe, dass die Summe der C-Atome und der Heteroatome mindestens 5 ergibt. Die Heteroatome sind bevorzugt ausgewählt aus N, O und/oder S. Unter einem aromatischen oder heteroaromatischen Ringsystem im Sinne dieser Erfindung soll ein System verstanden werden, das nicht notwendigerweise nur Aryl- oder Heteroarylgruppen enthält, sondern in dem auch mehrere Aryl- oder Heteroarylgruppen durch eine kurze, nicht-aromatische Einheit (weniger als 10 % der von H verschiedenen Atome, bevorzugt weniger als 5 % der von H verschiedenen Atome), wie z. B. ein C-, N- oder O-Atom, unterbrochen sein können. So sollen beispielsweise auch Systeme wie 9,9'-Spirobifluoren, 9,9-Diarylfluoren, Triarylamin, Diarylether, etc. als aromatische Ringsysteme im Sinne dieser Erfindung verstanden werden.

Im Rahmen der vorliegenden Erfindung werden unter einer C₁- bis C₄₀-Alkylgruppe, in der auch einzelne H-Atome oder CH₂-Gruppen durch die oben genannten Gruppen substituiert sein können, besonders bevorzugt die Reste Methyl, Ethyl, n-Propyl, i-Propyl, n-Butyl, i-Butyl, s-Butyl, t-Butyl, 2-Methylbutyl, n-Pentyl, s-Pentyl, tert-Pentyl, 2-Pentyl, Cyclopentyl, n-Hexyl, s-Hexyl, tert-Hexyl, 2-Hexyl, 3-Hexyl, Cyclohexyl, 2-Methylpentyl, n-Heptyl, 2-Heptyl, 3-Heptyl, 4-Heptyl, Cycloheptyl, 1-Methylcyclohexyl, n-Octyl, 2-Ethylhexyl, Cyclooctyl, 1-Bicyclo[2,2,2]octyl, 2-Bicyclo[2,2,2]-octyl, 2-(2,6-Dimethyl)octyl, 3-(3,7-Dimethyl)octyl, Trifluormethyl, Pentafluorethyl, 2,2,2-Trifluorethyl, Ethenyl, Propenyl, Butenyl, Pentenyl, Cyclopentenyl, Hexenyl, Cyclohexenyl, Heptenyl, Cycloheptenyl, Octenyl, Cyclooctenyl, Ethinyl, Propinyl, Butinyl, Pentinyl, Hexinyl, Heptinyl oder Octinyl verstanden. Unter einer C₁- bis C₄₀-Alkoxygruppe werden besonders bevorzugt Methoxy, Ethoxy, n-Propoxy, i-Propoxy, n-Butoxy, i-Butoxy, s-Butoxy, t-Butoxy oder 2-Methylbutoxy verstanden. Unter einer C₂-C₂₄ Aryl- oder Heteroarylgruppe, die je nach Verwendung monovalent oder bivalent sein kann, die noch jeweils mit den oben genannten Resten R¹ substituiert sein kann und die über beliebige Positionen am Aromaten bzw. Heteroaromaten verknüpft sein kann, werden insbesondere Gruppen verstanden, welche abgeleitet sind von Benzol, Naphthalin, Anthracen, Phenanthren, Pyren, Dihydropyren, Chrysen, Perylen, Fluoranthen, Benzanthracen, Benzphenanthren, Benzfluoranthen, Tetracen, Pentacen, Benzpyren, Furan, Benzofuran, Isobenzofuran, Dibenzofuran, Thiophen, Benzothiophen, Isobenzothiophen, Dibenzothiophen, Pyrrol, Indol, Isoindol, Carbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, Pyrazin, Phenazin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol. Unter aromatischen und heteroaromatischen Ringsystemen im Sinne dieser Erfindung werden außer den oben genannten Aryl- und Heteroarylgruppen insbesondere Biphenylen, Terphenylen, Fluoren, Benzofluoren, Dibenzofluoren, Spirobifluoren, Dihydrophenanthren, Tetrahydropyren, cis- oder trans-Indenofluoren, cis- oder trans-Monobenzoindenofluoren oder cis- oder trans-Dibenzoindenofluoren verstanden.

In einer bevorzugten Ausführungsform der Erfindung beträgt die Summe aller π-Elektronen der Gruppen Ar¹, Ar² und Ar³ zwischen 28 und 50, besonders bevorzugt zwischen 28 und 46, ganz besonders bevorzugt zwischen 28 und 42, insbesondere zwischen 28 und 36.

Bevorzugt stehen die Symbole Ar¹, Ar² und Ar³ bei jedem Auftreten gleich oder verschieden für eine Arylgruppe mit 6 bis 18 aromatischen Ringatomen, insbesondere ausgewählt aus Benzol, Naphthalin, Anthracen, Phenanthren, Fluoranthen, Naphthacen, Benzanthracen, Chrysen, Pyren, Benzfluoranthen und Triphenylen.

Besonders bevorzugte Gruppen Ar¹ und Ar³, welche mit Ar² einen Fünfring bilden, sind die im Folgenden aufgeführten Gruppen der Formeln (2) bis (85), welche jeweils durch einen oder mehrere Reste R¹ substituiert sein können. Dabei steht das Symbol * für die Position der Verknüpfung von Ar¹ bzw. Ar³ mit Ar² und das Symbol # steht für die Position der Verknüpfung von Ar¹ bzw. Ar³ mit X.

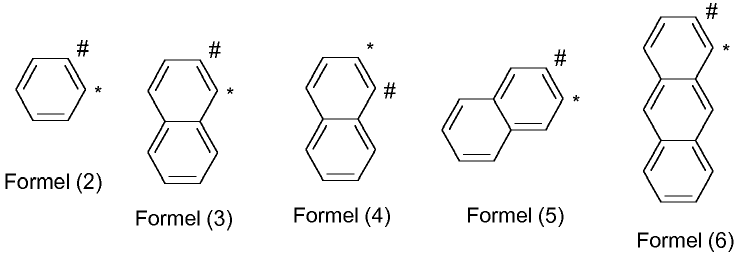

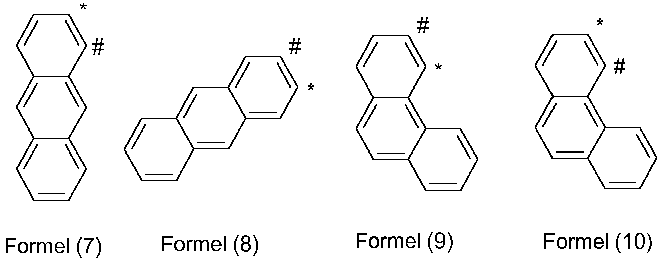

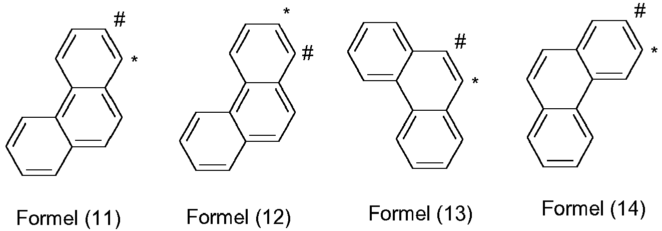

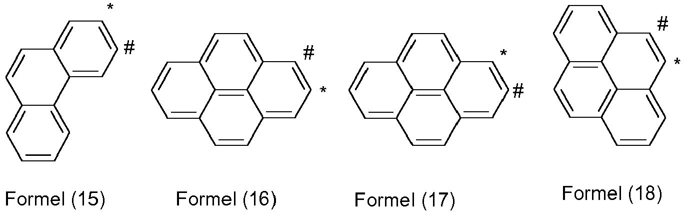

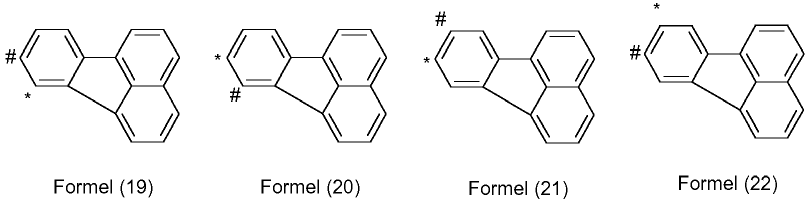

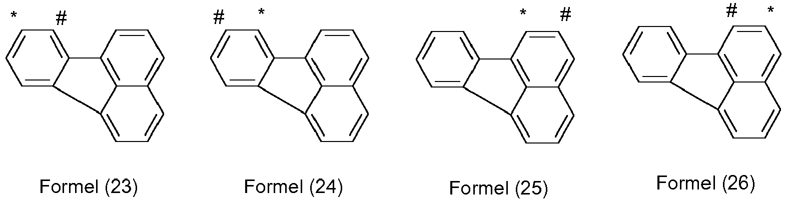

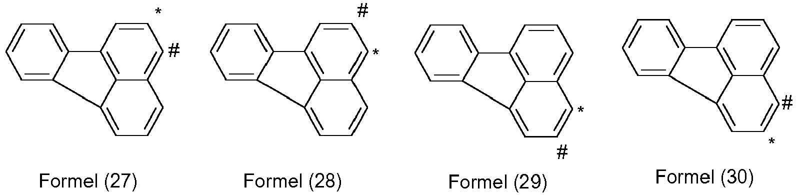

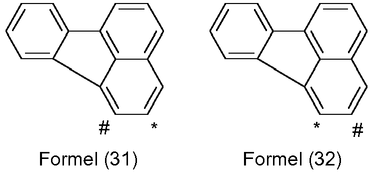

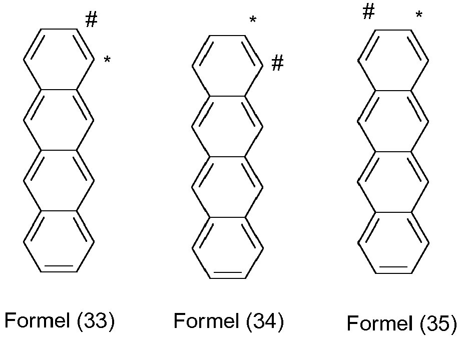

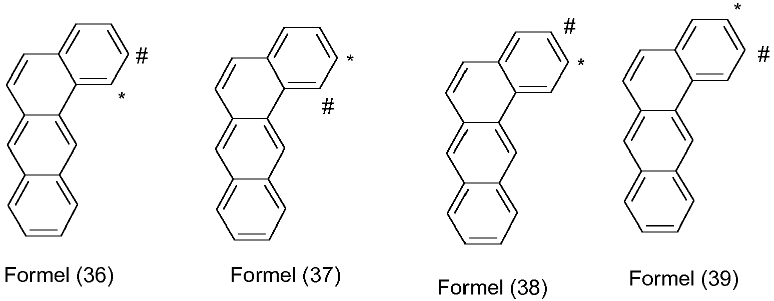

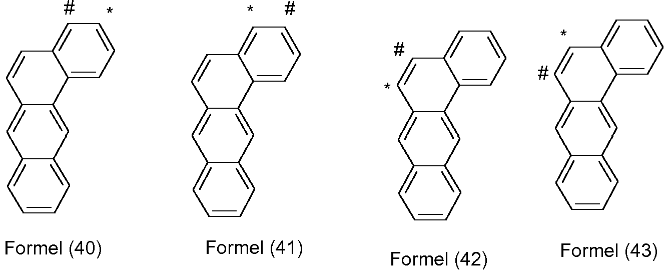

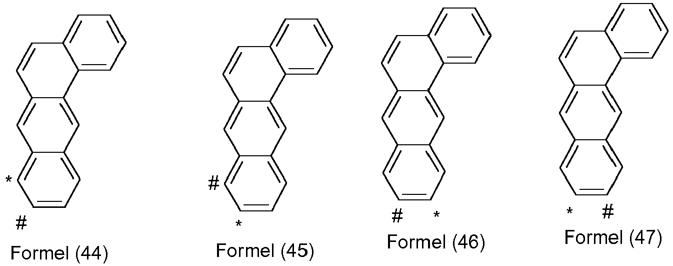

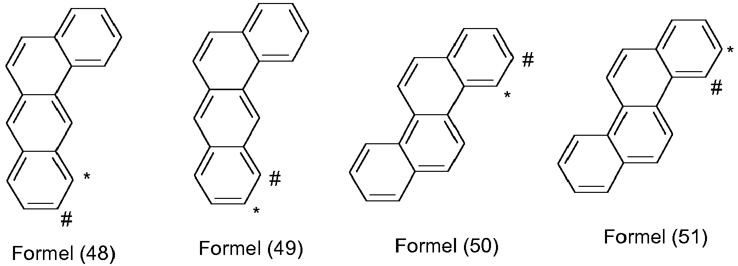

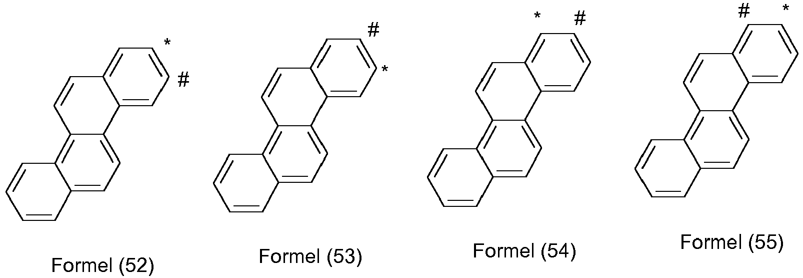

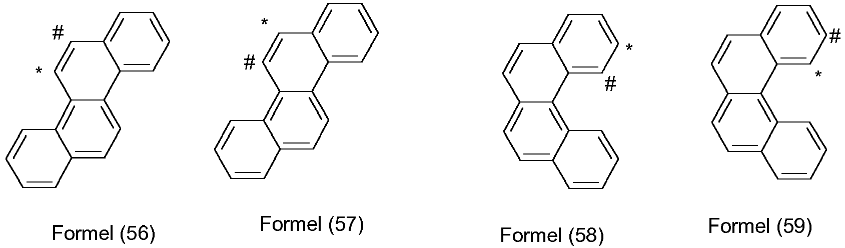

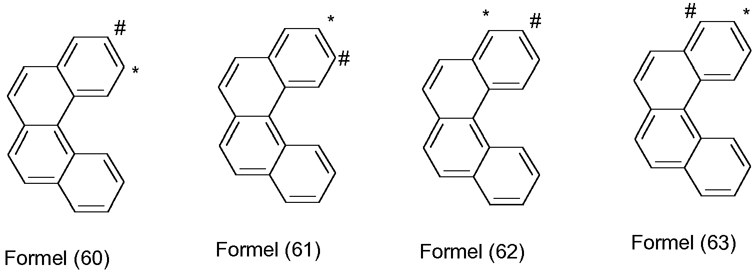

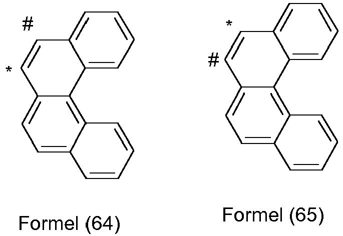

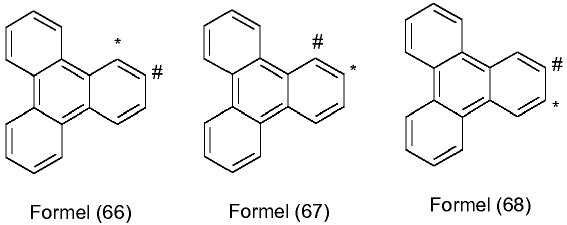

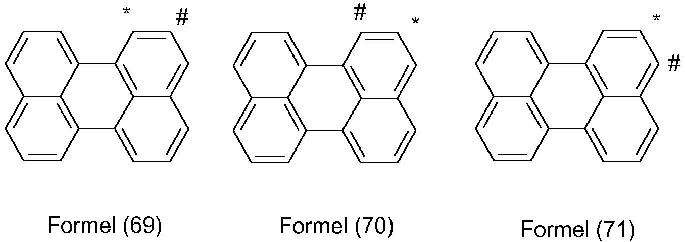

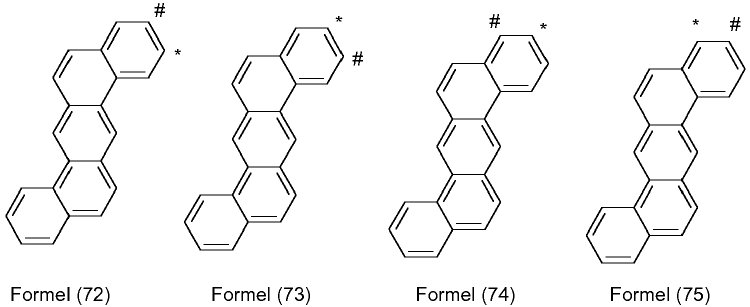

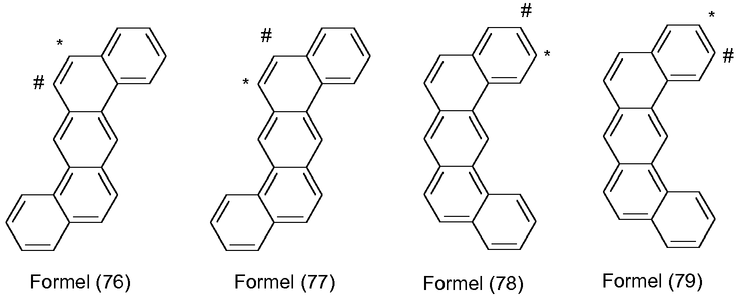

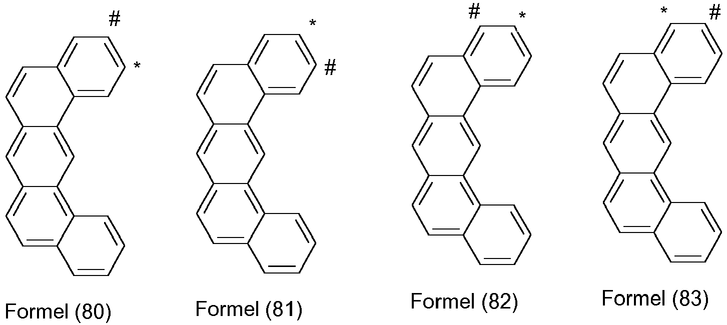

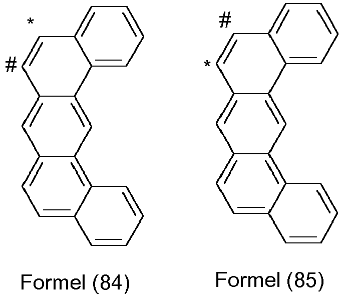

Ebenso bevorzugt sind die oben aufgeführten Gruppen Ar¹ und Ar³, welche mit Ar² einen Sechsring bilden. Die Bildung eines Sechsrings erfolgt über zwei peri-ständige Gruppen, wie im Folgenden exemplarisch am Beispiel einer Anthracengruppe abgebildet:

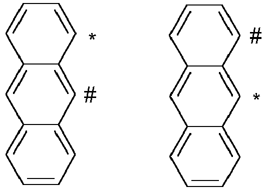

Besonders bevorzugte Gruppen Ar² sind die im Folgenden aufgeführten Gruppen der Formeln (86) bis (106), welche jeweils durch einen oder mehrere Reste R¹ substituiert sein können. Dabei steht das Symbol * für die Position der Verknüpfung von Ar² mit Ar¹ bzw. Ar³ und das Symbol # steht für die Position der Verknüpfung von Ar² mit X.

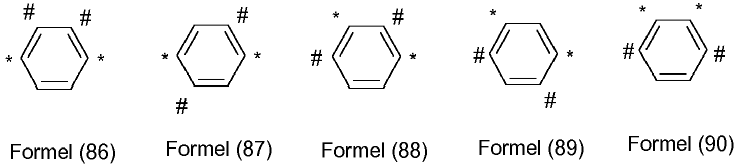

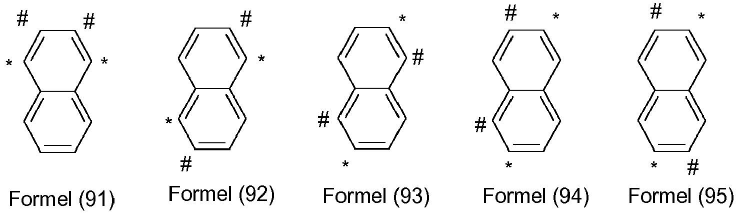

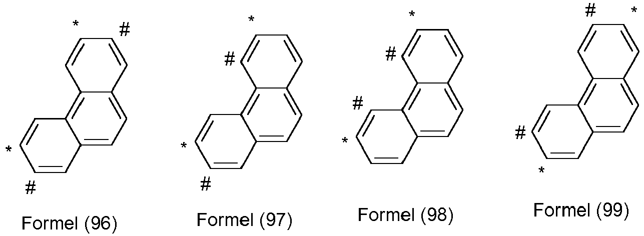

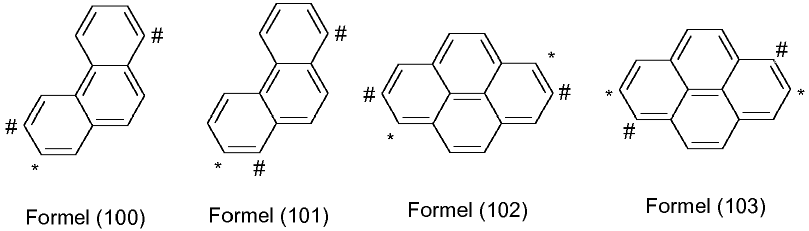

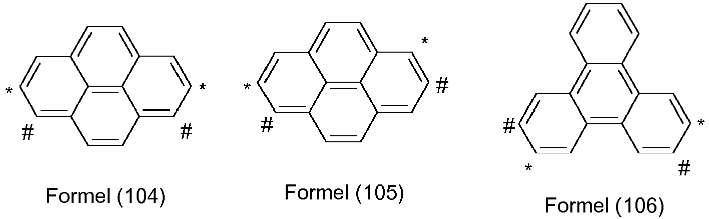

Ganz analog sind auch hier Gruppen Ar² möglich, welche mit Ar¹ bzw. Ar³ und X einen Sechsring bilden.
Bevorzugt sind weiterhin Verbindungen, in denen mindestens eine der Gruppen Ar¹, Ar² bzw. Ar³ mindestens 3 kondensierte Ringe, also mindestens 14 π-Elektronen aufweist. Besonders bevorzugt weist mindestens eine der Gruppen Ar¹, Ar² bzw. Ar³ mindestens 4 kondensierte Ringe, also mindestens 16 π-Elektronen auf. Ganz besonders bevorzugt weist mindestens eine der Gruppen Ar¹, Ar² bzw. Ar³ mindestens 4 kondensierte Ringe auf, also mindestens 16 π-Elektronen, und mindestens eine der anderen beiden Gruppen Ar¹, Ar² bzw. Ar³ weist mindestens 2 kondensierte Ringe, also mindestens 10 π-Elektronen, auf. Bevorzugte Kombinationen aus Ar¹, Ar² und Ar³ sind die in Tabelle 1 und Tabelle 2 aufgeführten Kombinationen. Dabei können Ar¹, Ar² und Ar³ auch durch einen oder mehrere Reste R¹ substituiert sein.

**Tabelle 1**

| Ar1 | Ar2 | Ar3 |
|---|---|---|
| Benzol | Benzol | Pyren |
| Benzol | Benzol | Naphthacen |
| Benzol | Benzol | Benzanthracen |
| Benzol | Benzol | Chrysen |
| Benzol | Benzol | Benzphenanthren |
| Benzol | Benzol | Fluoranthen |
| Benzol | Benzol | Triphenylen |
| Benzol | Naphthalin | Anthracen |
| Benzol | Naphthalin | Phenanthren |
| Benzol | Naphthalin | Pyren |
| Benzol | Naphthalin | Naphthacen |
| Benzol | Naphthalin | Benzanthracen |
| Benzol | Naphthalin | Chrysen |
| Benzol | Naphthalin | Benzphenanthren |
| Benzol | Naphthalin | Fluoranthen |
| Benzol | Naphthalin | Triphenylen |
| Naphthalin | Benzol | Anthracen |
| Naphthalin | Benzol | Phenanthren |
| Naphthalin | Benzol | Pyren |
| Naphthalin | Benzol | Naphthacen |
| Naphthalin | Benzol | Benzanthracen |
| Naphthalin | Benzol | Chrysen |
| Naphthalin | Benzol | Benzphenanthren |
| Naphthalin | Benzol | Fluoranthen |
| Naphthalin | Benzol | Triphenylen |
| Naphthalin | Naphthalin | Naphthalin |
| Naphthalin | Naphthalin | Anthracen |
| Naphthalin | Naphthalin | Phenanthren |
| Naphthalin | Naphthalin | Pyren |
| Naphthalin | Naphthalin | Naphthacen |
| Naphthalin | Naphthalin | Benzanthracen |
| Naphthalin | Naphthalin | Chrysen |
| Naphthalin | Naphthalin | Benzphenanthren |
| Naphthalin | Naphthalin | Fluoranthen |
| Naphthalin | Naphthalin | Triphenylen |
| Anthracen | Benzol | Anthracen |
| Anthracen | Benzol | Phenanthren |
| Anthracen | Benzol | Pyren |
| Anthracen | Benzol | Naphthacen |
| Anthracen | Benzol | Benzanthracen |
| Anthracen | Benzol | Chrysen |
| Anthracen | Benzol | Benzphenanthren |
| Anthracen | Benzol | Fluoranthen |
| Anthracen | Benzol | Triphenylen |
| Anthracen | Naphthalin | Anthracen |
| Anthracen | Naphthalin | Phenanthren |
| Anthracen | Naphthalin | Pyren |
| Anthracen | Naphthalin | Naphthacen |
| Anthracen | Naphthalin | Benzanthracen |
| Anthracen | Naphthalin | Chrysen |
| Anthracen | Naphthalin | Benzphenanthren |
| Anthracen | Naphthalin | Fluoranthen |
| Anthracen | Naphthalin | Triphenylen |
| Phenanthren | Benzol | Phenanthren |
| Phenanthren | Benzol | Pyren |
| Phenanthren | Benzol | Naphthacen |
| Phenanthren | Benzol | Benzanthracen |
| Phenanthren | Benzol | Chrysen |
| Phenanthren | Benzol | Benzphenanthren |
| Phenanthren | Benzol | Fluoranthen |
| Phenanthren | Benzol | Triphenylen |
| Phenanthren | Naphthalin | Phenanthren |
| Phenanthren | Naphthalin | Pyren |
| Phenanthren | Naphthalin | Naphthacen |
| Phenanthren | Naphthalin | Benzanthracen |
| Phenanthren | Naphthalin | Chrysen |
| Phenanthren | Naphthalin | Benzphenanthren |
| Phenanthren | Naphthalin | Fluoranthen |
| Phenanthren | Naphthalin | Triphenylen |
| Pyren | Benzol | Pyren |
| Pyren | Benzol | Naphthacen |
| Pyren | Benzol | Benzanthracen |
| Pyren | Benzol | Chrysen |
| Pyren | Benzol | Benzphenanthren |
| Pyren | Benzol | Fluoranthen |
| Pyren | Benzol | Triphenylen |
| Pyren | Naphthalin | Pyren |
| Pyren | Naphthalin | Naphthacen |
| Pyren | Naphthalin | Benzanthracen |
| Pyren | Naphthalin | Chrysen |
| Pyren | Naphthalin | Benzphenanthren |
| Pyren | Naphthalin | Fluoranthen |
| Pyren | Naphthalin | Triphenylen |
| Naphthacen | Benzol | Naphthacen |
| Naphthacen | Benzol | Benzanthracen |
| Naphthacen | Benzol | Chrysen |
| Naphthacen | Benzol | Benzphenanthren |
| Naphthacen | Benzol | Fluoranthen |
| Naphthacen | Benzol | Triphenylen |
| Naphthacen | Naphthalin | Naphthacen |
| Naphthacen | Naphthalin | Benzanthracen |
| Naphthacen | Naphthalin | Chrysen |
| Naphthacen | Naphthalin | Benzphenanthren |
| Naphthacen | Naphthalin | Fluoranthen |
| Naphthacen | Naphthalin | Triphenylen |
| Benzanthracen | Benzol | Benzanthracen |
| Benzanthracen | Benzol | Chrysen |
| Benzanthracen | Benzol | Benzphenanthren |
| Benzanthracen | Benzol | Fluoranthen |
| Benzanthracen | Benzol | Triphenylen |
| Benzanthracen | Naphthalin | Benzanthracen |
| Benzanthracen | Naphthalin | Chrysen |
| Benzanthracen | Naphthalin | Benzphenanthren |
| Benzanthracen | Naphthalin | Fluoranthen |
| Benzanthracen | Naphthalin | Triphenylen |
| Chrysen | Benzol | Chrysen |
| Chrysen | Benzol | Benzphenanthren |
| Chrysen | Benzol | Fluoranthen |
| Chrysen | Benzol | Triphenylen |
| Chrysen | Naphthalin | Chrysen |
| Chrysen | Naphthalin | Benzphenanthren |
| Chrysen | Naphthalin | Fluoranthen |
| Chrysen | Naphthalin | Triphenylen |
| Benzphenanthren | Benzol | Benzphenanthren |
| Benzphenanthren | Benzol | Fluoranthen |
| Benzphenanthren | Benzol | Triphenylen |
| Benzphenanthren | Naphthalin | Benzphenanthren |
| Benzphenanthren | Naphthalin | Fluoranthen |
| Benzphenanthren | Naphthalin | Triphenylen |
| Fluoranthen | Benzol | Fluoranthen |
| Fluoranthen | Benzol | Triphenylen |
| Fluoranthen | Naphthalin | Fluoranthen |
| Fluoranthen | Naphthalin | Triphenylen |
| Triphenylen | Benzol | Triphenylen |
| Triphenylen | Naphthalin | Triphenylen |

Dabei sind die oben genannten Einheiten bevorzugt ausgewählt aus den Einheiten der Formeln (2) bis (110). So ist beispielsweise in Tabelle 1 für Ar¹ bzw. Ar³ das Benzol ausgewählt aus der Formel (2), das Naphthalin ausgewählt aus Strukturen der Formel (3) bis (5), das Pyren ausgewählt aus Strukturen der Formel (16) bis (18), das Naphthacen aus Strukturen der Formel (33) bis (35), das Benzanthracen aus Strukturen der Formel (36) bis (49), das Chrysen aus Strukturen der Formel (50) bis (57), das Benzphenanthren aus Strukturen der Formel (58) bis (65), das Fluoranthen aus Strukturen der Formel (19) bis (32) und das Triphenylen aus Strukturen der Formel (66) bis (68). Für die Gruppe Ar² ist das Benzol ausgewählt aus Strukturen der Formel (96) bis (100) und das Naphthalin aus Strukturen der Formel (101) bis (105). Diese Strukturen können jeweils durch einen oder mehrere Reste R¹ substituiert sein.

**Tabelle 2**

| Ar1 | Ar2 | Ar3 |
|---|---|---|
| Benzol | Phenanthren | Naphthalin |
| Benzol | Phenanthren | Anthracen |
| Benzol | Phenanthren | Phenanthren |
| Benzol | Phenanthren | Pyren |
| Benzol | Phenanthren | Naphthacen |
| Benzol | Phenanthren | Benzanthracen |
| Benzol | Phenanthren | Chrysen |
| Benzol | Phenanthren | Benzphenanthren |
| Benzol | Phenanthren | Fluoranthen |
| Benzol | Phenanthren | Triphenylen |
| Benzol | Pyren | Benzol |
| Benzol | Pyren | Naphthalin |
| Benzol | Pyren | Anthracen |
| Benzol | Pyren | Phenanthren |
| Benzol | Pyren | Pyren |
| Benzol | Pyren | Naphthacen |
| Benzol | Pyren | Benzanthracen |
| Benzol | Pyren | Chrysen |
| Benzol | Pyren | Benzphenanthren |
| Benzol | Pyren | Fluoranthen |
| Benzol | Pyren | Triphenylen |
| Benzol | Benzanthracen | Benzol |
| Benzol | Benzanthracen | Naphthalin |
| Benzol | Benzanthracen | Anthracen |
| Benzol | Benzanthracen | Phenanthren |
| Benzol | Benzanthracen | Pyren |
| Benzol | Benzanthracen | Naphthacen |
| Benzol | Benzanthracen | Benzanthracen |
| Benzol | Benzanthracen | Chrysen |
| Benzol | Benzanthracen | Benzphenanthren |
| Benzol | Benzanthracen | Fluoranthen |
| Benzol | Benzanthracen | Triphenylen |
| Benzol | Chrysen | Benzol |
| Benzol | Chrysen | Naphthalin |
| Benzol | Chrysen | Anthracen |
| Benzol | Chrysen | Phenanthren |
| Benzol | Chrysen | Pyren |
| Benzol | Chrysen | Naphthacen |
| Benzol | Chrysen | Benzanthracen |
| Benzol | Chrysen | Chrysen |
| Benzol | Chrysen | Benzphenanthren |
| Benzol | Chrysen | Fluoranthen |
| Benzol | Chrysen | Triphenylen |
| Benzol | Benzphenanthren | Benzol |
| Benzol | Benzphenanthren | Naphthalin |
| Benzol | Benzphenanthren | Anthracen |
| Benzol | Benzphenanthren | Phenanthren |
| Benzol | Benzphenanthren | Pyren |
| Benzol | Benzphenanthren | Naphthacen |
| Benzol | Benzphenanthren | Benzanthracen |
| Benzol | Benzphenanthren | Chrysen |
| Benzol | Benzphenanthren | Benzphenanthren |
| Benzol | Benzphenanthren | Fluoranthen |
| Benzol | Benzphenanthren | Triphenylen |
| Benzol | Fluoranthen | Benzol |
| Benzol | Fluoranthen | Naphthalin |
| Benzol | Fluoranthen | Anthracen |
| Benzol | Fluoranthen | Phenanthren |
| Benzol | Fluoranthen | Pyren |
| Benzol | Fluoranthen | Naphthacen |
| Benzol | Fluoranthen | Benzanthracen |
| Benzol | Fluoranthen | Chrysen |
| Benzol | Fluoranthen | Benzphenanthren |
| Benzol | Fluoranthen | Fluoranthen |
| Benzol | Fluoranthen | Triphenylen |
| Benzol | Triphenylen | Benzol |
| Benzol | Triphenylen | Naphthalin |
| Benzol | Triphenylen | Anthracen |
| Benzol | Triphenylen | Phenanthren |
| Benzol | Triphenylen | Pyren |
| Benzol | Triphenylen | Naphthacen |
| Benzol | Triphenylen | Benzanthracen |
| Benzol | Triphenylen | Chrysen |
| Benzol | Triphenylen | Benzphenanthren |
| Benzol | Triphenylen | Fluoranthen |
| Benzol | Triphenylen | Triphenylen |
| Naphthalin | Phenanthren | Benzol |
| Naphthalin | Phenanthren | Naphthalin |
| Naphthalin | Phenanthren | Anthracen |
| Naphthalin | Phenanthren | Phenanthren |
| Naphthalin | Phenanthren | Pyren |
| Naphthalin | Phenanthren | Naphthacen |
| Naphthalin | Phenanthren | Benzanthracen |
| Naphthalin | Phenanthren | Chrysen |
| Naphthalin | Phenanthren | Benzphenanthren |
| Naphthalin | Phenanthren | Fluoranthen |
| Naphthalin | Phenanthren | Triphenylen |
| Naphthalin | Pyren | Benzol |
| Naphthalin | Pyren | Naphthalin |
| Naphthalin | Pyren | Anthracen |
| Naphthalin | Pyren | Phenanthren |
| Naphthalin | Pyren | Pyren |
| Naphthalin | Pyren | Naphthacen |
| Naphthalin | Pyren | Benzanthracen |
| Naphthalin | Pyren | Chrysen |
| Naphthalin | Pyren | Benzphenanthren |
| Naphthalin | Pyren | Fluoranthen |
| Naphthalin | Pyren | Triphenylen |
| Naphthalin | Benzanthracen | Benzol |
| Naphthalin | Benzanthracen | Naphthalin |
| Naphthalin | Benzanthracen | Anthracen |
| Naphthalin | Benzanthracen | Phenanthren |
| Naphthalin | Benzanthracen | Pyren |
| Naphthalin | Benzanthracen | Naphthacen |
| Naphthalin | Benzanthracen | Benzanthracen |
| Naphthalin | Benzanthracen | Chrysen |
| Naphthalin | Benzanthracen | Benzphenanthren |
| Naphthalin | Benzanthracen | Fluoranthen |
| Naphthalin | Benzanthracen | Triphenylen |
| Naphthalin | Chrysen | Benzol |
| Naphthalin | Chrysen | Naphthalin |
| Naphthalin | Chrysen | Anthracen |
| Naphthalin | Chrysen | Phenanthren |
| Naphthalin | Chrysen | Pyren |
| Naphthalin | Chrysen | Naphthacen |
| Naphthalin | Chrysen | Benzanthracen |
| Naphthalin | Chrysen | Chrysen |
| Naphthalin | Chrysen | Benzphenanthren |
| Naphthalin | Chrysen | Fluoranthen |
| Naphthalin | Chrysen | Triphenylen |
| Naphthalin | Benzphenanthren | Benzol |
| Naphthalin | Benzphenanthren | Naphthalin |
| Naphthalin | Benzphenanthren | Anthracen |
| Naphthalin | Benzphenanthren | Phenanthren |
| Naphthalin | Benzphenanthren | Pyren |
| Naphthalin | Benzphenanthren | Naphthacen |
| Naphthalin | Benzphenanthren | Benzanthracen |
| Naphthalin | Benzphenanthren | Chrysen |
| Naphthalin | Benzphenanthren | Benzphenanthren |
| Naphthalin | Benzphenanthren | Fluoranthen |
| Naphthalin | Benzphenanthren | Triphenylen |
| Naphthalin | Fluoranthen | Benzol |
| Naphthalin | Fluoranthen | Naphthalin |
| Naphthalin | Fluoranthen | Anthracen |
| Naphthalin | Fluoranthen | Phenanthren |
| Naphthalin | Fluoranthen | Pyren |
| Naphthalin | Fluoranthen | Naphthacen |
| Naphthalin | Fluoranthen | Benzanthracen |
| Naphthalin | Fluoranthen | Chrysen |
| Naphthalin | Fluoranthen | Benzphenanthren |
| Naphthalin | Fluoranthen | Fluoranthen |
| Naphthalin | Fluoranthen | Triphenylen |
| Naphthalin | Triphenylen | Benzol |
| Naphthalin | Triphenylen | Naphthalin |
| Naphthalin | Triphenylen | Anthracen |
| Naphthalin | Triphenylen | Phenanthren |
| Naphthalin | Triphenylen | Pyren |
| Naphthalin | Triphenylen | Naphthacen |
| Naphthalin | Triphenylen | Benzanthracen |
| Naphthalin | Triphenylen | Chrysen |
| Naphthalin | Triphenylen | Benzphenanthren |
| Naphthalin | Triphenylen | Fluoranthen |
| Naphthalin | Triphenylen | Triphenylen |

Dabei ist in Tabelle 2 für Ar¹ und Ar³ das Benzol eine Gruppe der Formel (2), und das Naphthalin ist ausgewählt aus Strukturen der Formel (3) bis (5). Für die Gruppe Ar² ist das Pyren ausgewählt aus Strukturen der Formel (112) bis (115), das Naphthacen ist ausgewählt aus Strukturen der Formel (117) bis (120) und das Triphenylen aus Strukturen der Formel (116). Diese Strukturen können jeweils durch einen oder mehrere Reste R¹ substituiert sein.

Konkrete besonders bevorzugte Kombinationen aus Ar¹, Ar² und Ar³ können beispielsweise der folgenden Tabelle 3 entnommen werden. Die Brücken X sind Gruppen C(R²)₂. Besonders bevorzugt stehen beide Brücken X für C(CH₃)₂ oder beide Brücken X stehen für C(Phenyl)₂ oder eine Brücke X steht für C(CH₃)₂ und die andere Brücke X steht für C(Phenyl)₂. Die Gruppen Ar¹, Ar² und Ar³ können dabei durch einen oder mehrere Reste R¹ substituiert sein, sind aber bevorzugt unsubstituiert.

**Tabelle 3**

| Nr. | Ar1 | Ar2 | Ar3 |
|---|---|---|---|
| 1 | Formel (2) | Formel (86) | Formel (17) |
| 2 | Formel (2) | Formel (86) | Formel (28) |
| 3 | Formel (2) | Formel (86) | Formel (41) |
| 4 | Formel (2) | Formel (87) | Formel (17) |
| 5 | Formel (2) | Formel (87) | Formel (28) |
| 6 | Formel (2) | Formel (87) | Formel (41) |
| 7 | Formel (2) | Formel (91) | Formel (7) |
| 8 | Formel (2) | Formel (91) | Formel (8) |
| 9 | Formel (2) | Formel (91) | Formel (13) |
| 10 | Formel (2) | Formel (91) | Formel (17) |
| 11 | Formel (2) | Formel (91) | Formel (28) |
| 12 | Formel (2) | Formel (91) | Formel (41) |
| 13 | Formel (2) | Formel (93) | Formel (7) |
| 14 | Formel (2) | Formel (93) | Formel (8) |
| 15 | Formel (2) | Formel (93) | Formel (13) |
| 16 | Formel (2) | Formel (93) | Formel (17) |
| 17 | Formel (2) | Formel (93) | Formel (28) |
| 18 | Formel (2) | Formel (93) | Formel (41) |
| 19 | Formel (2) | Formel (95) | Formel (7) |
| 20 | Formel (2) | Formel (95) | Formel (8) |
| 21 | Formel (2) | Formel (95) | Formel (13) |
| 22 | Formel (2) | Formel (95) | Formel (17) |
| 23 | Formel (2) | Formel (95) | Formel (28) |
| 24 | Formel (2) | Formel (95) | Formel (41) |
| 25 | Formel (2) | Formel (102) | Formel (2) |
| 26 | Formel (2) | Formel (102) | Formel (3) |
| 27 | Formel (2) | Formel (102) | Formel (4) |
| 28 | Formel (2) | Formel (102) | Formel (7) |
| 29 | Formel (2) | Formel (102) | Formel (8) |
| 30 | Formel (2) | Formel (102) | Formel (13) |
| 31 | Formel (2) | Formel (102) | Formel (17) |
| 32 | Formel (2) | Formel (102) | Formel (28) |
| 33 | Formel (2) | Formel (102) | Formel (41) |
| 34 | Formel (3) | Formel (86) | Formel (7) |
| 35 | Formel (3) | Formel (86) | Formel (8) |
| 36 | Formel (3) | Formel (86) | Formel (13) |
| 37 | Formel (3) | Formel (86) | Formel (17) |
| 38 | Formel (3) | Formel (86) | Formel (28) |
| 39 | Formel (3) | Formel (86) | Formel (41) |
| 40 | Formel (3) | Formel (87) | Formel (7) |
| 41 | Formel (3) | Formel (87) | Formel (8) |
| 42 | Formel (3) | Formel (87) | Formel (13) |
| 43 | Formel (3) | Formel (87) | Formel (17) |
| 44 | Formel (3) | Formel (87) | Formel (28) |
| 45 | Formel (3) | Formel (87) | Formel (41) |
| 46 | Formel (3) | Formel (91) | Formel (3) |
| 47 | Formel (3) | Formel (91) | Formel (4) |
| 48 | Formel (3) | Formel (91) | Formel (7) |
| 49 | Formel (3) | Formel (91) | Formel (8) |
| 50 | Formel (3) | Formel (91) | Formel (13) |
| 51 | Formel (3) | Formel (91) | Formel (17) |
| 52 | Formel (3) | Formel (91) | Formel (28) |
| 52 | Formel (3) | Formel (91) | Formel (41) |
| 53 | Formel (3) | Formel (93) | Formel (3) |
| 54 | Formel (3) | Formel (93) | Formel (4) |
| 55 | Formel (3) | Formel (93) | Formel (7) |
| 56 | Formel (3) | Formel (93) | Formel (8) |
| 57 | Formel (3) | Formel (93) | Formel (13) |
| 58 | Formel (3) | Formel (93) | Formel (17) |
| 59 | Formel (3) | Formel (93) | Formel (28) |
| 60 | Formel (3) | Formel (93) | Formel (41) |
| 61 | Formel (3) | Formel (95) | Formel (3) |
| 62 | Formel (3) | Formel (95) | Formel (4) |
| 63 | Formel (3) | Formel (95) | Formel (7) |
| 64 | Formel (3) | Formel (95) | Formel (8) |
| 65 | Formel (3) | Formel (95) | Formel (13) |
| 66 | Formel (3) | Formel (95) | Formel (17) |
| 67 | Formel (3) | Formel (95) | Formel (28) |
| 68 | Formel (3) | Formel (95) | Formel (41) |
| 69 | Formel (3) | Formel (102) | Formel (2) |
| 70 | Formel (3) | Formel (102) | Formel (3) |
| 71 | Formel (3) | Formel (102) | Formel (4) |
| 72 | Formel (3) | Formel (102) | Formel (7) |
| 73 | Formel (3) | Formel (102) | Formel (8) |
| 74 | Formel (3) | Formel (102) | Formel (13) |
| 75 | Formel (3) | Formel (102) | Formel (17) |
| 76 | Formel (3) | Formel (102) | Formel (28) |
| 77 | Formel (3) | Formel (102) | Formel (41) |
| 78 | Formel (4) | Formel (86) | Formel (7) |
| 79 | Formel (4) | Formel (86) | Formel (8) |
| 80 | Formel (4) | Formel (86) | Formel (13) |
| 81 | Formel (4) | Formel (86) | Formel (17) |
| 82 | Formel (4) | Formel (86) | Formel (28) |
| 83 | Formel (4) | Formel (86) | Formel (41) |
| 84 | Formel (4) | Formel (87) | Formel (7) |
| 85 | Formel (4) | Formel (87) | Formel (8) |
| 86 | Formel (4) | Formel (87) | Formel (13) |
| 87 | Formel (4) | Formel (87) | Formel (17) |
| 88 | Formel (4) | Formel (87) | Formel (28) |
| 89 | Formel (4) | Formel (87) | Formel (41) |
| 90 | Formel (4) | Formel (91) | Formel (3) |
| 91 | Formel (4) | Formel (91) | Formel (4) |
| 92 | Formel (4) | Formel (91) | Formel (7) |
| 93 | Formel (4) | Formel (91) | Formel (8) |
| 94 | Formel (4) | Formel (91) | Formel (13) |
| 95 | Formel (4) | Formel (91) | Formel (17) |
| 96 | Formel (4) | Formel (91) | Formel (28) |
| 97 | Formel (4) | Formel (91) | Formel (41) |
| 98 | Formel (4) | Formel (93) | Formel (2) |
| 99 | Formel (4) | Formel (93) | Formel (3) |
| 100 | Formel (4) | Formel (93) | Formel (4) |
| 101 | Formel (4) | Formel (93) | Formel (7) |
| 102 | Formel (4) | Formel (93) | Formel (8) |
| 103 | Formel (4) | Formel (93) | Formel (13) |
| 104 | Formel (4) | Formel (93) | Formel (17) |
| 105 | Formel (4) | Formel (93) | Formel (28) |
| 106 | Formel (4) | Formel (93) | Formel (41) |
| 107 | Formel (4) | Formel (95) | Formel (2) |
| 108 | Formel (4) | Formel (95) | Formel (3) |
| 109 | Formel (4) | Formel (95) | Formel (4) |
| 110 | Formel (4) | Formel (95) | Formel (7) |
| 111 | Formel (4) | Formel (95) | Formel (8) |
| 112 | Formel (4) | Formel (95) | Formel (13) |
| 113 | Formel (4) | Formel (95) | Formel (17) |
| 114 | Formel (4) | Formel (95) | Formel (28) |
| 115 | Formel (4) | Formel (95) | Formel (41) |
| 116 | Formel (4) | Formel (102) | Formel (2) |
| 117 | Formel (4) | Formel (102) | Formel (3) |
| 118 | Formel (4) | Formel (102) | Formel (4) |
| 119 | Formel (4) | Formel (102) | Formel (7) |
| 120 | Formel (4) | Formel (102) | Formel (8) |
| 121 | Formel (4) | Formel (102) | Formel (13) |
| 122 | Formel (4) | Formel (102) | Formel (17) |
| 123 | Formel (4) | Formel (102) | Formel (28) |
| 124 | Formel (4) | Formel (102) | Formel (41) |
| 125 | Formel (7) | Formel (86) | Formel (3) |
| 126 | Formel (7) | Formel (86) | Formel (4) |
| 127 | Formel (7) | Formel (86) | Formel (7) |
| 128 | Formel (7) | Formel (86) | Formel (8) |
| 129 | Formel (7) | Formel (86) | Formel (13) |
| 130 | Formel (7) | Formel (86) | Formel (17) |
| 131 | Formel (7) | Formel (86) | Formel (28) |
| 132 | Formel (7) | Formel (86) | Formel (41) |
| 133 | Formel (7) | Formel (87) | Formel (3) |
| 134 | Formel (7) | Formel (87) | Formel (4) |
| 135 | Formel (7) | Formel (87) | Formel (7) |
| 136 | Formel (7) | Formel (87) | Formel (8) |
| 137 | Formel (7) | Formel (87) | Formel (13) |
| 138 | Formel (7) | Formel (87) | Formel (17) |
| 139 | Formel (7) | Formel (87) | Formel (28) |
| 140 | Formel (7) | Formel (87) | Formel (41) |
| 141 | Formel (7) | Formel (91) | Formel (2) |
| 142 | Formel (7) | Formel (91) | Formel (3) |
| 143 | Formel (7) | Formel (91) | Formel (4) |
| 144 | Formel (7) | Formel (91) | Formel (7) |
| 145 | Formel (7) | Formel (91) | Formel (8) |
| 146 | Formel (7) | Formel (91) | Formel (13) |
| 147 | Formel (7) | Formel (91) | Formel (17) |
| 148 | Formel (7) | Formel (91) | Formel (28) |
| 149 | Formel (7) | Formel (91) | Formel (41) |
| 150 | Formel (7) | Formel (93) | Formel (2) |
| 151 | Formel (7) | Formel (93) | Formel (3) |
| 152 | Formel (7) | Formel (93) | Formel (4) |
| 153 | Formel (7) | Formel (93) | Formel (7) |
| 154 | Formel (7) | Formel (93) | Formel (8) |
| 155 | Formel (7) | Formel (93) | Formel (13) |
| 156 | Formel (7) | Formel (93) | Formel (17) |
| 157 | Formel (7) | Formel (93) | Formel (28) |
| 158 | Formel (7) | Formel (93) | Formel (41) |
| 159 | Formel (7) | Formel (95) | Formel (2) |
| 160 | Formel (7) | Formel (95) | Formel (3) |
| 161 | Formel (7) | Formel (95) | Formel (4) |
| 162 | Formel (7) | Formel (95) | Formel (7) |
| 163 | Formel (7) | Formel (95) | Formel (8) |
| 164 | Formel (7) | Formel (95) | Formel (13) |
| 165 | Formel (7) | Formel (95) | Formel (17) |
| 166 | Formel (7) | Formel (95) | Formel (28) |
| 167 | Formel (7) | Formel (95) | Formel (41) |
| 168 | Formel (7) | Formel (102) | Formel (2) |
| 169 | Formel (7) | Formel (102) | Formel (3) |
| 170 | Formel (7) | Formel (102) | Formel (4) |
| 171 | Formel (7) | Formel (102) | Formel (7) |
| 172 | Formel (7) | Formel (102) | Formel (8) |
| 173 | Formel (7) | Formel (102) | Formel (13) |
| 174 | Formel (7) | Formel (102) | Formel (17) |
| 175 | Formel (7) | Formel (102) | Formel (28) |
| 176 | Formel (7) | Formel (102) | Formel (41) |
| 177 | Formel (8) | Formel (86) | Formel (3) |
| 178 | Formel (8) | Formel (86) | Formel (4) |
| 179 | Formel (8) | Formel (86) | Formel (7) |
| 180 | Formel (8) | Formel (86) | Formel (8) |
| 181 | Formel (8) | Formel (86) | Formel (13) |
| 182 | Formel (8) | Formel (86) | Formel (17) |
| 183 | Formel (8) | Formel (86) | Formel (28) |
| 184 | Formel (8) | Formel (86) | Formel (41) |
| 185 | Formel (8) | Formel (87) | Formel (3) |
| 186 | Formel (8) | Formel (87) | Formel (4) |
| 187 | Formel (8) | Formel (87) | Formel (7) |
| 188 | Formel (8) | Formel (87) | Formel (8) |
| 189 | Formel (8) | Formel (87) | Formel (13) |
| 190 | Formel (8) | Formel (87) | Formel (17) |
| 191 | Formel (8) | Formel (87) | Formel (28) |
| 192 | Formel (8) | Formel (87) | Formel (41) |
| 193 | Formel (8) | Formel (91) | Formel (2) |
| 194 | Formel (8) | Formel (91) | Formel (3) |
| 195 | Formel (8) | Formel (91) | Formel (4) |
| 196 | Formel (8) | Formel (91) | Formel (7) |
| 197 | Formel (8) | Formel (91) | Formel (8) |
| 198 | Formel (8) | Formel (91) | Formel (13) |
| 199 | Formel (8) | Formel (91) | Formel (17) |
| 200 | Formel (8) | Formel (91) | Formel (28) |
| 201 | Formel (8) | Formel (91) | Formel (41) |
| 202 | Formel (8) | Formel (93) | Formel (2) |
| 203 | Formel (8) | Formel (93) | Formel (3) |
| 204 | Formel (8) | Formel (93) | Formel (4) |
| 205 | Formel (8) | Formel (93) | Formel (7) |
| 206 | Formel (8) | Formel (93) | Formel (8) |
| 207 | Formel (8) | Formel (93) | Formel (13) |
| 208 | Formel (8) | Formel (93) | Formel (17) |
| 209 | Formel (8) | Formel (93) | Formel (28) |
| 210 | Formel (8) | Formel (93) | Formel (41) |
| 211 | Formel (8) | Formel (95) | Formel (2) |
| 212 | Formel (8) | Formel (95) | Formel (3) |
| 213 | Formel (8) | Formel (95) | Formel (4) |
| 214 | Formel (8) | Formel (95) | Formel (7) |
| 215 | Formel (8) | Formel (95) | Formel (8) |
| 216 | Formel (8) | Formel (95) | Formel (13) |
| 217 | Formel (8) | Formel (95) | Formel (17) |
| 218 | Formel (8) | Formel (95) | Formel (28) |
| 219 | Formel (8) | Formel (95) | Formel (41) |
| 220 | Formel (8) | Formel (102) | Formel (2) |
| 221 | Formel (8) | Formel (102) | Formel (3) |
| 222 | Formel (8) | Formel (102) | Formel (4) |
| 223 | Formel (8) | Formel (102) | Formel (7) |
| 224 | Formel (8) | Formel (102) | Formel (8) |
| 225 | Formel (8) | Formel (102) | Formel (13) |
| 226 | Formel (8) | Formel (102) | Formel (17) |
| 227 | Formel (8) | Formel (102) | Formel (28) |
| 228 | Formel (8) | Formel (102) | Formel (41) |
| 229 | Formel (13) | Formel (86) | Formel (3) |
| 230 | Formel (13) | Formel (86) | Formel (4) |
| 231 | Formel (13) | Formel (86) | Formel (7) |
| 232 | Formel (13) | Formel (86) | Formel (8) |
| 233 | Formel (13) | Formel (86) | Formel (13) |
| 234 | Formel (13) | Formel (86) | Formel (17) |
| 235 | Formel (13) | Formel (86) | Formel (28) |
| 236 | Formel (13) | Formel (86) | Formel (41) |
| 237 | Formel (13) | Formel (87) | Formel (3) |
| 238 | Formel (13) | Formel (87) | Formel (4) |
| 239 | Formel (13) | Formel (87) | Formel (7) |
| 240 | Formel (13) | Formel (87) | Formel (8) |
| 241 | Formel (13) | Formel (87) | Formel (13) |
| 242 | Formel (13) | Formel (87) | Formel (17) |
| 243 | Formel (13) | Formel (87) | Formel (28) |
| 244 | Formel (13) | Formel (87) | Formel (41) |
| 245 | Formel (13) | Formel (91) | Formel (2) |
| 246 | Formel (13) | Formel (91) | Formel (3) |
| 247 | Formel (13) | Formel (91) | Formel (4) |
| 248 | Formel (13) | Formel (91) | Formel (7) |
| 249 | Formel (13) | Formel (91) | Formel (8) |
| 250 | Formel (13) | Formel (91) | Formel (13) |
| 251 | Formel (13) | Formel (91) | Formel (17) |
| 252 | Formel (13) | Formel (91) | Formel (28) |
| 253 | Formel (13) | Formel (91) | Formel (41) |
| 254 | Formel (13) | Formel (93) | Formel (2) |
| 255 | Formel (13) | Formel (93) | Formel (3) |
| 256 | Formel (13) | Formel (93) | Formel (4) |
| 257 | Formel (13) | Formel (93) | Formel (7) |
| 258 | Formel (13) | Formel (93) | Formel (8) |
| 259 | Formel (13) | Formel (93) | Formel (13) |
| 260 | Formel (13) | Formel (93) | Formel (17) |
| 261 | Formel (13) | Formel (93) | Formel (28) |
| 262 | Formel (13) | Formel (93) | Formel (41) |
| 263 | Formel (13) | Formel (95) | Formel (2) |
| 264 | Formel (13) | Formel (95) | Formel (3) |
| 265 | Formel (13) | Formel (95) | Formel (4) |
| 266 | Formel (13) | Formel (95) | Formel (7) |
| 267 | Formel (13) | Formel (95) | Formel (8) |
| 268 | Formel (13) | Formel (95) | Formel (13) |
| 269 | Formel (13) | Formel (95) | Formel (17) |
| 270 | Formel (13) | Formel (95) | Formel (28) |
| 271 | Formel (13) | Formel (95) | Formel (41) |
| 272 | Formel (13) | Formel (102) | Formel (2) |
| 273 | Formel (13) | Formel (102) | Formel (3) |
| 274 | Formel (13) | Formel (102) | Formel (4) |
| 275 | Formel (13) | Formel (102) | Formel (7) |
| 276 | Formel (13) | Formel (102) | Formel (8) |
| 277 | Formel (13) | Formel (102) | Formel (13) |
| 278 | Formel (13) | Formel (102) | Formel (17) |
| 279 | Formel (13) | Formel (102) | Formel (28) |
| 280 | Formel (13) | Formel (102) | Formel (41) |
| 281 | Formel (17) | Formel (86) | Formel (2) |
| 282 | Formel (17) | Formel (86) | Formel (3) |
| 283 | Formel (17) | Formel (86) | Formel (4) |
| 284 | Formel (17) | Formel (86) | Formel (7) |
| 285 | Formel (17) | Formel (86) | Formel (8) |
| 286 | Formel (17) | Formel (86) | Formel (13) |
| 287 | Formel (17) | Formel (86) | Formel (17) |
| 288 | Formel (17) | Formel (86) | Formel (28) |
| 289 | Formel (17) | Formel (86) | Formel (41) |
| 290 | Formel (17) | Formel (87) | Formel (2) |
| 291 | Formel (17) | Formel (87) | Formel (3) |
| 292 | Formel (17) | Formel (87) | Formel (4) |
| 293 | Formel (17) | Formel (87) | Formel (7) |
| 294 | Formel (17) | Formel (87) | Formel (8) |
| 295 | Formel (17) | Formel (87) | Formel (13) |
| 296 | Formel (17) | Formel (87) | Formel (17) |
| 297 | Formel (17) | Formel (87) | Formel (28) |
| 298 | Formel (17) | Formel (87) | Formel (41) |
| 299 | Formel (17) | Formel (91) | Formel (2) |
| 300 | Formel (17) | Formel (91) | Formel (3) |
| 301 | Formel (17) | Formel (91) | Formel (4) |
| 302 | Formel (17) | Formel (91) | Formel (7) |
| 303 | Formel (17) | Formel (91) | Formel (8) |
| 304 | Formel (17) | Formel (91) | Formel (13) |
| 305 | Formel (17) | Formel (91) | Formel (17) |
| 306 | Formel (17) | Formel (91) | Formel (28) |
| 307 | Formel (17) | Formel (91) | Formel (41) |
| 308 | Formel (17) | Formel (93) | Formel (2) |
| 309 | Formel (17) | Formel (93) | Formel (3) |
| 310 | Formel (17) | Formel (93) | Formel (4) |
| 311 | Formel (17) | Formel (93) | Formel (7) |
| 312 | Formel (17) | Formel (93) | Formel (8) |
| 313 | Formel (17) | Formel (93) | Formel (13) |
| 314 | Formel (17) | Formel (93) | Formel (17) |
| 315 | Formel (17) | Formel (93) | Formel (28) |
| 316 | Formel (17) | Formel (93) | Formel (41) |
| 317 | Formel (17) | Formel (95) | Formel (2) |
| 318 | Formel (17) | Formel (95) | Formel (3) |
| 319 | Formel (17) | Formel (95) | Formel (4) |
| 320 | Formel (17) | Formel (95) | Formel (7) |
| 321 | Formel (17) | Formel (95) | Formel (8) |
| 322 | Formel (17) | Formel (95) | Formel (13) |
| 323 | Formel (17) | Formel (95) | Formel (17) |
| 324 | Formel (17) | Formel (95) | Formel (28) |
| 325 | Formel (17) | Formel (95) | Formel (41) |
| 326 | Formel (17) | Formel (102) | Formel (2) |
| 327 | Formel (17) | Formel (102) | Formel (3) |
| 328 | Formel (17) | Formel (102) | Formel (4) |
| 329 | Formel (17) | Formel (102) | Formel (7) |
| 330 | Formel (17) | Formel (102) | Formel (8) |
| 331 | Formel (17) | Formel (102) | Formel (13) |
| 332 | Formel (17) | Formel (102) | Formel (17) |
| 333 | Formel (17) | Formel (102) | Formel (28) |
| 334 | Formel (17) | Formel (102) | Formel (41) |
| 335 | Formel (28) | Formel (86) | Formel (2) |
| 336 | Formel (28) | Formel (86) | Formel (3) |
| 337 | Formel (28) | Formel (86) | Formel (4) |
| 338 | Formel (28) | Formel (86) | Formel (7) |
| 339 | Formel (28) | Formel (86) | Formel (8) |
| 340 | Formel (28) | Formel (86) | Formel (13) |
| 341 | Formel (28) | Formel (86) | Formel (17) |
| 342 | Formel (28) | Formel (86) | Formel (28) |
| 343 | Formel (28) | Formel (86) | Formel (41) |
| 344 | Formel (28) | Formel (87) | Formel (2) |
| 345 | Formel (28) | Formel (87) | Formel (3) |
| 346 | Formel (28) | Formel (87) | Formel (4) |
| 347 | Formel (28) | Formel (87) | Formel (7) |
| 348 | Formel (28) | Formel (87) | Formel (8) |
| 349 | Formel (28) | Formel (87) | Formel (13) |
| 350 | Formel (28) | Formel (87) | Formel (17) |
| 351 | Formel (28) | Formel (87) | Formel (28) |
| 352 | Formel (28) | Formel (87) | Formel (41) |
| 353 | Formel (28) | Formel (91) | Formel (2) |
| 354 | Formel (28) | Formel (91) | Formel (3) |
| 355 | Formel (28) | Formel (91) | Formel (4) |
| 356 | Formel (28) | Formel (91) | Formel (7) |
| 357 | Formel (28) | Formel (91) | Formel (8) |
| 358 | Formel (28) | Formel (91) | Formel (13) |
| 359 | Formel (28) | Formel (91) | Formel (17) |
| 360 | Formel (28) | Formel (91) | Formel (28) |
| 361 | Formel (28) | Formel (91) | Formel (41) |
| 362 | Formel (28) | Formel (93) | Formel (2) |
| 363 | Formel (28) | Formel (93) | Formel (3) |
| 364 | Formel (28) | Formel (93) | Formel (4) |
| 365 | Formel (28) | Formel (93) | Formel (7) |
| 366 | Formel (28) | Formel (93) | Formel (8) |
| 367 | Formel (28) | Formel (93) | Formel (13) |
| 368 | Formel (28) | Formel (93) | Formel (17) |
| 369 | Formel (28) | Formel (93) | Formel (28) |
| 370 | Formel (28) | Formel (93) | Formel (41) |
| 371 | Formel (28) | Formel (95) | Formel (2) |
| 372 | Formel (28) | Formel (95) | Formel (3) |
| 373 | Formel (28) | Formel (95) | Formel (4) |
| 374 | Formel (28) | Formel (95) | Formel (7) |
| 375 | Formel (28) | Formel (95) | Formel (8) |
| 376 | Formel (28) | Formel (95) | Formel (13) |
| 377 | Formel (28) | Formel (95) | Formel (17) |
| 378 | Formel (28) | Formel (95) | Formel (28) |
| 379 | Formel (28) | Formel (95) | Formel (41) |
| 380 | Formel (28) | Formel (102) | Formel (2) |
| 381 | Formel (28) | Formel (102) | Formel (3) |
| 382 | Formel (28) | Formel (102) | Formel (4) |
| 383 | Formel (28) | Formel (102) | Formel (7) |
| 384 | Formel (28) | Formel (102) | Formel (8) |
| 385 | Formel (28) | Formel (102) | Formel (13) |
| 386 | Formel (28) | Formel (102) | Formel (17) |
| 387 | Formel (28) | Formel (102) | Formel (28) |
| 388 | Formel (28) | Formel (102) | Formel (41) |
| 389 | Formel (41) | Formel (86) | Formel (2) |
| 390 | Formel (41) | Formel (86) | Formel (3) |
| 391 | Formel (41) | Formel (86) | Formel (4) |
| 392 | Formel (41) | Formel (86) | Formel (7) |
| 393 | Formel (41) | Formel (86) | Formel (8) |
| 394 | Formel (41) | Formel (86) | Formel (13) |
| 395 | Formel (41) | Formel (86) | Formel (17) |
| 396 | Formel (41) | Formel (86) | Formel (28) |
| 397 | Formel (41) | Formel (86) | Formel (41) |
| 398 | Formel (41) | Formel (87) | Formel (2) |
| 399 | Formel (41) | Formel (87) | Formel (3) |
| 400 | Formel (41) | Formel (87) | Formel (4) |
| 401 | Formel (41) | Formel (87) | Formel (7) |
| 402 | Formel (41) | Formel (87) | Formel (8) |
| 403 | Formel (41) | Formel (87) | Formel (13) |
| 404 | Formel (41) | Formel (87) | Formel (17) |
| 405 | Formel (41) | Formel (87) | Formel (28) |
| 406 | Formel (41) | Formel (87) | Formel (41) |
| 407 | Formel (41) | Formel (91) | Formel (2) |
| 408 | Formel (41) | Formel (91) | Formel (3) |
| 409 | Formel (41) | Formel (91) | Formel (4) |
| 410 | Formel (41) | Formel (91) | Formel (7) |
| 411 | Formel (41) | Formel (91) | Formel (8) |
| 412 | Formel (41) | Formel (91) | Formel (13) |
| 413 | Formel (41) | Formel (91) | Formel (17) |
| 414 | Formel (41) | Formel (91) | Formel (28) |
| 415 | Formel (41) | Formel (91) | Formel (41) |
| 416 | Formel (41) | Formel (93) | Formel (2) |
| 417 | Formel (41) | Formel (93) | Formel (3) |
| 418 | Formel (41) | Formel (93) | Formel (4) |
| 419 | Formel (41) | Formel (93) | Formel (7) |
| 420 | Formel (41) | Formel (93) | Formel (8) |
| 421 | Formel (41) | Formel (93) | Formel (13) |
| 422 | Formel (41) | Formel (93) | Formel (17) |
| 423 | Formel (41) | Formel (93) | Formel (28) |
| 424 | Formel (41) | Formel (93) | Formel (41) |
| 425 | Formel (41) | Formel (95) | Formel (2) |
| 426 | Formel (41) | Formel (95) | Formel (3) |
| 427 | Formel (41) | Formel (95) | Formel (4) |
| 428 | Formel (41) | Formel (95) | Formel (7) |
| 429 | Formel (41) | Formel (95) | Formel (8) |
| 430 | Formel (41) | Formel (95) | Formel (13) |
| 431 | Formel (41) | Formel (95) | Formel (17) |
| 432 | Formel (41) | Formel (95) | Formel (28) |
| 433 | Formel (41) | Formel (95) | Formel (41) |
| 434 | Formel (41) | Formel (102) | Formel (2) |
| 435 | Formel (41) | Formel (102) | Formel (3) |
| 436 | Formel (41) | Formel (102) | Formel (4) |
| 437 | Formel (41) | Formel (102) | Formel (7) |
| 438 | Formel (41) | Formel (102) | Formel (8) |
| 439 | Formel (41) | Formel (102) | Formel (13) |
| 440 | Formel (41) | Formel (102) | Formel (17) |
| 441 | Formel (41) | Formel (102) | Formel (28) |
| 442 | Formel (41) | Formel (102) | Formel (41) |

Die Symbole X stehen gleich oder verschieden bei jedem Auftreten für C(R²)₂. Dabei steht R² bevorzugt für eine Alkyl- oder Arylgruppe.

Besonders bevorzugt sind Ar¹, Ar² und Ar³ gewählt, wie in den Tabellen 1 und 2 dargestellt, und X steht gleichzeitig gleich oder verschieden bei jedem Auftreten für C(R²)₂. Dabei steht R² bevorzugt für eine Alkyl- oder Arylgruppe.

Besonders bevorzugt sind Verbindungen gemäß Formel (1), ausgewählt aus den Formeln (111) bis (141), wobei die aromatischen Systeme jeweils auch durch einen oder mehrere Reste R¹ substituiert sein können:

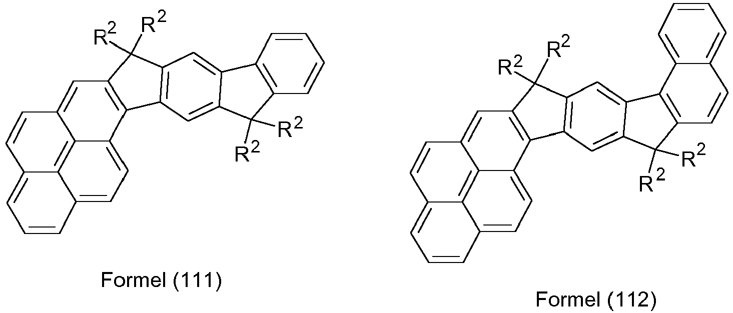

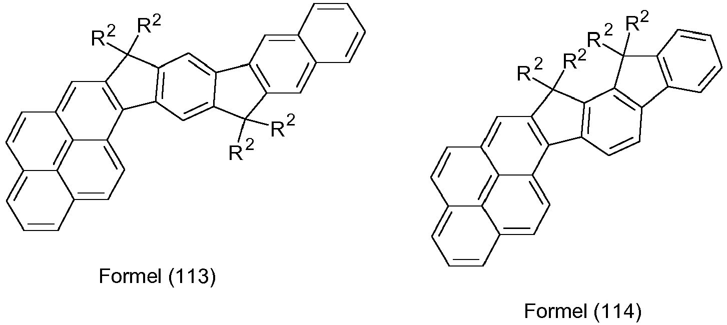

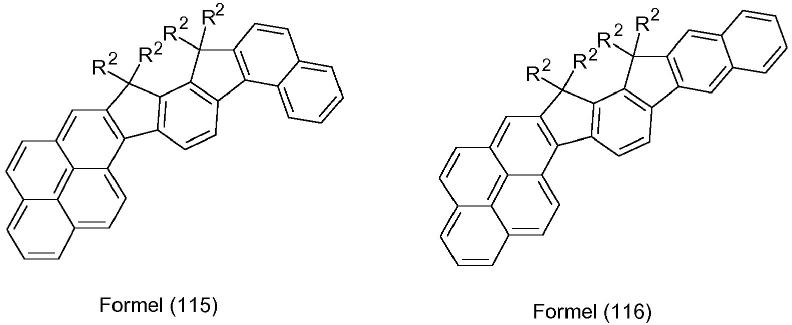

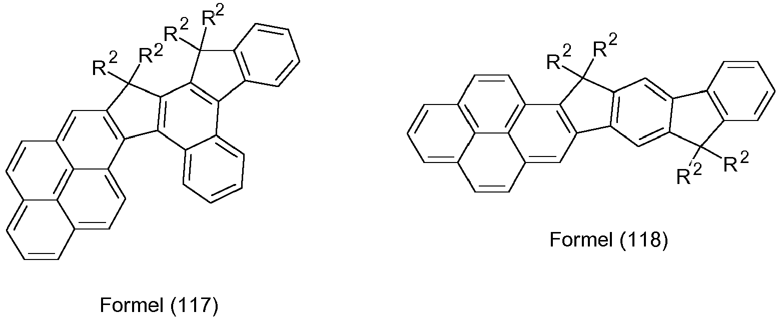

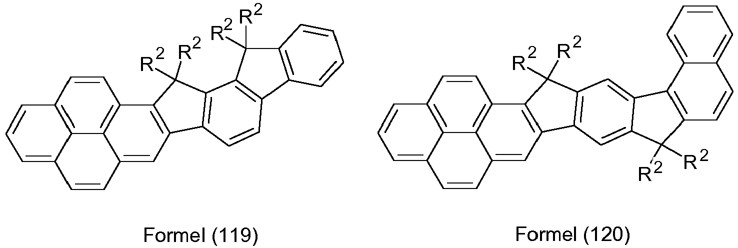

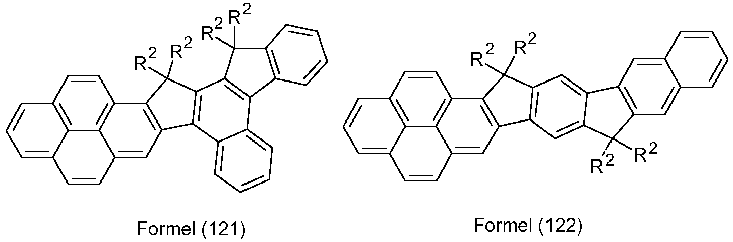

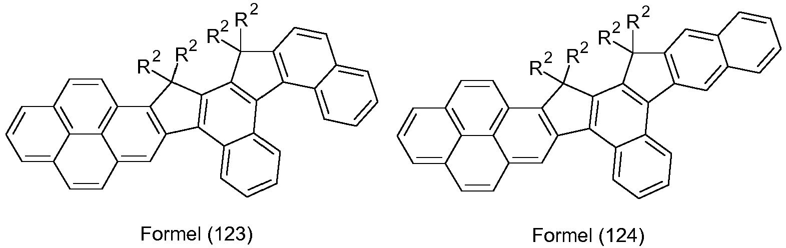

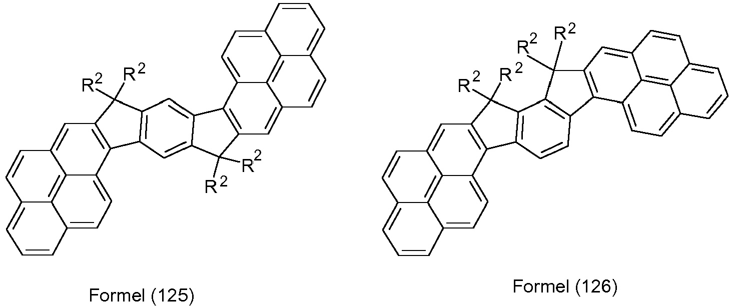

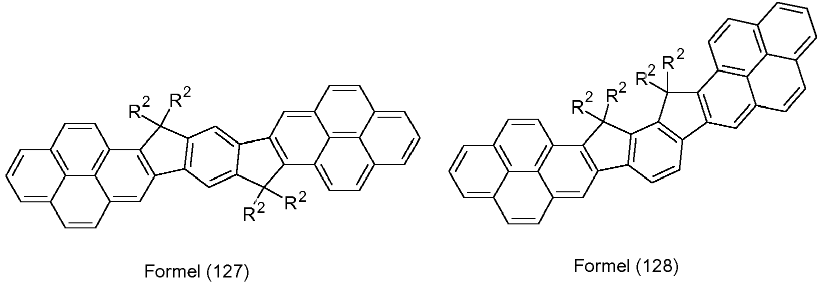

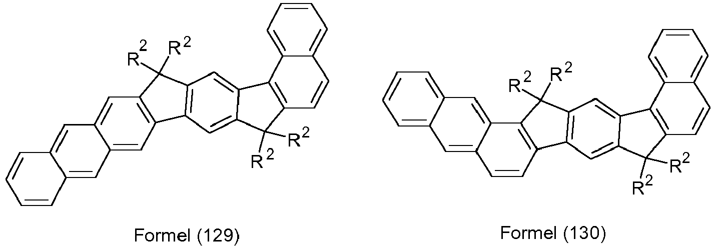

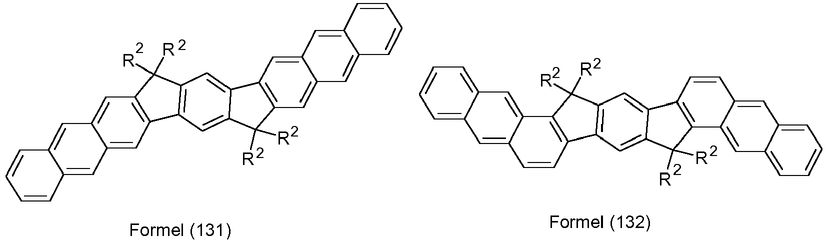

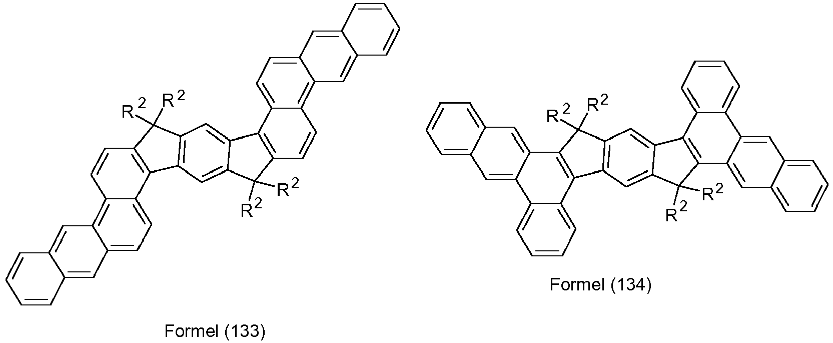

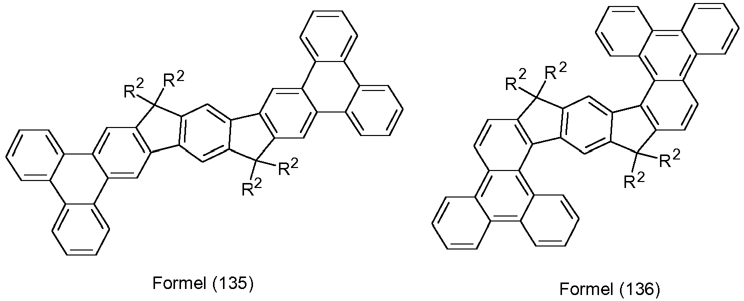

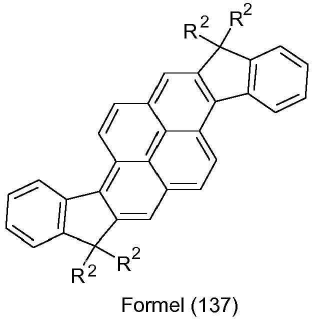

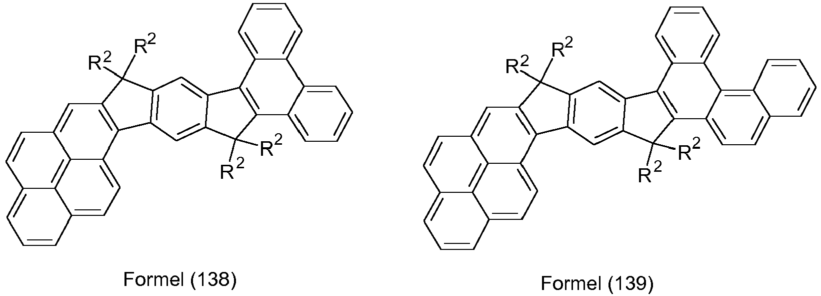

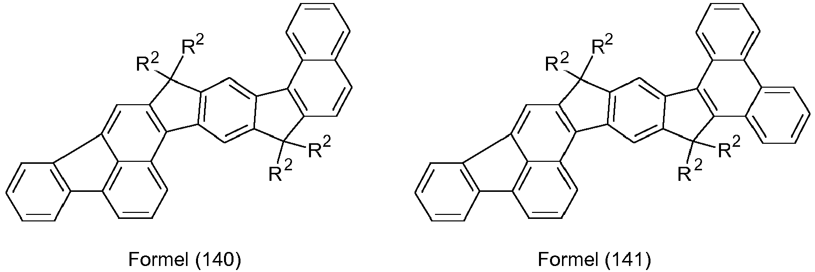

Bevorzugt ist der Substituent R¹ ausgewählt aus H, D, geradkettigen Alkylgruppen mit 1 bis 6 C-Atomen, verzweigten oder cyclischen Alkylgruppen mit 3 bis 6 C-Atomen oder einem aromatischen oder heteroaromatischen Ringsystem mit 5 bis 24 aromatischen Ringatomen; dabei können zwei oder mehrere Substituenten R¹ auch miteinander ein mono- oder polycyclisches Ringsystem bilden. Besonders bevorzugt ist der Substituent R¹ ausgewählt aus H, D, Alkylgruppen, ausgewählt aus Methyl, Ethyl, n-Propyl, iso-Propyl, n-Butyl, iso-Butyl, tert-Butyl, Cyclopentyl oder Cyclohexyl, insbesondere Methyl oder tert-Butyl, und aromatischen oder heteroaromatischen Ringsystemen, ausgewählt aus der Gruppe bestehend aus unsubstituiertem oder durch R³ substituiertem Phenyl oder Naphthyl, Benzimidazol, welches auch durch Phenyl oder andere Reste R³ substituiert sein kann, Phenyl-Benzimidazol, wobei das Benzimidazol auch durch Phenyl oder andere Reste R³ substituiert sein kann, oder Triazin, welches auch durch Phenyl oder andere Reste R³ substituiert sein kann.

Bevorzugt sind die Reste R² ausgesucht aus geradkettigen Alkylgruppen mit 1 bis 4 C-Atomen oder verzweigten Alkylgruppen mit 3 oder 4 C-Atomen, insbesondere Methylgruppen, oder Phenylgruppen; dabei können zwei oder mehrere Reste R² miteinander ein Ringsystem bilden. Wenn mehrere Reste R² miteinander ein Ringsystem bilden, wird hierdurch eine Spiro-Struktur gebildet. Dies kann insbesondere dann bevorzugt sein, wenn die Reste R² für Phenylgruppen stehen oder wenn zwei Reste R² für Alkylgruppen stehen, die miteinander ein Ringsystem bilden.

Beispiele für bevorzugte Verbindungen gemäß Formel (1) sind die im Folgenden abgebildeten Strukturen (1) bis (246).

| | |
|---|---|
| 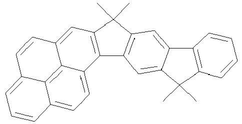 | 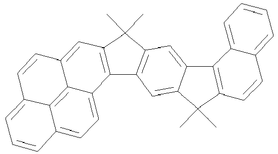 |
| (1) | (2) |
| 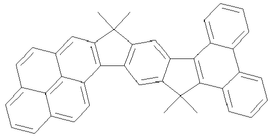 | 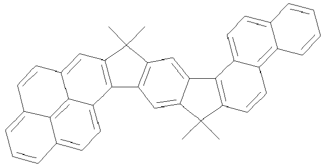 |
| (3) | (4) |
| 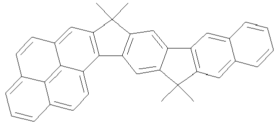 | 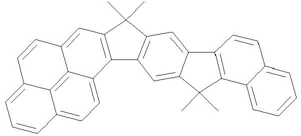 |
| (5) | (6) |
| 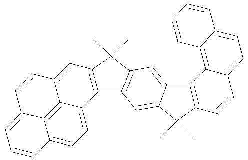 | 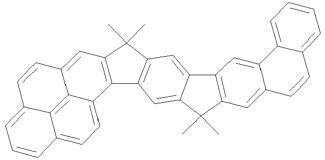 |
| (7) | (8) |
| 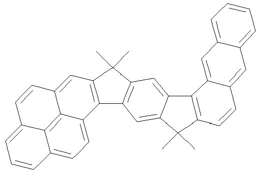 | 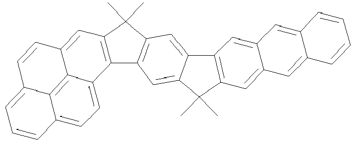 |
| (9) | (10) |
| 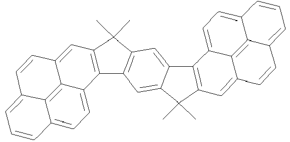 | 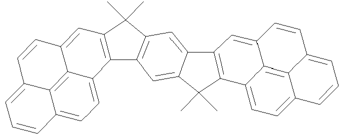 |
| (11) | (12) |
| 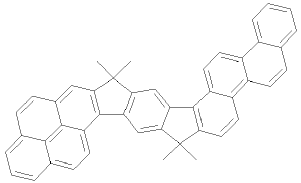 | 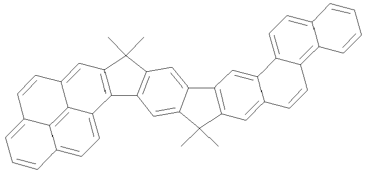 |
| (13) | (14) |
| 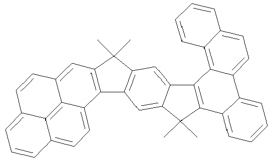 | 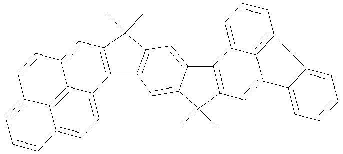 |
| (15) | (16) |
| 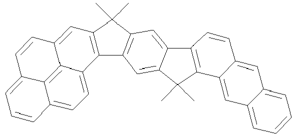 | 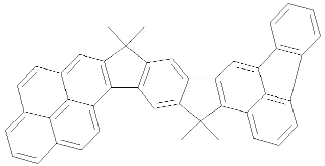 |
| (17) | (18) |
| 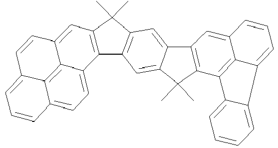 | 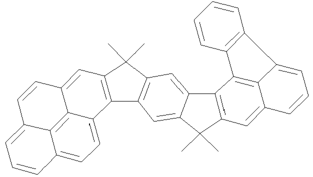 |
| (19) | (20) |
| 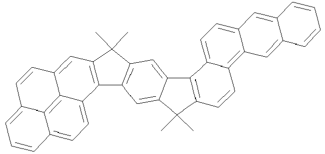 | 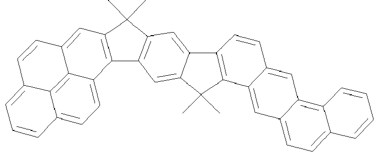 |
| (21) | (22) |
| 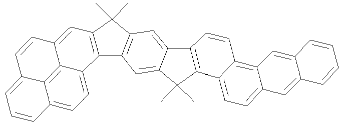 | 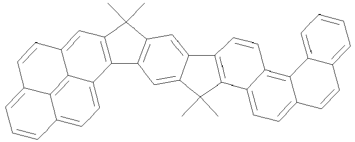 |
| (23) | (24) |
| 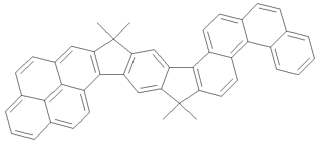 | 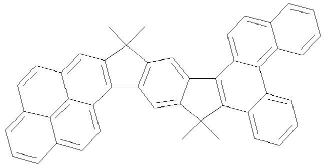 |
| (25) | (26) |
| 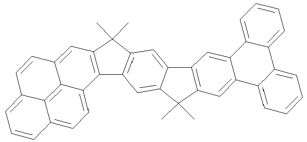 | 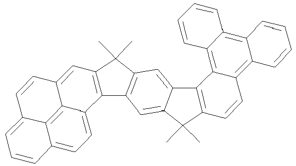 |
| (27) | (28) |
| 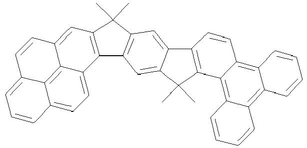 | 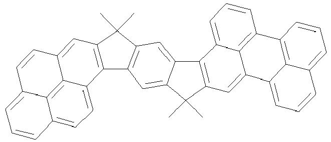 |
| (29) | (30) |
| 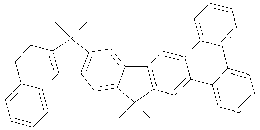 | 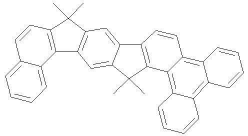 |
| (31) | (32) |
| 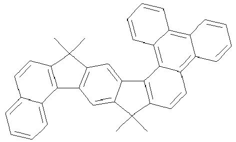 | 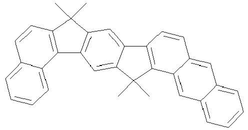 |
| (33) | (34) |
| 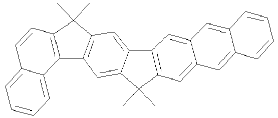 | 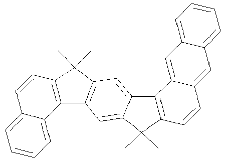 |
| (35) | (36) |
| 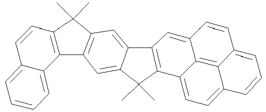 | 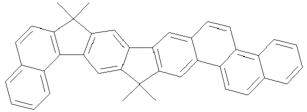 |
| (37) | (38) |
| 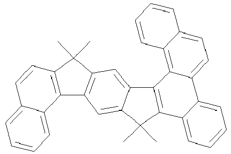 | 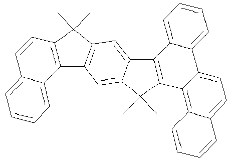 |
| (39) | (40) |
| 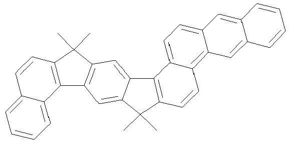 | 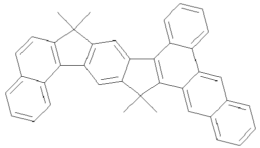 |
| (41) | (42) |
| 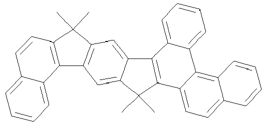 | 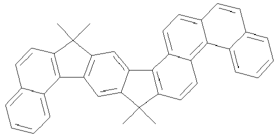 |
| (43) | (44) |
| 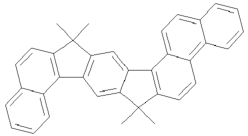 | 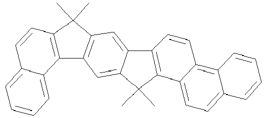 |
| (45) | (46) |
| 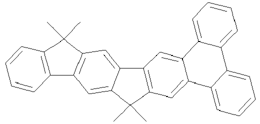 | 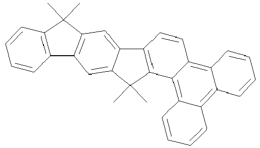 |
| (47) | (48) |
| 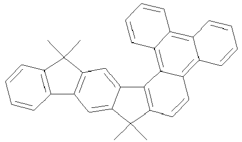 | 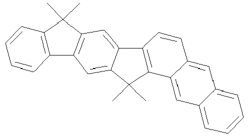 |
| (49) | (50) |
| 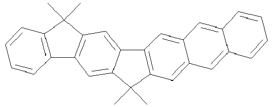 | 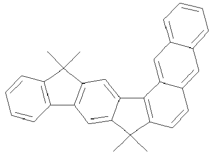 |
| (51) | (52) |
| 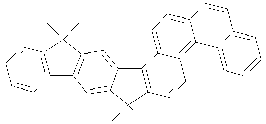 | 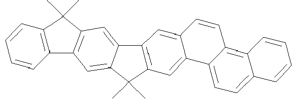 |
| (53) | (54) |
| 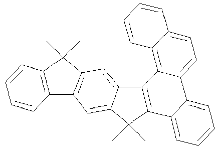 | 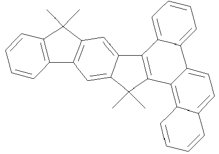 |
| (55) | (56) |
| 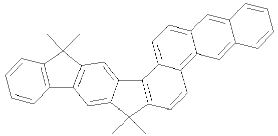 | 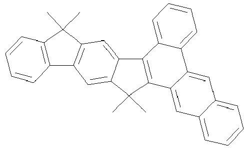 |
| (57) | (58) |
| 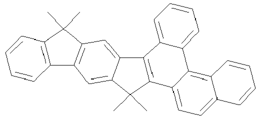 | 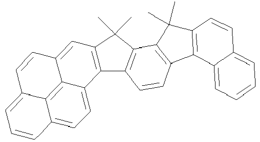 |
| (59) | (60) |
| 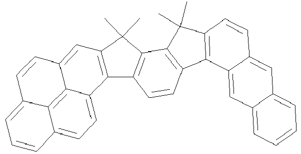 | 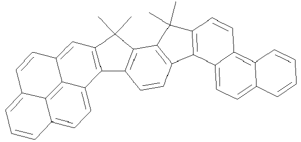 |
| (61) | (62) |
| 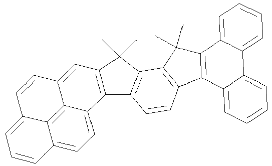 | 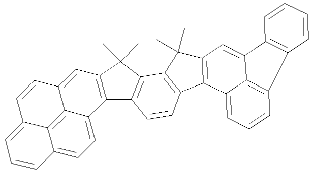 |
| (63) | (64) |
| 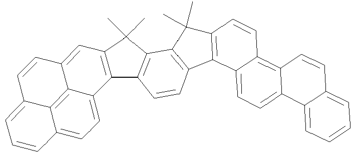 | 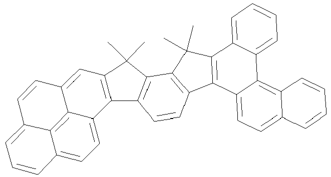 |
| (65) | (66) |
| 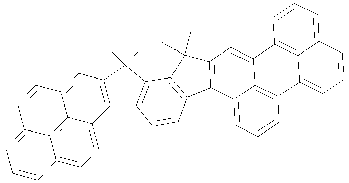 | 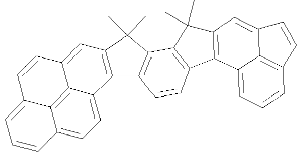 |
| (67) | (68) |
| 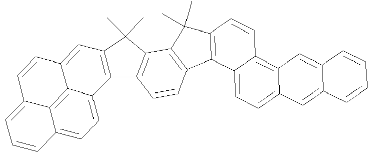 | 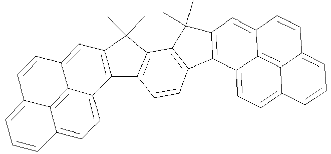 |
| (69) | (70) |
| 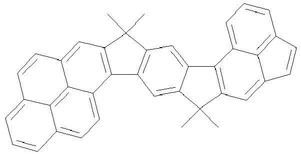 | 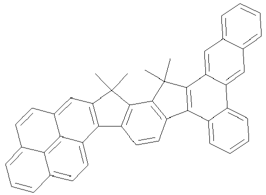 |
| (71) | (72) |
| 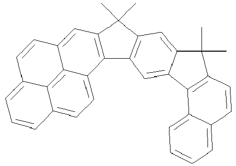 | 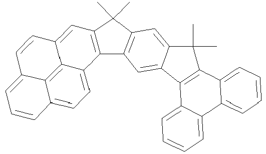 |
| (73) | (74) |
| 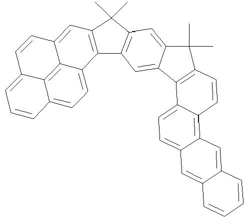 | 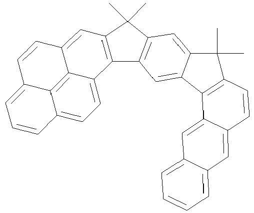 |
| (75) | (76) |
| 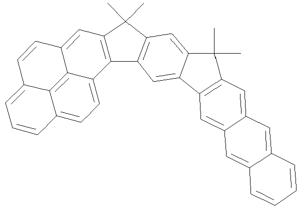 | 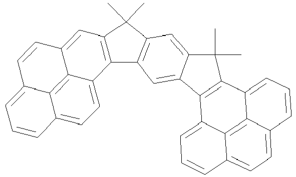 |
| (77) | (78) |
| 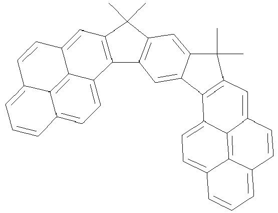 | 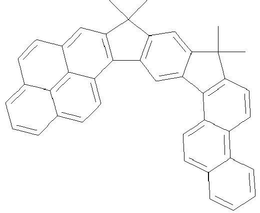 |
| (79) | (80) |
| 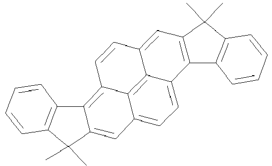 | 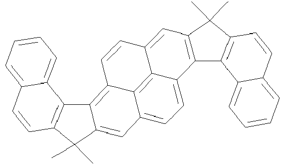 |
| (81) | (82) |
| 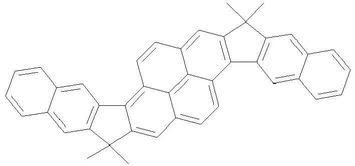 | 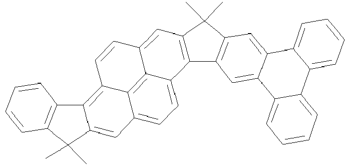 |
| (83) | (84) |
| 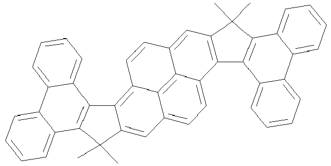 | 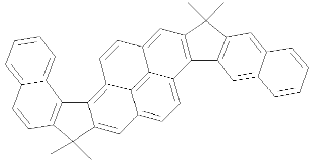 |
| (85) | (86) |
| 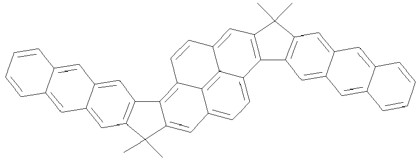 | 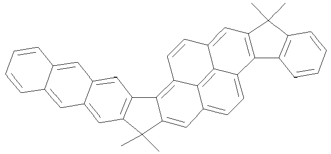 |
| (87) | (88) |
| 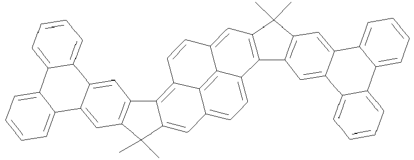 | 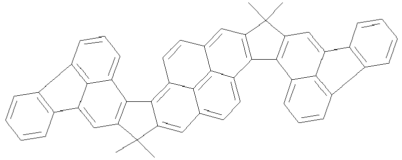 |
| (89) | (90) |
| 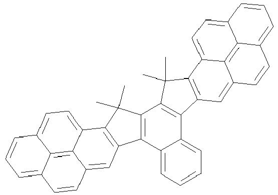 | 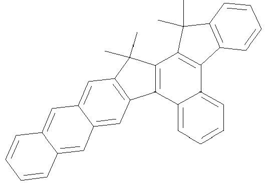 |
| (91) | (92) |
| 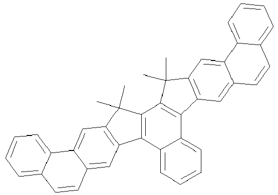 | 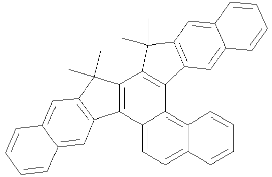 |
| (93) | (94) |
| 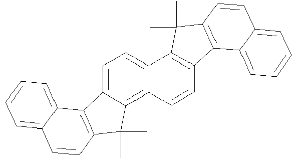 | 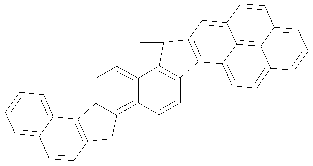 |
| (95) | (96) |
| 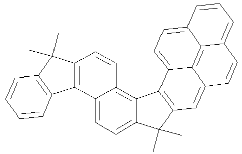 | 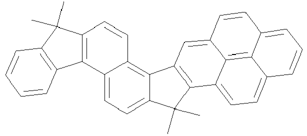 |
| (97) | (98) |
| 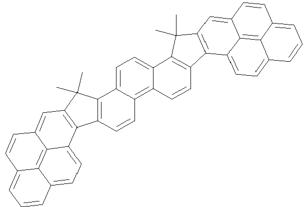 | 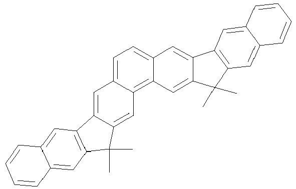 |
| (99) | (100) |
| 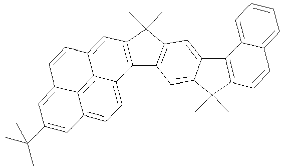 | 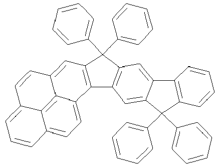 |
| (101) | (102) |
| 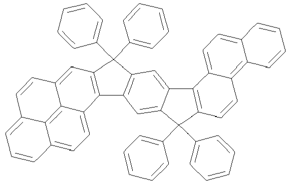 | 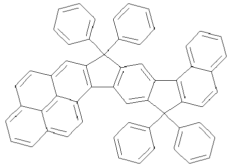 |
| (103) | (104) |
| 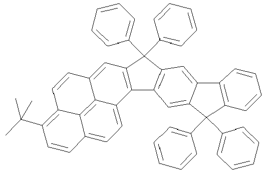 | 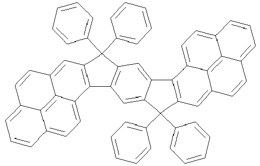 |
| (105) | (106) |
| 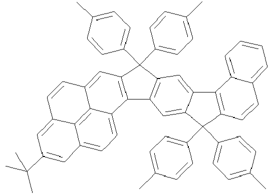 | 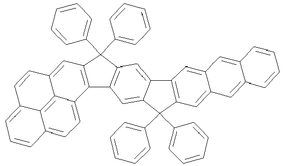 |
| (107) | (108) |
| 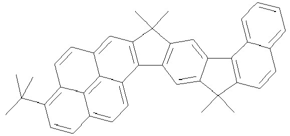 | 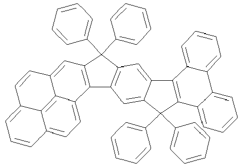 |
| (109) | (110) |
| 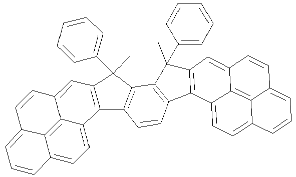 | 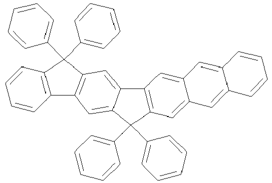 |
| (111) | (112) |
| 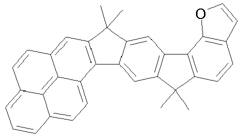 | 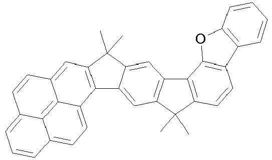 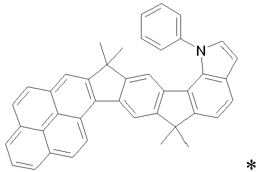 |
| (113) | (114) |
| 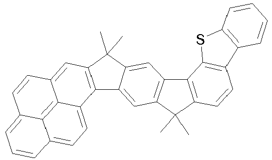 | 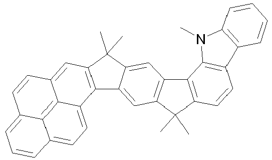 |
| (115)* | (116)* |
| 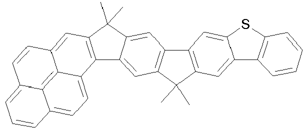 | 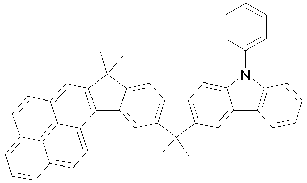 |
| (117)* | (118)* |
| 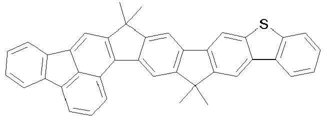 | 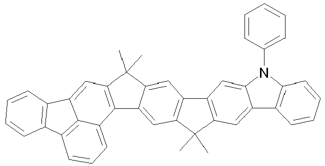 |
| (119)* | (120)* |
| 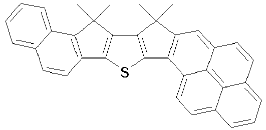 | 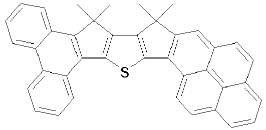 |
| (121)* | (122)* |
| 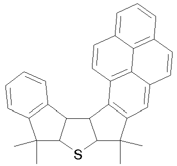 | 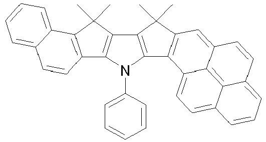 |
| (123)* | (124)* |
| 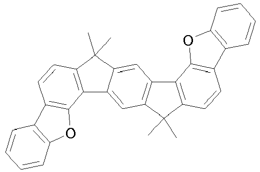 | 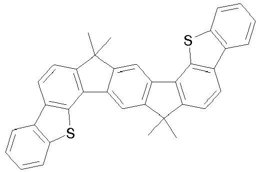 |
| (125)* | (126)* |
| 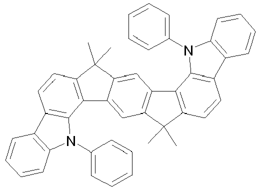 | 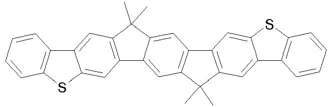 |
| (127)* | (128)* |
| 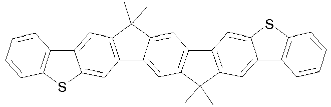 | 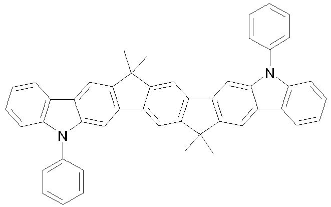 |
| (129)* | (130)* |
| 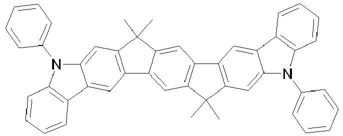 | 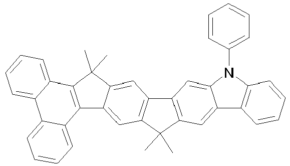 |
| (131)* | (132)* |
| 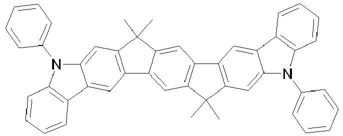 | 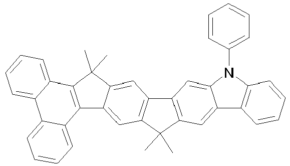 |
| (133)* | (134)* |
| 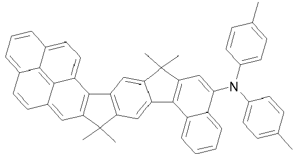 | 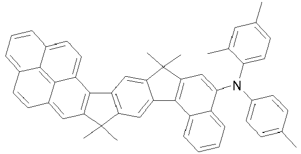 |
| (135)* | (136)* |
| 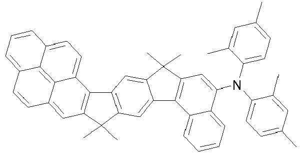 | 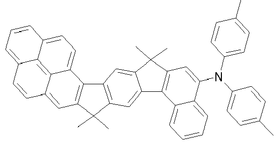 |
| (137)* | (138)* |
| 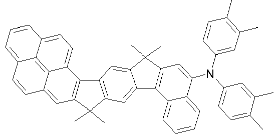 | 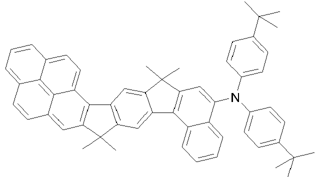 |
| (139)* | (140)* |
| 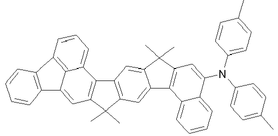 | 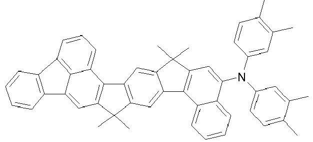 |
| (141)* | (142)* |
| 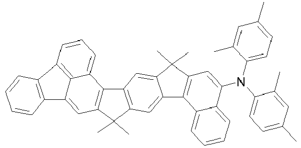 | 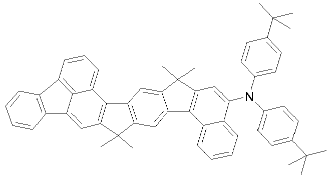 |
| (143)* | (144)* |
| 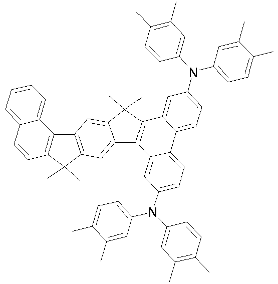 | 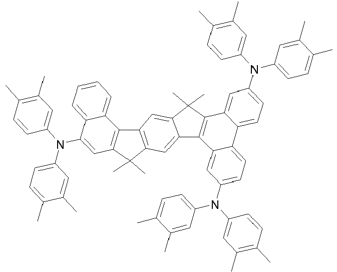 |
| (145)* | (146)* |
| 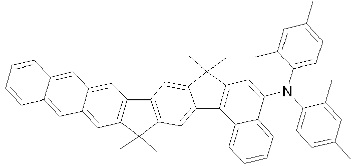 | 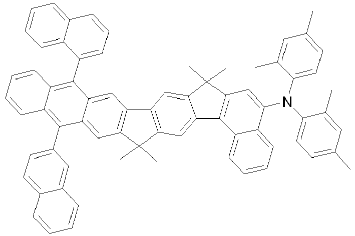 |
| (147)* | (148)* |
| 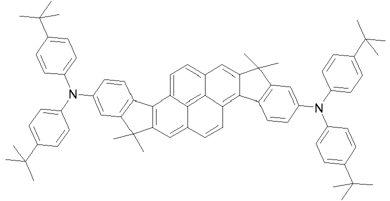 | 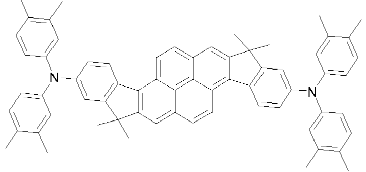 |
| (149)* | (150)* |
| 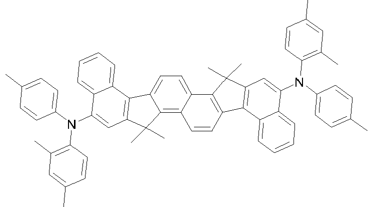 | 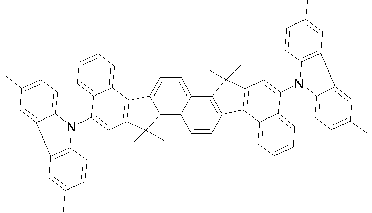 |
| (151)* | (152) |
| 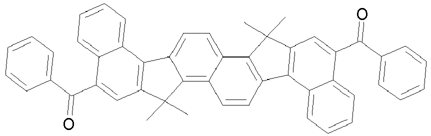 | 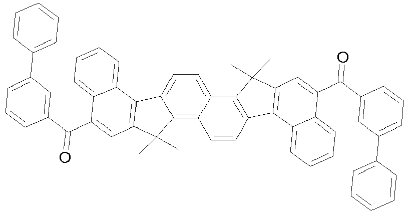 |
| (153)* | (154)* |
| 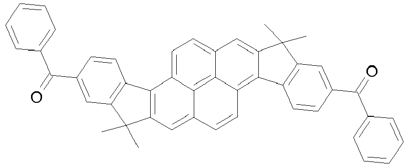 | 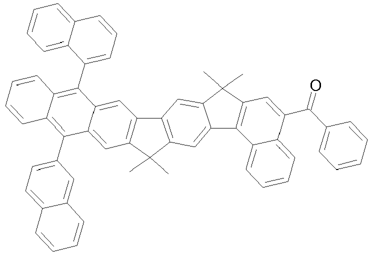 |
| (155)* | (156)* |
| 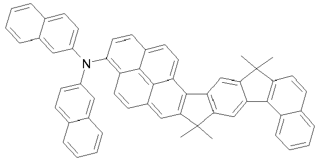 | 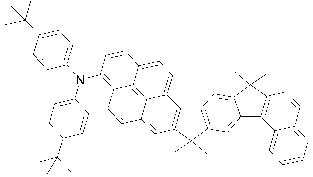 |
| (157)* | (158)* |
| 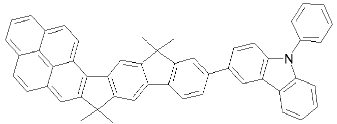 | 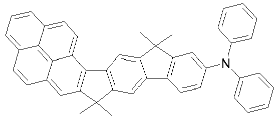 |
| (159) | (160)* |
| 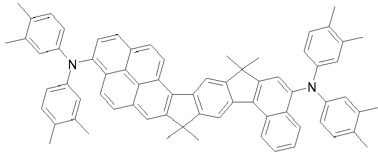 | 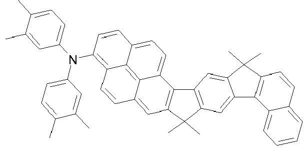 |
| (161)* | (162)* |
| 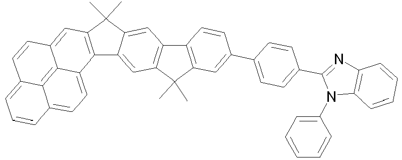 | 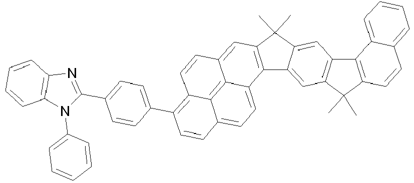 |
| (163) | (164) |
| 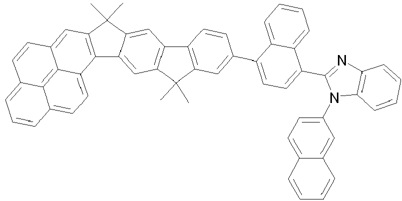 | 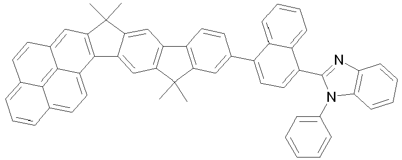 |
| (165) | (166) |
| 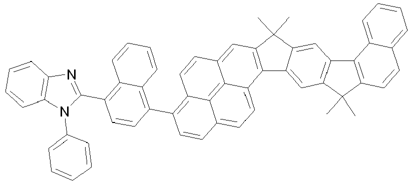 | 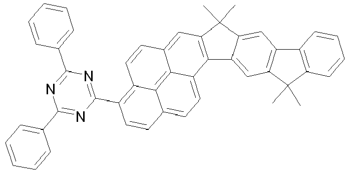 |
| (167) | (168) |
| 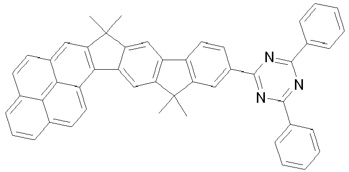 | 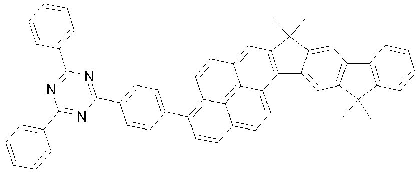 |
| (169) | (170) |
| 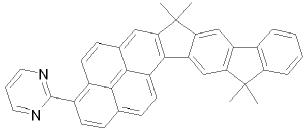 | 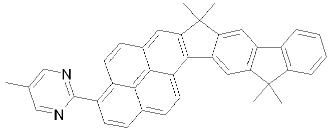 |
| (171) | (172) |
| 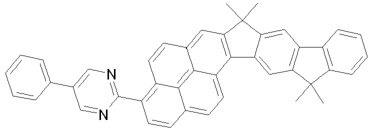 | 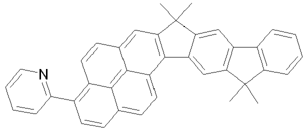 |
| (173) | (174) |
| 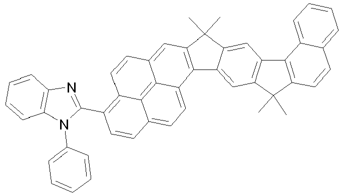 | 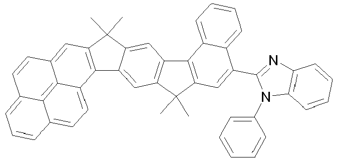 |
| (175) | (176) |
| 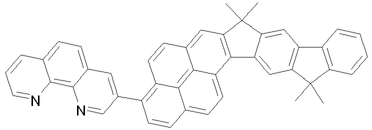 | 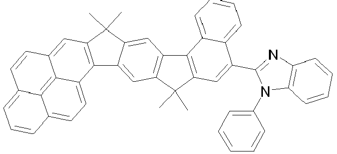 |
| (177) | (178) |
| 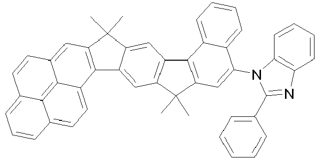 | 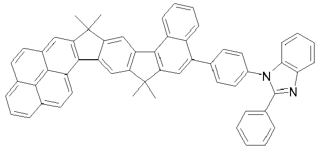 |
| (179) | (180) |
| 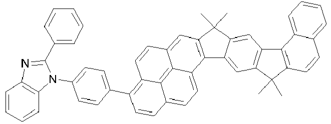 | 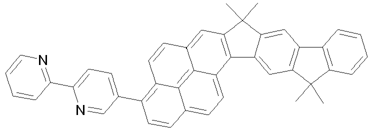 |
| (181) | (182) |
| 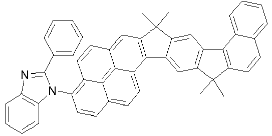 | 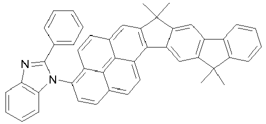 |
| (183) | (184) |
| 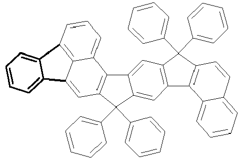 | 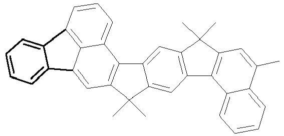 |
| (185) | (186) |
| 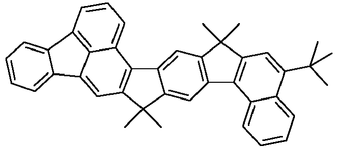 | 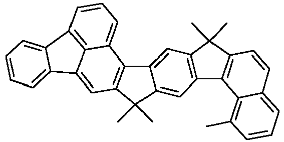 |
| (187) | (188) |
| 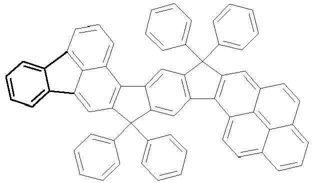 | 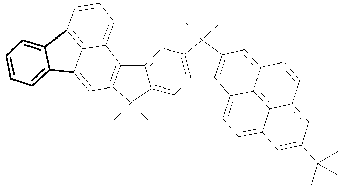 |
| (189) | (190) |
| 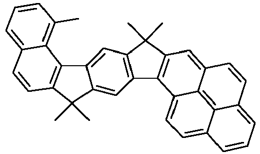 | 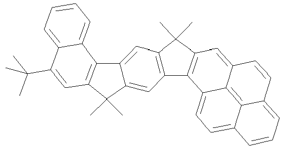 |
| (191) | (192) |
| 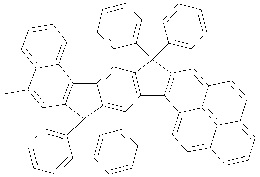 | 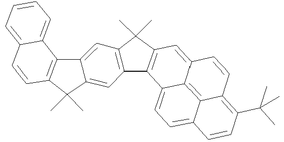 |
| (193) | (194) |
| 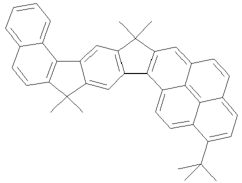 | 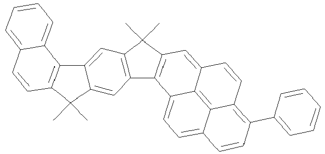 |
| (195) | (196) |
| 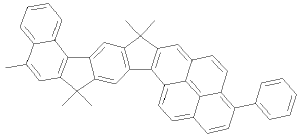 | 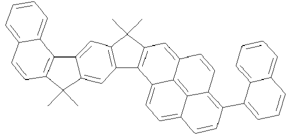 |
| (197) | (198) |
| 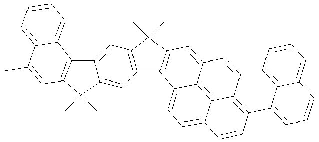 | 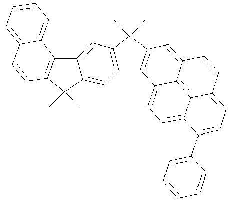 |
| (199) | (200) |
| 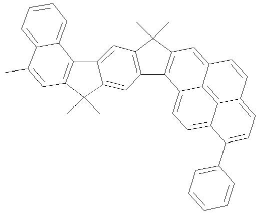 | 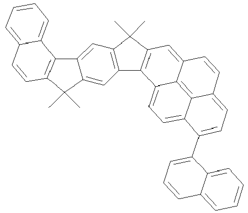 |
| (201) | (202) |
| 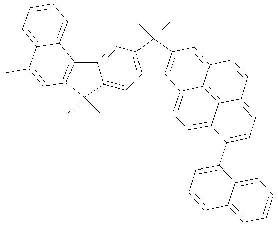 | 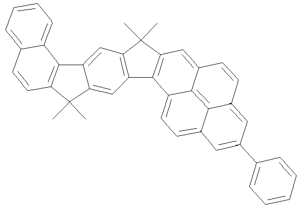 |
| (203) | (204) |
| 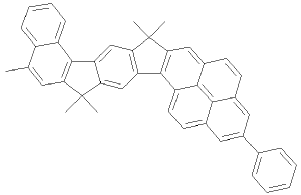 | 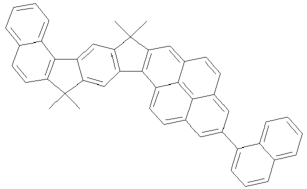 |
| (205) | (206) |
| 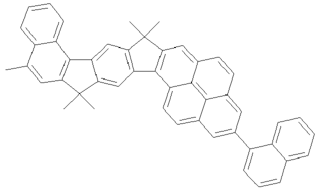 | 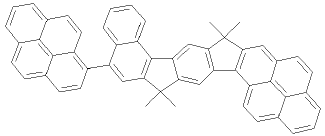 |
| (207) | (208) |
| 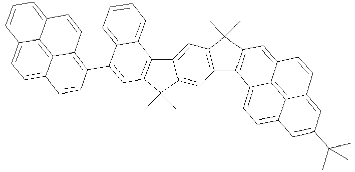 | 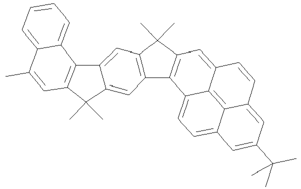 |
| (209) | (210) |
| 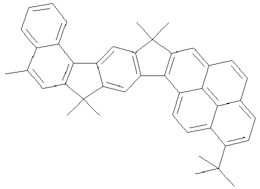 | 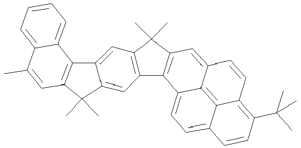 |
| (211) | (212) |
| 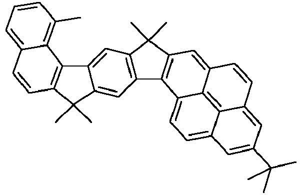 | 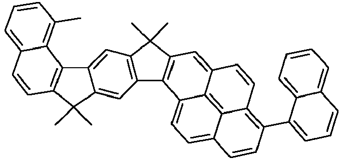 |
| (213) | (214) |
| 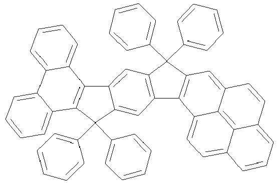 | 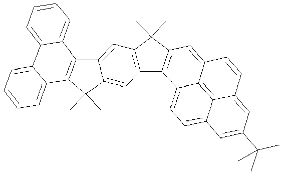 |
| (215) | (216) |
| 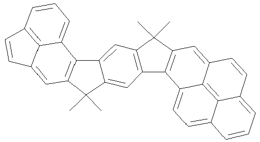 | 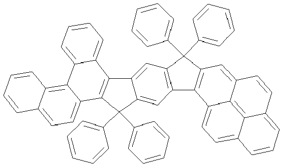 |
| (217) | (218) |
| 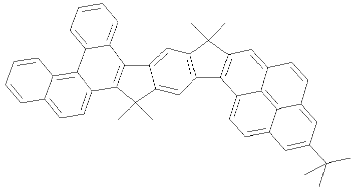 | 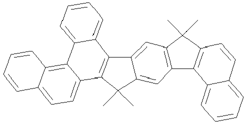 |
| (219) | (220) |
| 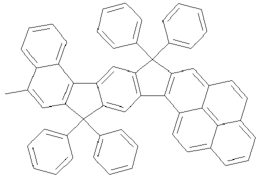 | 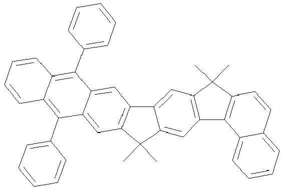 |
| (221) | (222) |
| 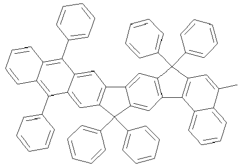 | 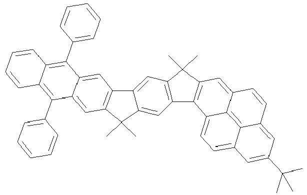 |
| (223) | (224) |
| 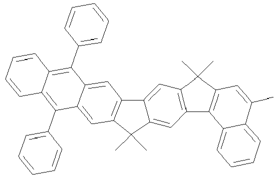 | 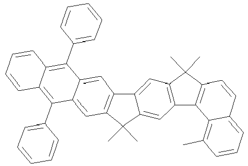 |
| (225) | (226) |
| 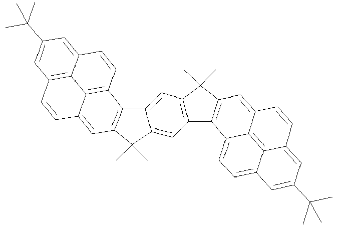 | 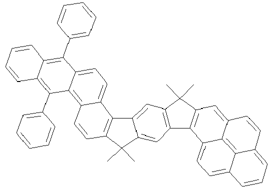 |
| (227) | (228) |
| 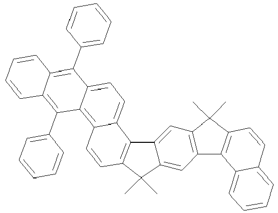 | 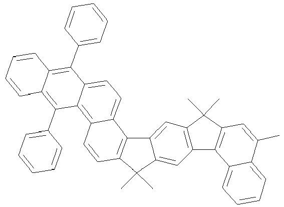 |
| (229) | (230) |
| 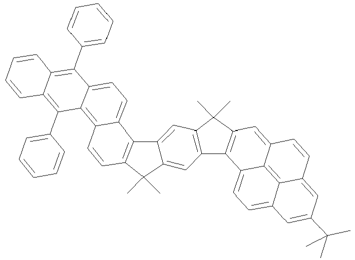 | 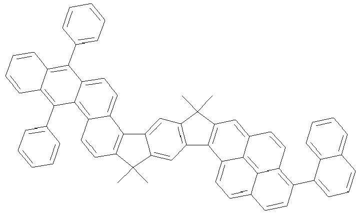 |
| (231) | (232) |
| 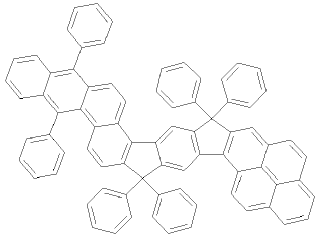 | 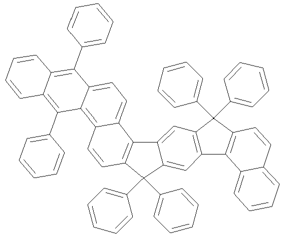 |
| (233) | (234) |
| 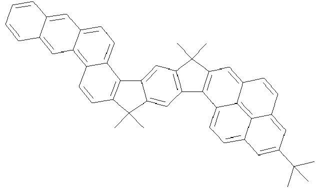 | 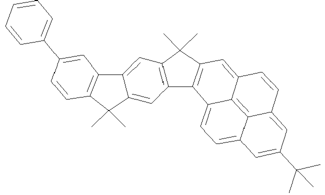 |
| (235) | (236) |
| 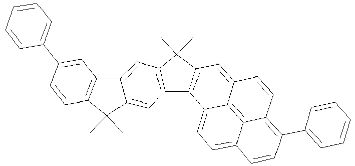 | 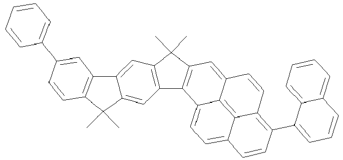 |
| (237) | (238) |
| 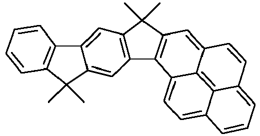 | 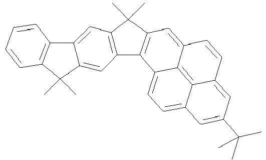 |
| (239) | (240) |
| 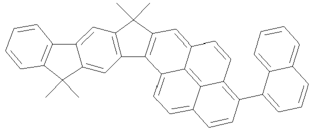 | 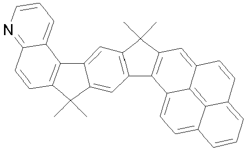 |
| (241) | (242) |
| 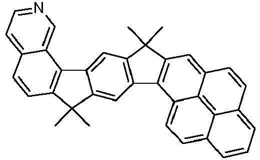 | 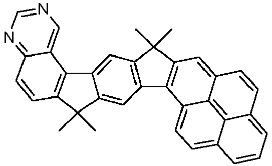 |
| (243) | (244) |
| 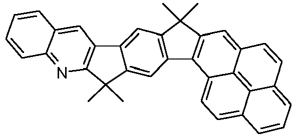 | 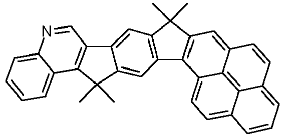 |
| (245) | (246) |

| | |
|---|---|
| **nicht erfindungsgemäß* | |

Die erfindungsgemäßen Verbindungen können nach dem Fachmann bekannten Syntheseschritten, wie z. B. Suzuki-Kupplung und Cyclisierungsreaktionen, dargestellt werden, wie in Schema 1 für Verbindungen gemäß Formel (112) gezeigt. Ganz analog kann die Synthese mit anderen Arylgruppen Ar¹, Ar² und Ar³ durchgeführt werden. Ebenso kann zunächst die Kupplung mit dem Naphthalin und dann die Kupplung mit dem Pyren erfolgen.

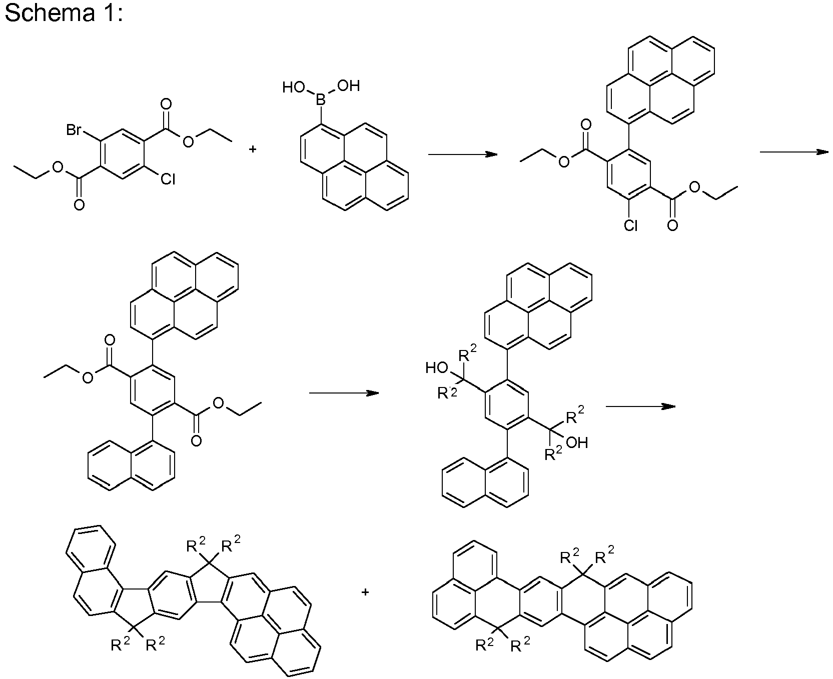

Dazu wird ein Boronsäurederivat des Aromaten Ar¹, in diesem Fall Pyrenboronsäure, mit einem Brom-chlor-bis(carbonsäureester)-Derivat des Aromaten Ar², in diesem Fall 2-Brom-5-chlor-terephthalsäurediethylester, unter Palladiumkatalyse gekuppelt, gefolgt von der Kupplung eines Boronsäurederivats des Aromaten Ar³, in diesem Fall 1-Naphthylboronsäure. Diese selektiven Kupplungsschritte gelingen durch die unterschiedliche Reaktivität von Chlor und Brom. Die Carbonsäureestergruppen werden durch Addition einer Alkyl- oder Arylmetallverbindung, beispielsweise einer Alkyl- oder Aryllithiumverbindung oder einer Alkyl- oder Aryl-Grignardverbindung, zum entsprechenden Alkohol umgesetzt. Dieser kann unter sauren Bedingungen cyclisiert werden, wobei die genauen Reaktionsbedingungen bestimmen, ob ein Fünfring, ein Sechsring oder eine Mischung aus Fünfring und Sechsring gebildet wird. Falls eine Mischung aus Fünfring und Sechsring gebildet wird, kann diese aufgetrennt werden, beispielsweise durch Umkristallisation oder chromatographische Methoden. Die Reaktion ist völlig analog mit anderen Arylboronsäurederivaten und anderen Chlor-brom-dicarbonsäurederivaten möglich. Ebenso können hier bereits substituierte Arylgruppen eingesetzt werden. Durch Einsatz eines 3-Brom-6-chlor-phthalsäureesters lassen sich weiterhin die entsprechenden cis-verknüpften Derivate synthetisieren. Ebenso können statt einer Suzuki-Kupplung andere C-C-Verknüpfungsreaktionen verwendet weren.

Ein weiterer Gegenstand der vorliegenden Erfindung ist daher ein Verfahren zur Herstellung der erfindungsgemäßen Verbindungen, umfassend die Reaktionsschritte:
a) Kupplung von geeignet substituiertem Ar¹, Ar² und Ar³, wobei geeignete Substituenten beispielsweise Carbonestergruppen sein können;und
b) Cylisierung der Substituenten zur Einführung der Brücken X.

Bevorzugt ist ein Verfahren zur Herstellung der Verbindungen gemäß Formel (1), umfassend die Reaktionsschritte:
a) Kupplung einer Boronsäure oder eines Boronsäurederivats von Ar¹ mit einem Brom-chlor-bis(carbonsäureester)-Derivat von Ar²;
b) Kupplung des Reaktionsprodukts aus a) mit einer Boronsäure oder einem Boronsäurederivat von Ar³;
c) Umsetzung der Carbonsäureestergruppen zu Alkoholgruppen; und
d) Cyclisierung unter sauren Bedingungen.

Die oben beschriebenen erfindungsgemäßen Verbindungen, insbesondere Verbindungen, welche mit reaktiven Abgangsgruppen, wie Brom, lod, Boronsäure oder Boronsäureester substituiert sind, können auch als Comonomere zur Erzeugung entsprechender konjugierter, teilkonjugierter oder nicht-konjugierter Polymere, Oligomere oder auch als Kern von Dendrimeren Verwendung finden. Die Polymerisation erfolgt dabei bevorzugt über die Halogenfunktionalität bzw. die Boronsäurefunktionalität.

Weiterer Gegenstand der Erfindung sind somit Polymere, Oligomere oder Dendrimere enthaltend eine oder mehrere erfindungsgemäße Verbindungen, wobei ein oder mehrere Reste R¹ oder R² Bindungen der Verbindung zum Polymer oder Dendrimer darstellen. Diese Polymere, Oligomere oder Dendrimere können konjugiert, teilkonjugiert oder nichtkonjugiert sein.

Für die erfindungsgemäßen Wiederholeinheiten in den Polymeren gelten dieselben Bevorzugungen wie oben beschrieben.

Diese Verbindungen werden homopolymerisiert oder mit weiteren Monomeren copolymerisiert. Geeignete und bevorzugte Comonomere sind gewählt aus Fluorenen (z. B. gemäß EP 842208 oder WO 00/22026), Spirobifluorenen (z. B. gemäß EP 707020, EP 894107 oder WO 06/061181), Para-phenylenen (z. B. gemäß WO 92/18552), Carbazolen (z. B. gemäß WO 04/070772 oder WO 04/113468), Thiophenen (z. B. gemäß EP 1028136), Dihydrophenanthrenen (z. B. gemäß WO 05/014689), cis- und trans-Indenofluorenen (z. B. gemäß WO 04/041901 oder WO 04/113412), Ketonen (z. B. gemäß WO 05/040302), Phenanthrenen (z. B. gemäß WO 05/104264 oder der nicht offen gelegten Anmeldung DE 102005037734.3) oder auch mehreren dieser Einheiten. Diese Polymere enthalten üblicherweise noch weitere Einheiten, beispielsweise emittierende (fluoreszierende oder phosphoreszierende) Einheiten, wie z. B. Vinyltriarylamine (z. B. gemäß der nicht offen gelegten Anmeldung DE 102005060473.0) oder phosphoreszierende Metallkomplexe (z. B. gemäß WO 06/003000), und/oder Ladungstransporteinheiten, insbesondere solche basierend auf Triarylaminen.

Die erfindungsgemäßen Verbindungen gemäß Formel (1) bzw. die entsprechenden Oligomere, Polymere oder Dendrimere eignen sich für den Einsatz in elektronischen Vorrichtungen, insbesondere in organischen Elektrolumineszenzvorrichtungen (OLEDs, PLEDs). Abhängig von der Struktur werden die Verbindungen in unterschiedlichen Funktionen und Schichten eingesetzt. Die genaue Verwendung der Verbindungen hängt dabei insbesondere von der Wahl der Arylgruppen Ar¹, Ar² und Ar³ und von den Gruppen X ab.

Ein weiterer Gegenstand der Erfindung ist daher die Verwendung der erfindungsgemäßen Verbindungen gemäß Formel (1) bzw. der entsprechenden Oligomere, Polymere oder Dendrimere in elektronischen Vorrichtungen, insbesondere in organischen Elektrolumineszenzvorrichtungen (OLEDs), organischen Feld-Effekt-Transistoren (O-FETs), organischen Dünnfilmtransistoren (O-TFTs), organischen lichtemittierenden Transistoren (O-LETs), organischen integrierten Schaltungen (O-ICs), organischen Solarzellen (O-SCs), organischen Feld-Quench-Devices (O-FQDs), lichtemittierenden elektrochemischen Zellen (LECs), organischen Photorezeptoren oder organischen Laserdioden (O-Laser).

Nochmals ein weiterer Gegenstand der Erfindung sind elektronische Vorrichtungen, insbesondere die oben genannten elektronischen Vorrichtungen, enthaltend mindestens eine Verbindung gemäß Formel (1) bzw. ein entsprechendes Oligomer, Polymer oder Dendrimer, insbesondere organische Elektrolumineszenzvorrichtungen, enthaltend Anode, Kathode und mindestens eine emittierende Schicht, dadurch gekennzeichnet, dass mindestens eine organische Schicht, die eine emittierende Schicht oder eine andere Schicht sein kann, mindestens eine Verbindung gemäß Formel (1) enthält.

Dabei gelten für die Verwendung in der elektronischen Vorrichtung die oben genannten bevorzugten Ausführungsformen.

Außer Kathode, Anode und der emittierenden Schicht kann die organische Elektrolumineszenzvorrichtung noch weitere Schichten enthalten. Diese sind beispielsweise gewählt aus jeweils einer oder mehreren Lochinjektionsschichten, Lochtransportschichten, Lochblockierschichten, Elektronentransportschichten, Elektroneninjektionsschichten, Elektronenblockierschichten, Excitonenblockierschichten, Ladungserzeugungsschichten (Charge-Generation Layers) und/oder organischen oder anorganischen p/n-Übergängen. Es sei aber darauf hingewiesen, dass nicht notwendigerweise jede dieser Schichten vorhanden sein muss und die Wahl der Schichten immer von den verwendeten Verbindungen abhängt und insbesondere auch von der Tatsache, ob es sich um eine fluoreszierende oder phosphoreszierende Elektrolumineszenzvorrichtung handelt.

Die organische Elektrolumineszenzvorrichtung kann auch mehrere emittierende Schichten enthalten, wobei mindestens eine organische Schicht mindestens eine Verbindung gemäß Formel (1) bzw. ein entsprechendes Oligomer, Polymer oder Dendrimer enthält. Besonders bevorzugt weisen diese Emissionsschichten insgesamt mehrere Emissionsmaxima zwischen 380 nm und 750 nm auf, so dass insgesamt weiße Emission resultiert, d. h. in den emittierenden Schichten werden verschiedene emittierende Verbindungen verwendet, die fluoreszieren oder phosphoreszieren können und die blaues und gelbes, orange oder rotes Licht emittieren. Insbesondere bevorzugt sind Dreischichtsysteme, also Systeme mit drei emittierenden Schichten, wobei mindestens eine dieser Schichten mindestens eine Verbindung gemäß Formel (1) bzw. ein entsprechendes Oligomer, Polymer oder Dendrimer enthält und wobei die drei Schichten blaue, grüne und orange oder rote Emission zeigen (für den prinzipiellen Aufbau siehe z. B. WO 05/011013) und Systeme, welche mehr als drei emittierende Schichten aufweisen. Ebenso eignen sich für weiße Emission Emitter, welche breitbandige Emissionsbanden aufweisen und dadurch weiße Emission zeigen.

Es ist insbesondere bevorzugt, wenn die Verbindungen gemäß Formel (1) in einer emittierenden Schicht eingesetzt werden. Dabei können sie entweder als emittierendes Material (emittierender Dotand) oder als Hostmaterial für ein emittierendes Material eingesetzt werden. Besonders bevorzugt eignen sich die Verbindungen gemäß Formel (1) als emittierendes Material.

Wenn die Verbindung gemäß Formel (1) als emittierendes Material in einer emittierenden Schicht eingesetzt wird, wird sie bevorzugt in Kombination mit einem Hostmaterial eingesetzt. Unter einem Hostmaterial wird in einem System aus Host und Dotand diejenige Komponente verstanden, die in dem System im höheren Anteil vorliegt. Bei einem System aus einem Host und mehreren Dotanden wird als Host diejenige Komponente verstanden, deren Anteil der höchste in der Mischung ist.

Der Anteil der Verbindung gemäß Formel (1) in der Mischung der emittierenden Schicht beträgt zwischen 0.1 und 50.0 Vol.-%, bevorzugt zwischen 0.5 und 20.0 Vol.-%, besonders bevorzugt zwischen 1.0 und 10.0 Vol.-%. Entsprechend beträgt der Anteil des Hostmaterials zwischen 50.0 und 99.9 Vol.-%, bevorzugt zwischen 80.0 und 99.5 Vol.-%, besonders bevorzugt zwischen 90.0 und 99.0 Vol.-%.

Als Hostmaterialien kommen hierfür Materialien verschiedener Stoffklassen in Frage. Bevorzugte Hostmaterialien sind ausgewählt aus den Klassen der Oligoarylene (z. B. 2,2',7,7'-Tetraphenylspirobifluoren gemäß EP 676461 oder Dinaphthylanthracen), insbesondere der Oligoarylene enthaltend kondensierte aromatische Gruppen, der Oligoarylenvinylene (z. B. DPVBi oder Spiro-DPVBi gemäß EP 676461), der polypodalen Metallkomplexe (z. B. gemäß WO 04/081017), der lochleitenden Verbindungen (z. B. gemäß WO 04/058911), der elektronenleitenden Verbindungen, insbesondere Ketone, Phosphinoxide, Sulfoxide, etc. (z. B. gemäß WO 05/084081 und WO 05/084082), der Atropisomere (z. B. gemäß WO 06/048268), der Boronsäurederivate (z. B. gemäß

WO 06/117052) oder der Benzanthracene (z. B. gemäß der nicht offen gelegten Anmeldung DE 102007024850.6). Besonders bevorzugte Hostmaterialien sind ausgewählt aus den Klassen der Oligoarylene, enthaltend Naphthalin, Anthracen, Benzanthracen und/oder Pyren oder Atropisomere dieser Verbindungen, der Ketone, der Phosphinoxide und der Sulfoxide. Ganz besonders bevorzugte Hostmaterialien sind ausgewählt aus den Klassen der Oligoarylene, enthaltend Anthracen, Benzanthracen und/oder Pyren oder Atropisomere dieser Verbindungen. Unter einem Oligoarylen im Sinne dieser Erfindung soll eine Verbindung verstanden werden, in der mindestens drei Aryl- bzw. Arylengruppen aneinander gebunden sind.

Bevorzugte Hostmaterialien sind insbesondere ausgewählt aus Verbindungen der Formel (138),

Ar⁴-(Ar⁵)ₚ-Ar⁶ Formel (138)

wobei Ar⁴, Ar⁵, Ar⁶ bei jedem Auftreten gleich oder verschieden eine Aryl- oder Heteroarylgruppe mit 5 bis 30 aromatischen Ringatomen ist, die mit einem oder mehreren Resten R¹ substituiert sein kann, und R¹ und p dieselbe Bedeutung haben, wie oben beschrieben; dabei gilt, dass die Summe der π-Elektronen in Ar⁴, Ar⁵ und Ar⁶ mindestens 30 beträgt, wenn p = 1 ist, und mindestens 36 beträgt, wenn p = 2 ist, und mindestens 42 beträgt, wenn p = 3 ist.

Besonders bevorzugt steht in den Hostmaterialien der Formel (138) die Gruppe Ar⁵ für Anthracen, welches durch einen oder mehrere Reste R¹ substituiert sein kann, und die Gruppen Ar⁴ und Ar⁶ sind in 9- und 10-Position gebunden. Ganz besonders bevorzugt ist mindestens eine der Gruppen Ar⁴ und/oder Ar⁶ eine kondensierte Arylgruppe, ausgewählt aus 1- oder 2-Naphthyl, 2-, 3- oder 9-Phenanthrenyl oder 2-, 3-, 4-, 5-, 6- oder 7-Benzanthracenyl, welche jeweils durch einen oder mehrere Reste R¹ substituiert sein kann.

Es ist weiterhin bevorzugt, wenn die Verbindung gemäß Formel (1) als Hostmaterial, insbesondere für einen fluoreszierenden Dotanden eingesetzt wird.

Geeignete fluoreszierende Emitter sind beispielsweise gewählt aus der Gruppe der Monostyrylamine, der Distyrylamine, der Tristyrylamine, der Tetrastyrylamine, der Styrylphosphine, der Styrylether und der Arylamine. Unter einem Monostyrylamin wird eine Verbindung verstanden, die eine substituierte oder unsubstituierte Styrylgruppe und mindestens ein, bevorzugt aromatisches, Amin enthält. Unter einem Distyrylamin wird eine Verbindung verstanden, die zwei substituierte oder unsubstituierte Styrylgruppen und mindestens ein, bevorzugt aromatisches, Amin enthält. Unter einem Tristyrylamin wird eine Verbindung verstanden, die drei substituierte oder unsubstituierte Styrylgruppen und mindestens ein, bevorzugt aromatisches, Amin enthält. Unter einem Tetrastyrylamin wird eine Verbindung verstanden, die vier substituierte oder unsubstituierte Styrylgruppen und mindestens ein, bevorzugt aromatisches, Amin enthält. Die Styrylgruppen sind besonders bevorzugt Stilbene, die auch noch weiter substituiert sein können. Entsprechende Phosphine und Ether sind in Analogie zu den Aminen definiert. Unter einem Arylamin bzw. einem aromatischen Amin im Sinne dieser Erfindung wird eine Verbindung verstanden, die drei substituierte oder unsubstituierte aromatische oder heteroaromatische Ringsysteme direkt an den Stickstoff gebunden enthält. Bevorzugt ist mindestens eines dieser aromatischen oder heteroaromatischen Ringsysteme ein kondensiertes Ringsystem, besonders bevorzugt mit mindestens 14 aromatischen Ringatomen. Bevorzugte Beispiele hierfür sind aromatische Anthracenamine, aromatische Pyrenamine, aromatische Pyrendiamine, aromatische Chrysenamine oder aromatische Chrysendiamine. Unter einem aromatischen Anthracenamin wird eine Verbindung verstanden, in der eine Diarylaminogruppe direkt an eine Anthracengruppe gebunden ist, vorzugsweise in 9-Position oder in 2-Position. Aromatische Pyrenamine, Pyrendiamine, Chrysenamine und Chrysendiamine sind analog dazu definiert, wobei die Diarylaminogruppen am Pyren bevorzugt in 1-Position bzw. in 1,6-Position gebunden sind. Weitere bevorzugte Dotanden sind gewählt aus Indenofluorenaminen bzw. -diaminen, beispielsweise gemäß WO 06/122630, Benzoindenofluorenaminen bzw. -diaminen, beispielsweise gemäß WO 08/006449, und Dibenzoindenofluorenaminen bzw. -diaminen, beispielsweise gemäß WO 07/140847. Beispiele für Dotanden aus der Klasse der Styrylamine sind substituierte oder unsubstituierte Tristilbenamine oder die Dotanden, die in WO 06/000388, WO 06/058737, WO 06/000389, WO 07/065549 und WO 07/115610 beschrieben sind.

Je nach Substitutionsmuster können die Verbindungen gemäß Formel (1) auch in anderen Schichten eingesetzt werden.

Eine mögliche weitere Verwendung von Verbindungen gemäß Formel (1) ist die Verwendung als Lochtransport- oder Lochinjektionsmaterial in einer Lochtransport- oder einer Lochinjektionsschicht. Diese Verwendung eignet sich insbesondere dann, wenn eine oder mehrere Brücken X für S oder NR² stehen.

Eine weitere mögliche Verwendung von Verbindungen gemäß Formel (1) ist die Verwendung als Elektronentransportmaterial in einer Elektronentransportschicht. Hierfür eignen sich insbesondere Verbindungen gemäß Formel (1), welche mit mindestens einem elektronenarmen Heteroaromaten substituiert sind. Elektronenarme Heteroaromaten sind 6-Ring-Heteroaromaten mit mindestens einem Stickstoffatom und entsprechende kondensierte Systeme, beispielsweise Pyridin, Pyrazin, Pyrimidin, Pyridazin, Triazin, Chinolin, Chinoxalin oder Phenanthrolin, oder 5-Ring-Heteroaromaten mit mindestens einem Stickstoffatom und einem weiteren Heteroatom, ausgewählt aus N, O oder S, und entsprechende kondensierte Systeme, beispielsweise Pyrazol, Imidazol, Oxazol, Oxadiazol oder Benzimidazol. Weiterhin eignen sich als Elektronentransportmaterial Verbindungen, in denen Ar¹, Ar² und/oder Ar³ für einen elektronenarmen Heterocyclus stehen.

Weiterhin bevorzugt ist eine organische Elektrolumineszenzvorrichtung, dadurch gekennzeichnet, dass eine oder mehrere Schichten mit einem Sublimationsverfahren beschichtet werden. Dabei werden die Materialien in Vakuum-Sublimationsanlagen bei einem Anfangsdruck kleiner 10⁻⁵ mbar, bevorzugt kleiner 10⁻⁶ mbar aufgedampft. Es ist aber auch möglich, dass der Anfangsdruck noch geringer ist, beispielsweise kleiner 10⁻⁷ mbar.

Bevorzugt ist ebenfalls eine organische Elektrolumineszenzvorrichtung, dadurch gekennzeichnet, dass eine oder mehrere Schichten mit dem OVPD (Organic Vapour Phase Deposition) Verfahren oder mit Hilfe einer Trägergassublimation beschichtet werden. Dabei werden die Materialien bei einem Druck zwischen 10⁻⁵ mbar und 1 bar aufgebracht. Ein Spezialfall dieses Verfahrens ist das OVJP (Organic Vapour Jet Printing) Verfahren, bei dem die Materialien direkt durch eine Düse aufgebracht und so strukturiert werden (z. B. M. S. Arnold et al., Appl. Phys. Lett. 2008, 92, 053301).

Weiterhin bevorzugt ist eine organische Elektrolumineszenzvorrichtung, dadurch gekennzeichnet, dass eine oder mehrere Schichten aus Lösung, wie z. B. durch Spincoating, oder mit einem beliebigen Druckverfahren, wie z. B. Siebdruck, Flexodruck oder Offsetdruck, besonders bevorzugt aber LITI (Light Induced Thermal Imaging, Thermotransferdruck) oder Ink-Jet Druck (Tintenstrahldruck), hergestellt werden. Hierfür sind lösliche Verbindungen gemäß Formel (1) nötig. Hohe Löslichkeit lässt sich durch geeignete Substitution der Verbindungen erreichen. Insbesondere auch für Oligomere, Polymere oder Dendrimere eignet sich ein Bechichtungsverfahren aus Lösung.

Zum Aufbringen aus Lösung sind Lösungen der erfindungsgemäßen Verbindungen in einem oder mehreren Lösemitteln erforderlich. Ein weiterer Gegenstand sind daher Lösungen der erfindungsgemäßen Verbindungen bzw. entsprechenden Oligomere, Polymere oder Dendrimere in einem oder mehreren Lösemitteln. Die Lösung kann dabei auch weitere Bestandteile enthalten, beispielsweise ein Hostmaterial für die erfindungsgemäße Verbindung.

Die erfindungsgemäßen Verbindungen weisen bei Verwendung in organischen Elektrolumineszenzvorrichtungen folgende überraschende Vorteile gegenüber dem Stand der Technik auf:
1. Die erfindungsgemäßen Verbindungen zeigen bei Verwendung als emittierende Materialien in organischen Elektrolumineszenzvorrichtungen tiefblaue Emission (CIE y im Bereich von 0.10 bis 0.13) und eignen sich somit hervorragend zur Herstellung tiefblau emittierender Elektrolumineszenzvorrichtungen.
2. Durch geeignete Wahl der Gruppen Ar¹, Ar² und Ar³ lässt sich mit den erfindungsgemäßen Verbindungen einfach der Farbort der Emission der Verbindung einstellen. Es sind so sowohl tiefblau wie auch heller blau emittierende Verbindungen zugänglich, wobei der Farbort jeweils für die gewünschte Verwendung optimiert werden kann.
3. Die Elektrolumineszenzvorrichtungen zeigen weiterhin sehr gute Effizienzen (EQE > 6 %).
4. Weiterhin zeigen Elektrolumineszenzvorrichtungen mit den erfindungsgemäßen Verbindungen eine deutliche Verbesserung in Bezug auf die Lebensdauer.
5. Insbesondere bei Verwendung von dotierten Elektronentransportmaterialien in der Elektroneninjektions- und -transportschicht, welche zu einem Elektronenüberschuss im Device führen, zeigen die erfindungsgemäßen Verbindungen, wenn sie als Emitter eingesetzt werden, deutliche Verbesserungen in Bezug auf Effizienz und Lebensdauer im Vergleich zu Emittern gemäß dem Stand der Technik, welche Diarylaminogruppen enthalten. Dies ist ein wesentlicher Vorteil, da gerade die Kombination von LiQ mit Benzimidazolderivaten häufig als Elektronentransportmaterial verwendet wird.

Die Erfindung wird durch die nachfolgenden Beispiele genauer beschrieben, ohne sie dadurch einschränken zu wollen. Der Fachmann kann, ohne erfinderisch tätig zu werden, die Erfindung im gesamten offenbarten Bereich ausführen und so weitere erfindungsgemäße Materialien und organische Elektrolumineszenzvorrichtungen herstellen.

### Beispiele:

Die nachfolgenden Synthesen wurden, sofern nicht anders angegeben, unter einer Schutzgasatmosphäre durchgeführt. Die Edukte wurden von den Firmen ALDRICH bzw. ABCR bezogen.

### Beispiel 1: Synthese von 1,1-Dimethylbenzindeno-1,1-dimethyl-indeno-[a]pyren

### a) 2-Chlor-5-pyren-1-yl-terephthalsäurediethylester

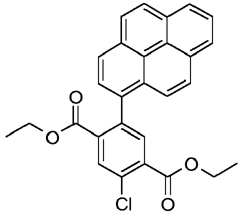

28.9 g (103 mmol) Brompyren werden in 275 mL trockenem THF gelöst, auf -75 °C abgekühlt und bei dieser Temperatur 52 mL (104 mmol) einer 2 M Lösung von n-Buthyllithium zugetropft. Die gelbe Suspension wird 1 h bei -75 °C gerührt und dann 17.5 mL (155 mmol) Trimethylborat zugetropft. Nach Erwärmen auf RT werden 34.5 g (103 mmol) Chlorbromterephthalsäurediethylester, 22 g (206 mmol) Na₂CO₃, 1.2 g (1.03 mmol) Tetrakis(triphenylphosphin)-palladium(0), 140 mL H₂O, 280 mL Toluol und 140 mL EtOH zugegeben und das Gemisch für 2 h zum Sieden erhitzt. Nach Abtrennen der organischen Phase, zweimaligem Waschen mit Wasser und Trocknen über Na₂SO₄ wird das Lösungsmittel im Vakuum entfernt und das verbleibende Öl in Heptan zur Kristallisation gebracht. Zweimalige Umkristallisation liefert das Produkt in Form eines farblosen Feststoffes (33 g, 70 %) und einer Reinheit von >98 %, was in dieser Form in die Folgereaktion eingesetzt wird.

### b) 2-Naphthalin-1-yl-5-pyren-1-yl-terephthalsäurediethylester

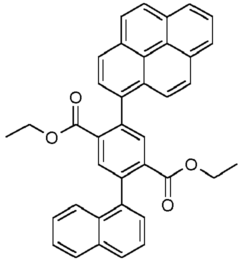

43.5 g (90 mmol) 2-Chlor-5-pyren-1-yl-terephthalsäurediethylester, 21.5 g (120 mmol) 1-Naphthylboronsäure und 58.1 g CS₂CO₃ werden in 230 mL trockenem Dioxan vorgelegt und 30 min mit N₂ gesättigt. Danach erfolgt Zugabe von 2.7 mL einer 1.0 M Lösung von Tri-*tert*-butylphosphin in Toluol, gefolgt von 300 mg (1.3 mmol) Pd(OAc)₂. Das Gemisch wird für 4 h zum Sieden erhitzt, mit Wasser und EtOH erweitert, der Niederschlag abgesaugt, mit Wasser und EtOH gewaschen und getrocknet. Der Feststoff wird dreimal aus Dioxan umkristallisiert und weist danach gemäss ¹H-NMR eine Reinheit von >99% auf. Die Ausbeute beträgt 44,2 g (90%) an farblosem Feststoff.

In Analogie zum oben beschriebenen Verfahren werden folgende Verbindungen hergestellt (Beispiele 2b bis 10b).

| Bsp. | Struktur | Ausbeute (%) |
|---|---|---|
| 2b | 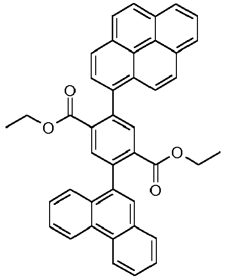 | 95 |
| 3b | 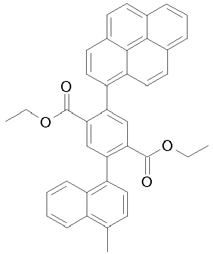 | 50 |
| 4b | 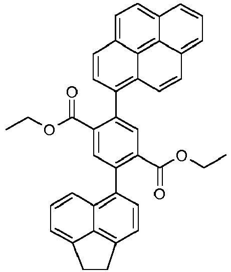 | 53 |
| 5b | 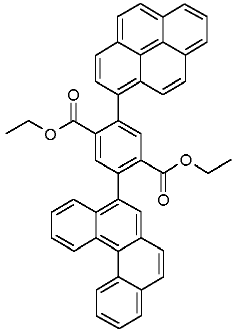 | 67 |
| 6b | 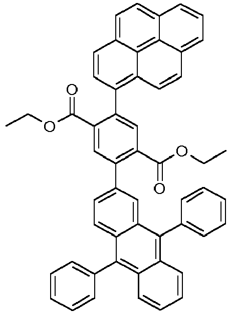 | 12 |
| 7b | 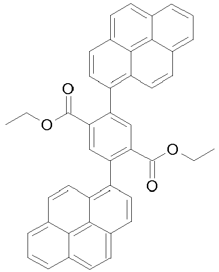 | 36 |
| 8b | 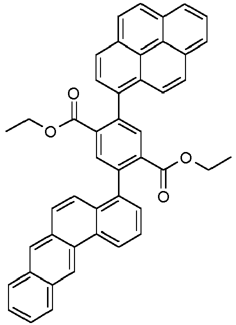 | 50 |
| 9b | 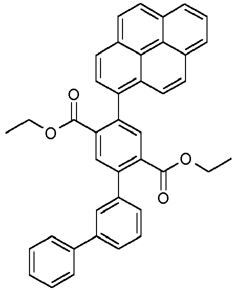 | 62 |
| 10b | 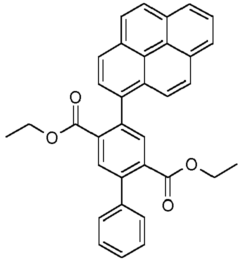 | 75 |

### c) 2-[4-(1-Hydroxy-1-methyl-ethyl)-2-naphthalin-1-yl-5-pyren-1-yl-phenyl]-propan-2-ol

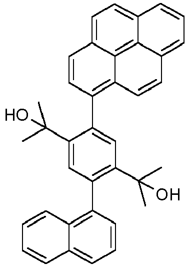

30 g (55 mol) 2-Naphthalin-1-yl-5-pyren-1-yl-terephthalsäurediethylester werden in 270 mL trockenem THF gelöst, bei 5 °C 110 mL (330 mmol) einer 3 M Methylmagnesiumchlorid Lösung in THF zugetropft und das Gemisch für 12 h bei RT gerührt. Nach Unterbrechung der Reaktion durch Zugabe von 180 mL 25 %iger Essigsäure wird extraktiv mit Essigsäureethylester/Wasser aufgearbeitet, über Na₂SO₄ getrocknet und einrotiert. Nach Umkristallisation aus EtOH/Toluol verbleiben 26.3 g (92 %) farbloser Feststoff, der nach ¹H-NMR eine Reinheit von >98 % aufweist.

In Analogie zum oben beschriebenen Verfahren werden folgende Verbindungen hergestellt (Beispiele 2c bis 9c). In Beispiel 10c wird anstelle von Methylmagnesiumchlorid Phenyllithium als Reagenz eingesetzt.

| Bsp. | Struktur | Ausbeute (%) |
|---|---|---|
| 2c | 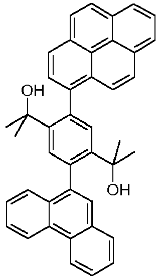 | 92 |
| 3c | 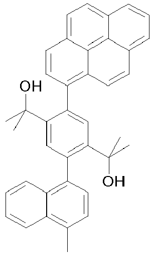 | 78 |
| 4c | 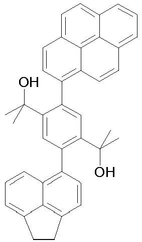 | 94 |
| 5c | 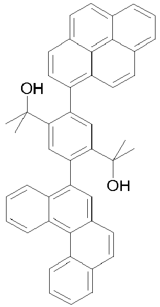 | 80 |
| 6c | 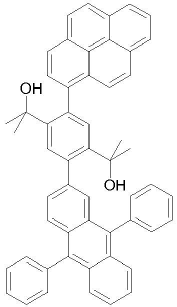 | 73 |
| 7c | 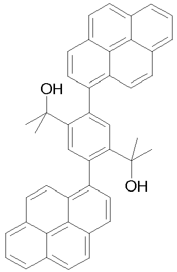 | 90 |
| 8c | 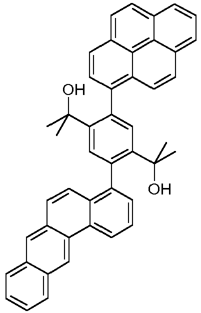 | quant. |
| 9c | 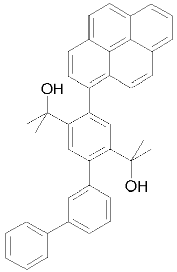 | quant. |
| 10c | 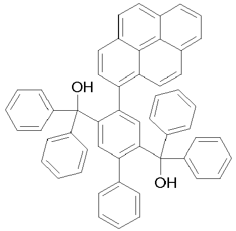 | quant. |

### d) 1,1-Dimethylbenzindeno-1,1-dimethylindeno[a]pyren

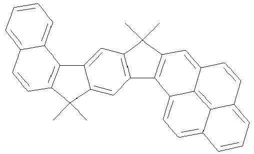

26.3 g (50.5 mmol) 2-[4-(1-Hydroxy-1-methyl-ethyl)-2-naphthalin-1-yl-5-pyren-1-yl-phenyl]-propan-2-ol werden in 750 mL Dichlormethan gelöst, 45 mL Methansulfonsäure in 70 g Polyphosphorsäure bei -20 °C zugetropft und 1 h bei dieser Temperatur gerührt. Nach vollständiger Umsetzung werden 400 mL EtOH zugetropft, 1 h zum Sieden erhitzt und der gelbe Feststoff abfiltriert. Nach viermaliger Umkristallisation aus NMP und zweimaliger Sublimation im Vakuum (p = 1 x 10⁻⁵ mbar, T = 340 °C) erhält man ein gelbes Pulver mit einer Reinheit >99.9 % (16 g, 65 %).

In Analogie zum oben beschriebenen Verfahren werden folgende Verbindungen hergestellt (Beispiele 2d bis 10d).

| Bsp. | Struktur | Ausbeute (%) |
|---|---|---|
| 2d | 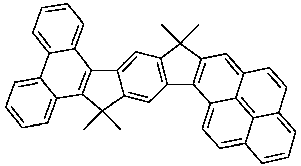 | 27 |
| 3d | 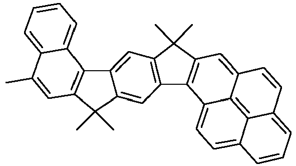 | 41 |
| 4d | 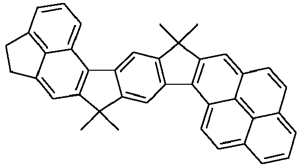 | 50 |
| 5d | 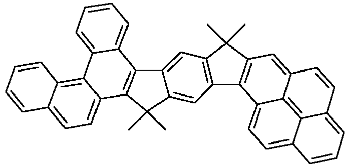 | 15 |
| 6d | 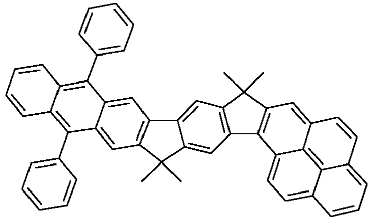 | 32 |
| 7d | 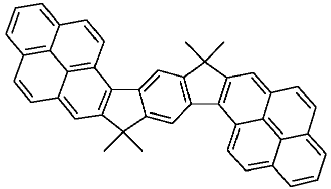 | 10 |
| 8d | 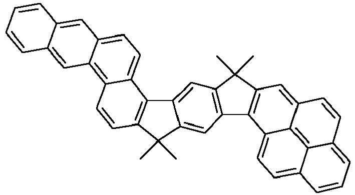 | 53 |
| 9d | 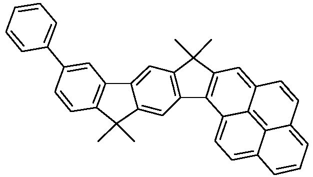 | 37 |
| 10d | 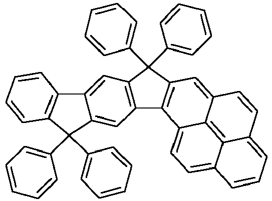 | 37 |

### Beispiel 11: Synthese von 1,1-Dimethylbenzindeno-1,1-dimethyl-indeno-[b]fluoranthen

### a) 2-Chlor-5-naphthalin-1-yl-terephthalsäurediethylester

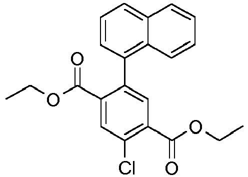

51 g (298 mmol) 1-Naphthylboronsäure, 100 g (298 mmol) Chlorbromterephthalsäurediethylester und 144 g (626 mmol) Kaliumphosphat-Monohydrat werden in einer Mischung aus 600 mL dest. Wasser, 400 mL Toluol und 200 mL Dioxan vorgelegt und 30 min mit N₂ gesättigt. Anschließend werden 5.4 g (18 mmol) Tri(*o*-tolyl)phosphin und 669 mg (3 mmol) Palladium(II)acetat zugegeben, und das Gemisch wird für 3 h zum Sieden erhitzt. Nach Verdünnen mit Toluol wird die organische Phase abgetrennt, zweimal mit Wasser gewaschen, über Na₂SO₄ getrocknet und im Vakuum eingeengt. Das verbleibende Öl wird am Dünnschichtverdampfer (p = 5 x 10⁻³ mbar, T = 130 °C) destilliert und in Form eines gelben Öls (74 g, 65 %) isoliert, welches gemäß ¹H-NMR eine Reinheit von >95 % aufweist.

### b) 2-Fluoranthen-3-yl-5-naphthalin-1-yl-terephthalsäurediethylester

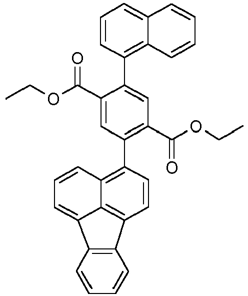

15.4 g (40 mmol) 2-Chlor-5-naphthalin-1-yl-terephthalsäurediethylester, 14.0 g (56 mmol) Fluoranthen-3-boronsäure und 17.7 g Cs₂CO₃ werden in 70 mL trockenem Dioxan vorgelegt und 30 min mit N₂ gesättigt. Danach erfolgt Zugabe von 0.8 mL einer 1.0 M Lösung von Tri-*tert*-butylphosphin in Toluol, gefolgt von 91 mg (0.4 mmol) Pd(OAc)₂. Das Gemisch wird für 4 h zum Sieden erhitzt, mit Wasser und EtOH erweitert, der Niederschlag abgesaugt, mit Heptan gewaschen und getrocknet. Der Feststoff wird aus Toluol umkristallisiert und weist danach gemäß ¹H-NMR eine Reinheit von >95 % auf. Die Ausbeute beträgt 8.5 g (38 %) an farblosem Feststoff.

### c) 2-[4-(1-Hydroxy-1-methylethyl)-2-fluoranthen-3-yl-5-naphthalin-1-yl-phenyl]-propan-2-ol

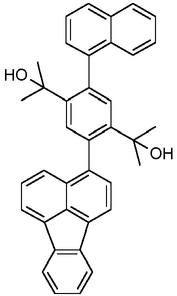

8.5 g (15 mol) 2-Fluoranthen-3-yl-5-naphthalin-1-yl-terephthalsäure-diethylester werden in 75 mL trockenem THF gelöst, bei 5 °C mit 31 mL (93 mmol) einer 3 M Methylmagnesiumchlorid-Lösung in THF versetzt und für 12 h bei RT gerührt. Nach Unterbrechung der Reaktion durch Zugabe von 30 mL 25 %iger Essigsäure wird extraktiv mit Essigsäureethylester / Wasser aufgearbeitet, über Na₂SO₄ getrocknet und einrotiert. Es werden 8.0 g (99 %) des Rohprodukts isoliert, welches ohne weitere Aufreinigung in der nächsten Stufe eingesetzt wird.

### d) 1,1-Dimethylbenzindeno-1,1-dimethylindeno[b]fluoranthen

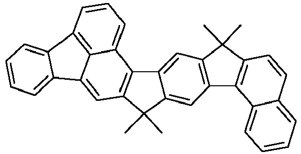

8.0 g (15.4 mmol) 2-[4-(1-Hydroxy-1-methylethyl)-2-fluoranthen-3-yl-5-naphthalin-1-yl-phenyl]-propan-2-ol werden in 250 mL Dichlormethan gelöst, 15 mL Methansulfonsäure in 22 g Polyphosphorsäure bei -20 °C zugetropft und 1 h bei dieser Temperatur gerührt. Nach vollständiger Umsetzung werden 130 mL EtOH zugetropft, 1 h zum Sieden erhitzt und der gelbe Feststoff abfiltriert. Nach zweimaliger Umkristallisation aus Toluol und zweimaliger Sublimation im Vakuum (p = 4 x 10⁻⁶ mbar, T = 300 °C) erhält man ein gelbes Pulver mit einer Reinheit >99.9% (1.9 g, 25%).

### Beispiel 12: Synthese von 1,1-Diphenylbenzindeno-1,1-diphenyl-indeno-[a]pyren

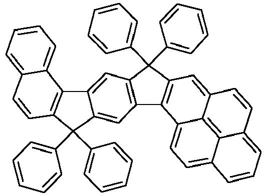

Die Synthese erfolgt analog zu Beispiel 1, wobei in Schritt c) Phenylmagnesiumchlorid-Lösung statt Methylmagnesiumchlorid-Lösung verwendet wird.

### Beispiel 13: Herstellung der OLEDs

Die Herstellung von OLEDs erfolgt nach einem Verfahren, das in WO 04/058911 allgemein beschrieben ist und das im Einzelfall auf die jeweiligen Gegebenheiten (z. B. Schichtdickenvariation, um optimale Effizienz bzw. Farbe zu erreichen) angepasst wird.

In den folgenden Beispielen 14 bis 31 werden die Ergebnisse verschiedener OLEDs vorgestellt. Glasplatten, die mit strukturiertem ITO (Indium Zinn Oxid) beschichtet sind, bilden die Substrate der OLEDs. Die OLEDs bestehen aus folgender Schichtenfolge: Substrat / Lochinjektionsschicht (HIM) / Lochtransportschicht (HTM1) 60 nm / Lochtransportschicht (HTM2) 20 nm / Emissionschicht (EML) 30 nm / Elektronentransportschicht (ETM) 20 nm und abschließend eine Kathode. Die Materialien werden in einer Vakuumkammer thermisch aufgedampft. Dabei besteht die Emissionsschicht immer aus einem Matrixmaterial (Host) und einem Dotierstoff (Dotand), der durch Coverdampfung dem Host beigemischt wird. Die Kathode wird durch eine 1 nm dünne LiF-Schicht und einer darauf abgeschiedenen 100 nm Al-Schicht gebildet. Die Tabelle 4 zeigt die chemischen Strukturen der zum Aufbau der OLEDs verwendeten Materialien.

Diese OLEDs werden standardmäßig charakterisiert; hierfür werden die Elektrolumineszenzspektren, die Effizienz (gemessen in cd/A), die Leistungseffizienz (gemessen in Im/W) in Abhängigkeit der Helligkeit, berechnet aus Strom-Spannungs-Helligkeit-Kennlinien (IUL-Kennlinien), und die Lebensdauer bestimmt. Als Lebensdauer wird die Zeit definiert, nach der die Anfangshelligkeit von 6000 cd/m² (für blau emittierende OLEDs) 25.000 cd/m² (für grün emittierende OLEDs) auf die Hälfte gesunken ist.

In Tabelle 5 und 6 sind die Ergebnisse einiger OLEDs (Beispiele 14 bis 31) zusammengefasst. Als erfindungsgemäße Hostmaterialien bzw. Emittermaterialien werden die Verbindungen der Beispiele 1d, 2d, 5d und 12 verwendet. Als Vergleichsbeispiele werden der Host H1 bzw. die Emitter D1, D2 oder D3 gemäß dem Stand der Technik verwendet.

Wie man aus den Ergebnissen in Tabelle 5 und 6 deutlich erkennen kann, weisen organische Elektrolumineszenzvorrichtungen enthaltend die erfindungsgemäßen Verbindungen eine deutlich höhere Lebensdauer für die Verwendung der erfindungsgemäßen Verbindung als Matrixmaterial und verbesserte Farbkoordinaten sowie eine deutlich höhere Lebensdauer für die Verwendung als Dotanden auf im Vergleich zu Materialien gemäß dem Stand der Technik.

**Tabelle 4**

| | | |
|---|---|---|
| 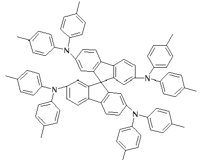 | 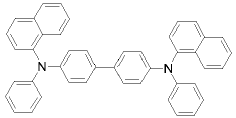 | 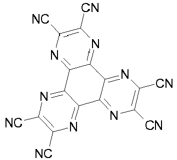 |
| HTM1 | HTM 2 | HIM |
| 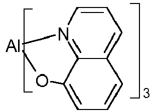 | 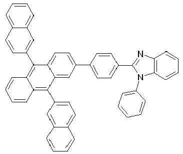 | 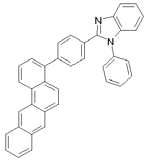 |
| ETM1 | ETM 2 | ETM3 |
| 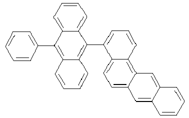 | 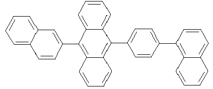 | 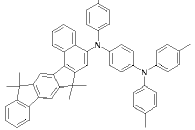 |
| H2 | H1 | D1 |
| 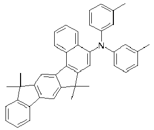 | 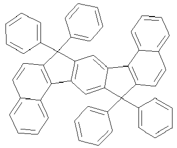 | 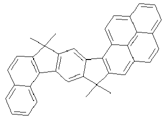 |
| D2 | D3 | Bsp. 1d |
| 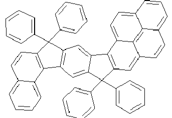 | 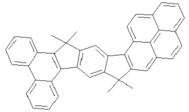 | 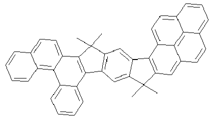 |
| Bsp. 12 | Bsp. 2d | Bsp. 5d |

**Tabelle 5**

| **Bsp.** | **EML** | **ETM** | **Farbe** | **Eff. (cd/A) bei 1000 cd/m²** | **Spannung (V) bei 1000 cd/m²** | **CIE** | **Lebens- dauer bei 25000 cd/m² (h)** |
|---|---|---|---|---|---|---|---|
| 14 (Vergl.) | H1 + 9% D1 | ETM2 | grün | 16.3 | 5.2 | x=0.29/y=0.60 | 300 |
| 15 | Bsp. 12 + 9% D1 | ETM2 | grün | 18.1 | 4.3 | x=0.29/y=0.61 | 320 |

**Tabelle 6**

| **Beispiel** | **EML** | **ETM** | **Farbe** | **Max. Effizienz (cd/A)** | **Spannung (V) bei 1000 cd/m²** | **CIE** | **Lebens- dauer bei 6000 cd/m² (h)** |
|---|---|---|---|---|---|---|---|
| 16 (Vergl.) | H1 + 5% D2 | ETM1 | blau | 4.1 | 5.3 | x=0.14/y=0.16 | 160 |
| 17 (Vergl.) | H2 + 5% D2 | ETM1 | blau | 4.3 | 5.2 | x=0.14/y=0.15 | 180 |
| 18 (Vergl.) | H2 + 5% D3 | ETM1 | blau | 1.5 | 5.1 | x=0.16/y=0.10 | 30 |
| 19 (Vergl.) | H1 + 5% D2 | H2 (50%) + ETM3 (50%) | blau | 4.9 | 5.0 | x=0.14/y=0.16 | 90 |
| 20 (Vergl.) | H2 + 5% D2 | H2 (50%) + ETM3 (50%) | blau | 5.3 | 4.9 | x=0.14/y=0.15 | 120 |
| 21 (Vergl.) | H2 + 5% D3 | H2 (50%) + ETM3 (50%) | blau | 1.9 | 5.0 | x=0.16/y=0.09 | 65 |
| 22 | H2 + 5% Bsp. 1d | ETM1 | blau | 3.5 | 5.9 | x=0.15/y=0.14 | 230 |
| 23 | H2 + 3% Bsp. 1d | ETM2 | blau | 3.0 | 5.8 | x=0.15/y=0.11 | 210 |
| 24 | H2 + 5% Bsp. 1d | H2 (50%) + ETM3 (50%) | blau | 6.4 | 4.6 | x=0.15/y=0.13 | 240 |
| 25 | H2 + 1% Bsp. 1d | H2 (50%) + ETM3 (50%) | blau | 5.5 | 4.4 | x=0.14/y=0.11 | 260 |
| 26 | H2 + 1% Bsp. 2d | H2 (50%) + ETM3 (50%) | blau | 4.7 | 4.8 | x=0.15/y=0.10 | 260 |
| 27 | H2 + 5% Bsp. 2d | H2 (50%) + ETM3 (50%) | blau | 5.0 | 4.7 | x=0.15/y=0.11 | 270 |
| 28 | H2 + 5% Bsp. 2d | ETM2 | blau | 4.6 | 5.4 | x=0.15/y=0.11 | 290 |
| 29 | H2 + 1% Bsp. 5d | H2 (50%) + ETM3 (50%) | blau | 7.4 | 4.7 | x=0.14/y=0.15 | 300 |
| 30 | H2 + 5% Bsp. 5d | H2 (50%) + ETM3 (50%) | blau | 7.8 | 4.5 | x=0.14/y=0.16 | 310 |
| 31 | H2 + 5% Bsp. 5d | ETM2 | blau | 6.8 | 5.3 | x=0.14/y=0.16 | 330 |

## Patentansprüche

1. Verbindung gemäß Formel (1), 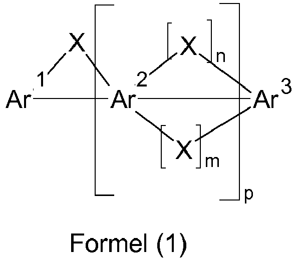 wobei für die verwendeten Symbole und Indizes gilt:
Ar¹, Ar², Ar³ ist bei jedem Auftreten gleich oder verschieden eine Aryl- oder Heteroarylgruppe mit 5 bis 22 aromatischen Ringatomen, ausgewählt aus Benzol, Naphthalin, Anthracen, Phenanthren, Fluoranthen, Naphthacen, Benzanthracen, Chrysen, Pyren, Benzfluoranthen, Triphenylen, Perylen, Dibenzanthracen, Benzpyren, Picen, Pentacen, Pentaphen, Pyridin, Pyrazin, Pyrimidin, Pyridazin, Chinolin, Isochinolin, Phenanthrolin und Acridin, die mit einem oder mehreren Resten R¹ substituiert sein kann, mit der Maßgabe, dass Ar² nicht für Anthracen, Naphthacen oder Pentacen steht;
X ist bei jedem Auftreten gleich oder verschieden eine Gruppe, ausgewählt aus C(R²)₂;
R¹ ist bei jedem Auftreten gleich oder verschieden eine Gruppe, ausgewählt aus H, D, F, geradkettigen Alkyl- oder Alkoxygruppen mit 1 bis 10 C-Atomen oder verzweigten oder cyclischen Alkyl- oder Alkoxygruppen mit 3 bis 10 C-Atomen, die jeweils mit einem oder mehreren Resten R³ substituiert sein kann, wobei jeweils eine oder mehrere nicht benachbarte CH2-Gruppen durch R³C=CR³ oder O ersetzt sein können und wobei ein oder mehrere H-Atome durch F ersetzt sein können, oder aromatischen oder heteroaromatischen Ringsystemen mit 5 bis 24 aromatischen Ringatomen; dabei können zwei oder mehrere Substituenten R¹ auch miteinander ein mono- oder polycyclisches, aliphatisches oder aromatisches Ringsystem bilden;
R² ist bei jedem Auftreten gleich oder verschieden eine Gruppe, ausgewählt aus H, geradkettigen Alkylgruppen mit 1 bis 10 C-Atomen oder verzweigten oder cyclischen Alkylgruppen mit 3 bis 10 C-Atomen, wobei jeweils eine oder mehrere nicht benachbarte CH2-Gruppen durch -R²C=CR²- oder -O- ersetzt sein können und wobei ein oder mehrere H-Atome durch F ersetzt sein können, oder einer monovalenten Aryl- oder Heteroarylgruppe mit 5 bis 16 aromatischen Ringatomen, die mit einem oder mehreren nicht-aromatischen Resten R² substituiert sein kann; dabei können auch zwei Reste R², die in derselben Gruppe X gebunden sind, miteinander ein Ringsystem bilden;
R³ ist bei jedem Auftreten gleich oder verschieden H oder ein aliphatischer oder aromatischer Kohlenwasserstoffrest mit 1 bis 20 C-Atomen;
m, n sind 0 oder 1, mit der Maßgabe, dass m + n = 1 ist;
p ist 1;
dabei bilden Ar¹, Ar² und X zusammen einen Fünfring oder einen Sechsring und Ar², Ar³ und X bilden zusammen einen Fünfring oder einen Sechsring, mit der Maßgabe, dass entweder alle Symbole X in der Verbindung gemäß Formel (1) in einem Fünfring gebunden sind oder dass alle Symbole X in der Verbindung gemäß Formel (1) in einem Sechsring gebunden sind;
**dadurch gekennzeichnet, dass** die Summe aller π-Elektronen der Gruppen Ar¹, Ar² und Ar³ mindestens 28 beträgt;
dabei sind die folgenden Verbindungen ausgenommen:

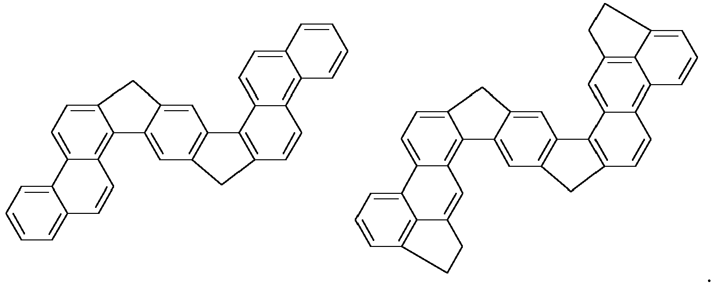

2. Verbindung nach Anspruch 1, **dadurch gekennzeichnet, dass** die Summe aller π-Elektronen der Gruppen Ar¹, Ar² und Ar³ zwischen 28 und 50, bevorzugt zwischen 28 und 46, besonders bevorzugt zwischen 28 und 42, und insbesondere zwischen 28 und 36 beträgt.

3. Verbindung nach Anspruch 1 oder 2, **dadurch gekennzeichnet, dass** Ar¹ und Ar³ unabhängig voneinander ausgewählt sind aus den Gruppen der Formeln (2) bis (85) sind, welche jeweils durch einen oder mehrere Reste R¹ substituiert sein können und wobei das Symbol * für die Position der Verknüpfung von Ar¹ bzw. Ar³ mit Ar² und das Symbol # für die Position der Verknüpfung von Ar¹ bzw. Ar³ mit X steht: 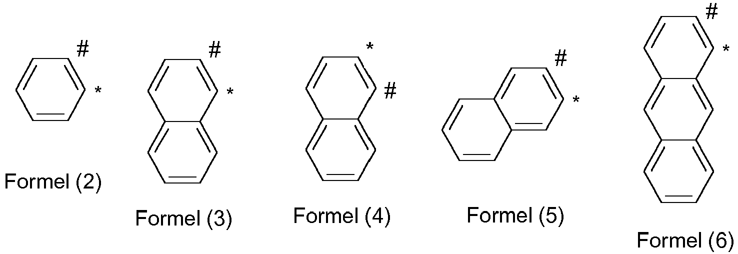 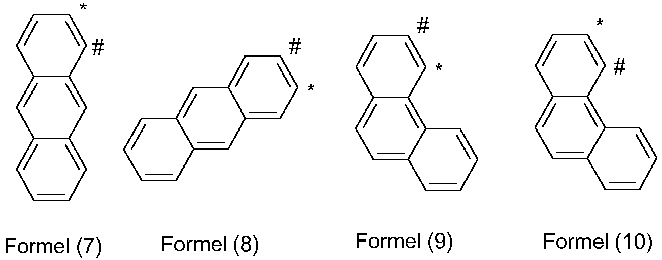 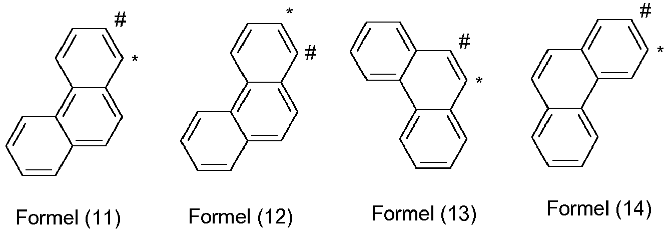 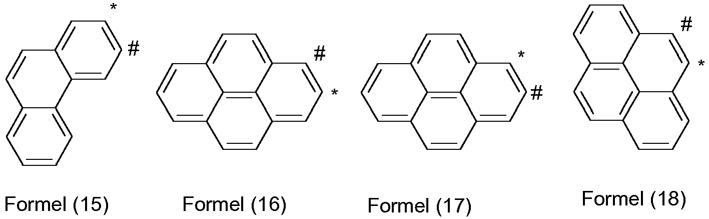 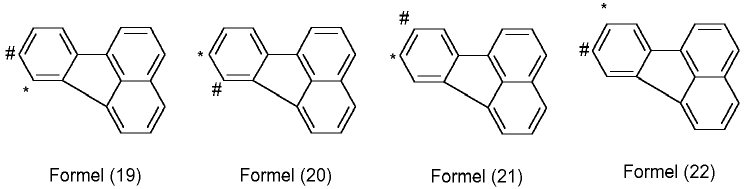 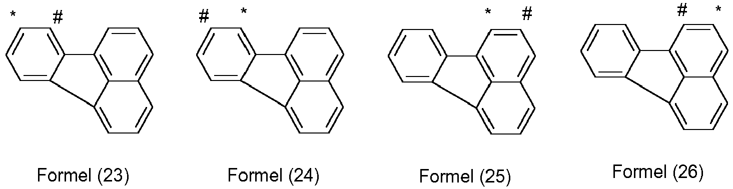 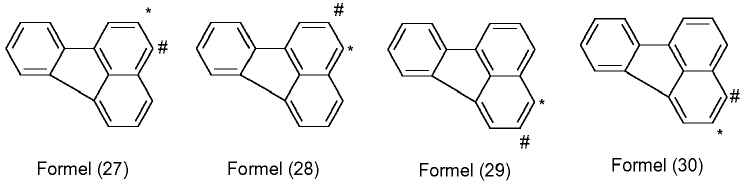 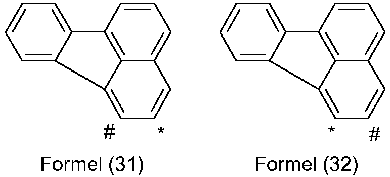 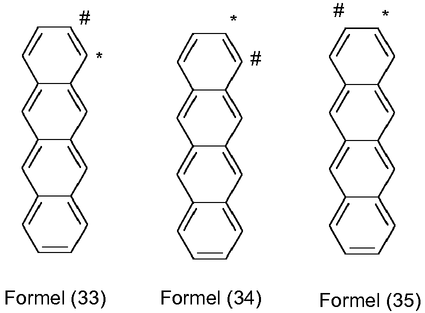 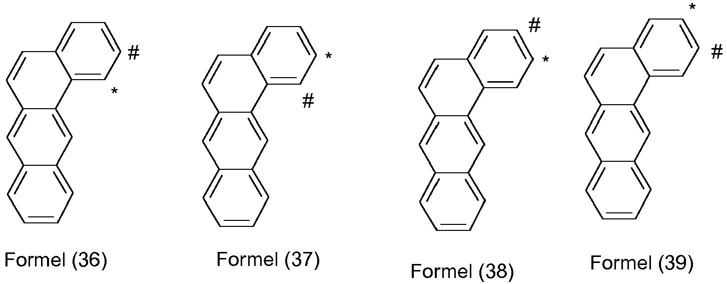 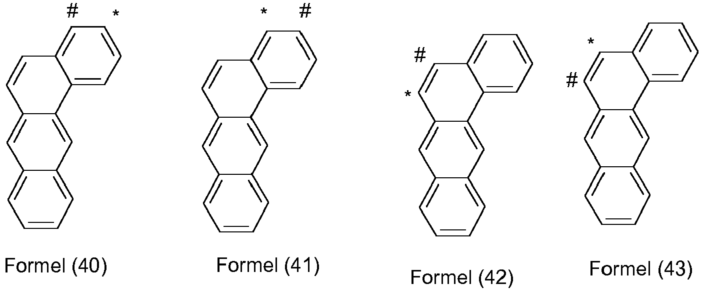 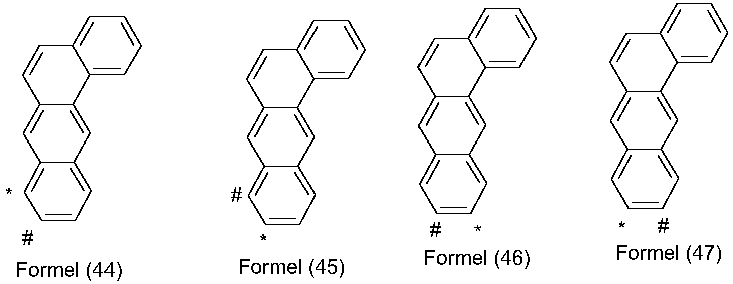 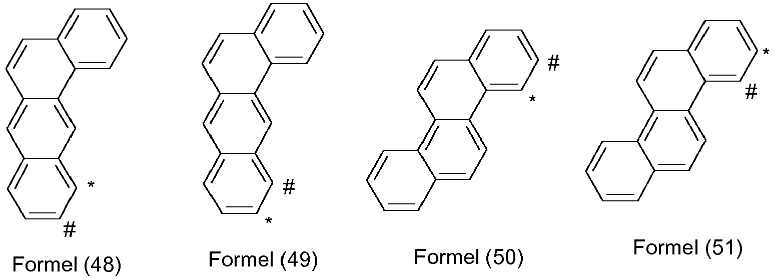 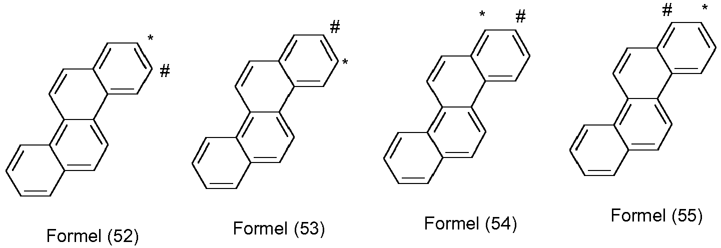 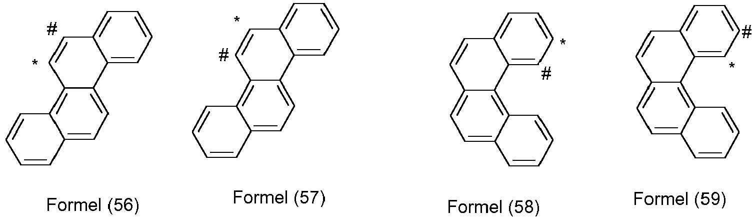 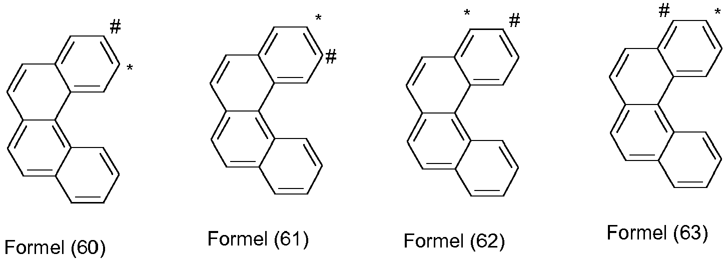 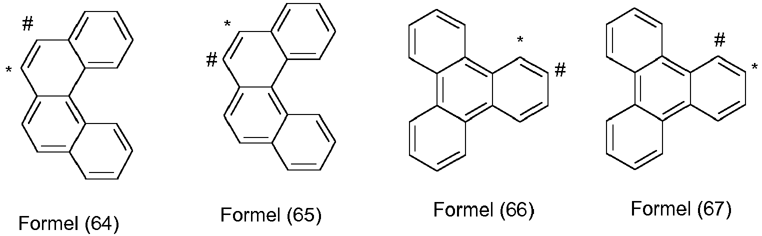 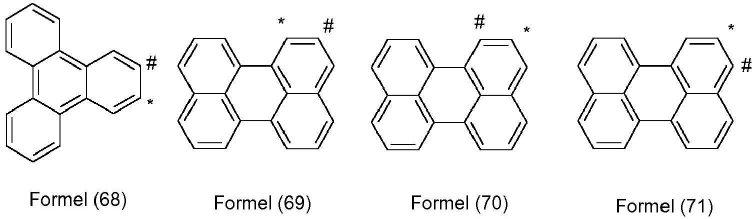 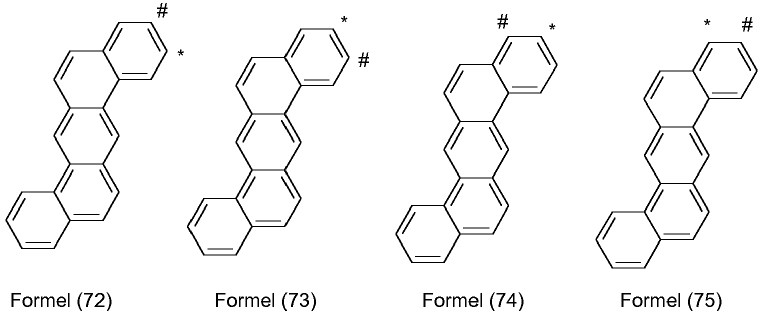 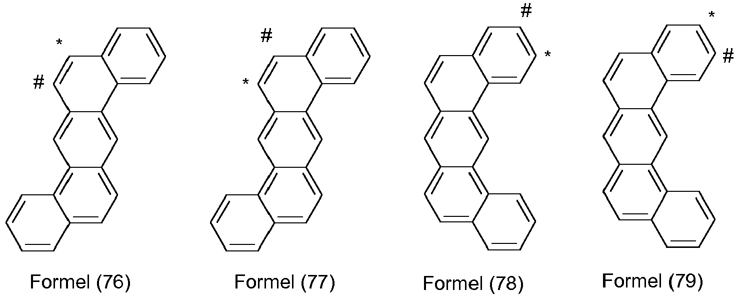 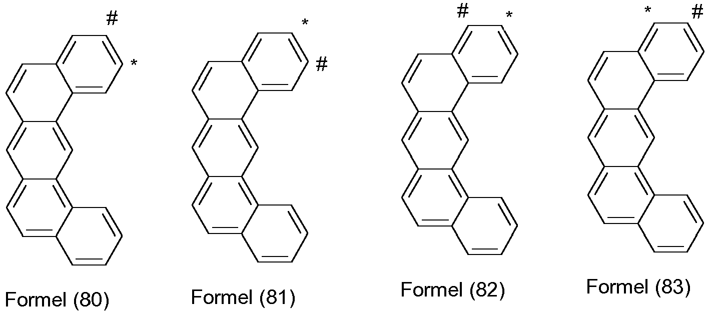 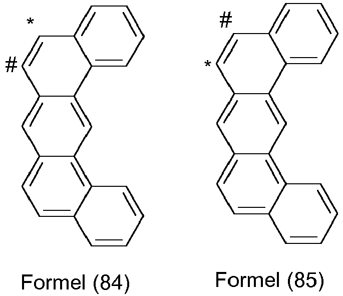 oder aus entsprechenden Gruppen, die zusammen mit Ar² einen Sechsring bilden.

4. Verbindung nach einem oder mehreren der Ansprüche 1 bis 3, **dadurch gekennzeichnet, dass** Ar² gleich oder verschieden bei jedem Auftreten ausgewählt ist aus den Gruppen der Formeln (86) bis (106) sind, welche jeweils durch einen oder mehrere Reste R¹ substituiert sein können und wobei das Symbol * für die Position der Verknüpfung von Ar² mit Ar¹ bzw. Ar³ und das Symbol # steht für die Position der Verknüpfung von Ar² mit X steht: 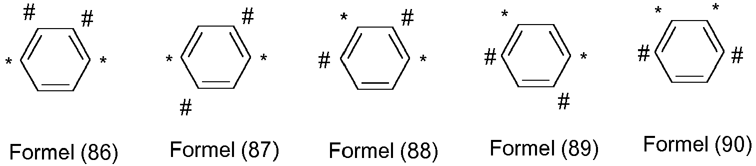 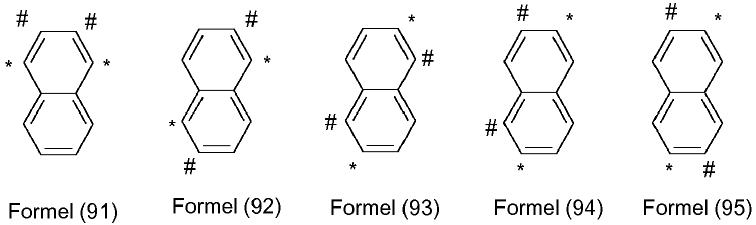 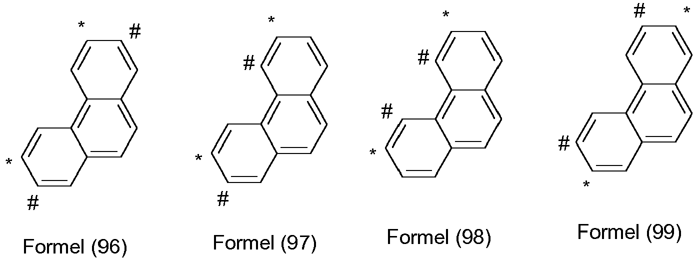 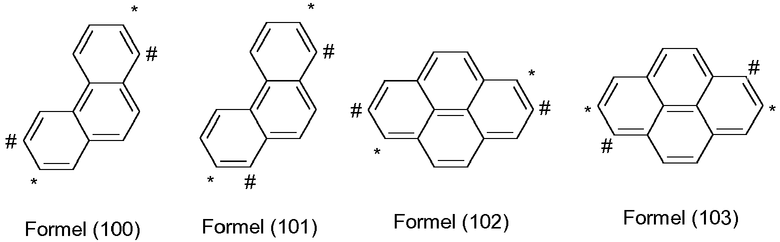 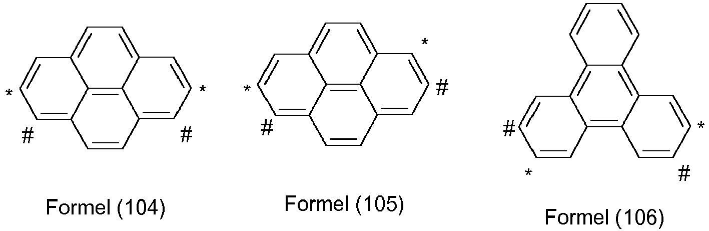 oder aus entsprechenden Gruppen, die zusammen mit Ar¹ bzw. Ar³ einen Sechsring bilden.

5. Verbindung nach einem oder mehreren der Ansprüche 1 bis 4, **dadurch gekennzeichnet, dass** mindestens eine der Gruppen Ar¹, Ar² bzw. Ar³ mindestens 4 kondensierte Ringe, also mindestens 16 π-Elektronen, aufweist.

6. Verbindung nach einem oder mehreren der Ansprüche 1 bis 5, **dadurch gekennzeichnet, dass** die Verbindungen ausgewählt sind aus den Formeln (111) bis (141), wobei die aromatischen Systeme jeweils auch durch einen oder mehrere Reste R¹ substituiert sein können:

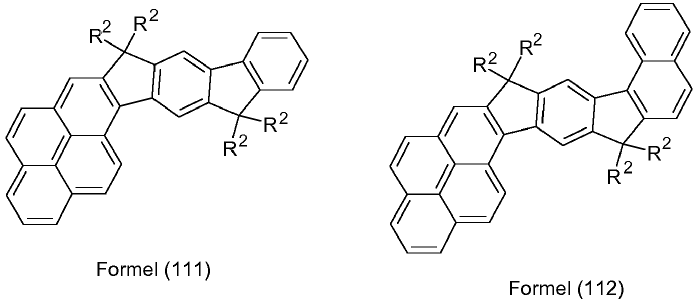

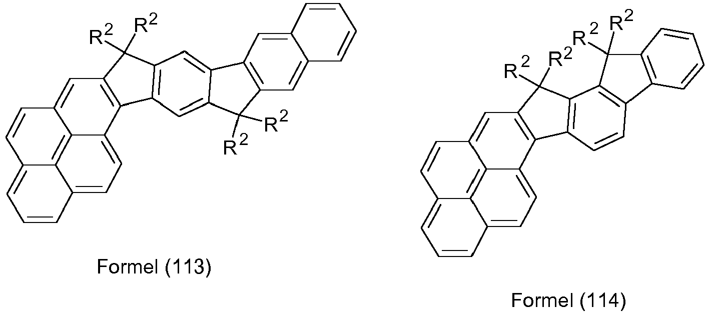

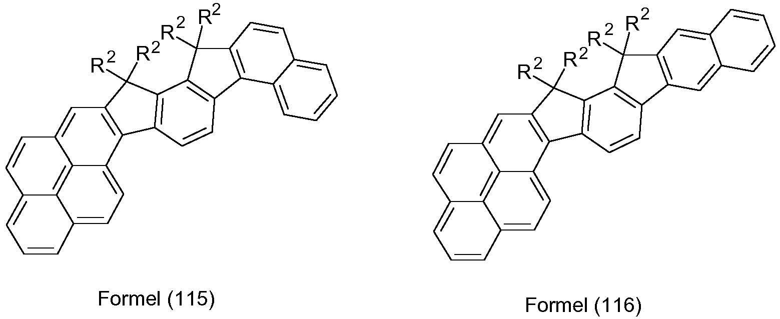

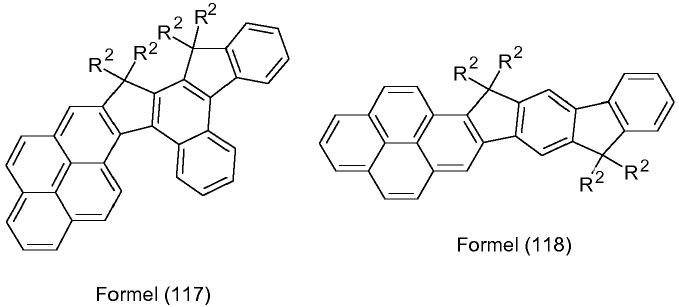

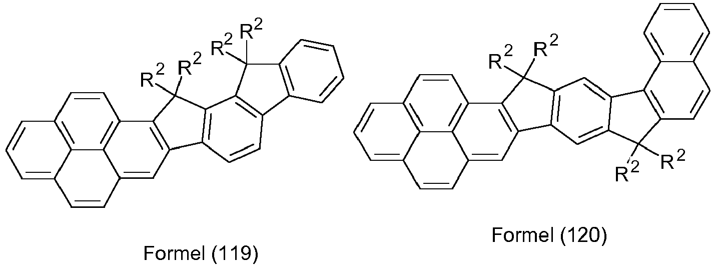

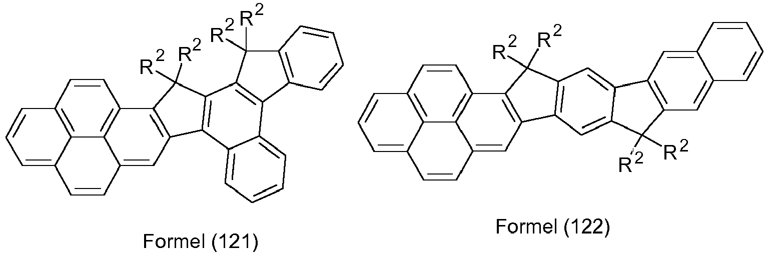

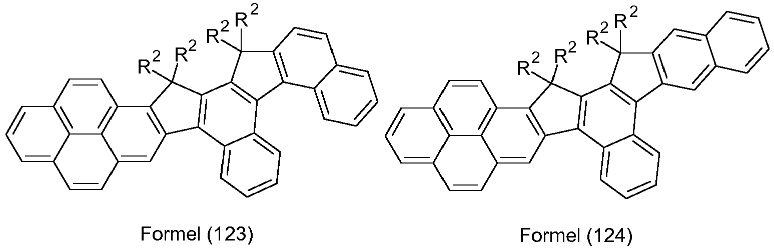

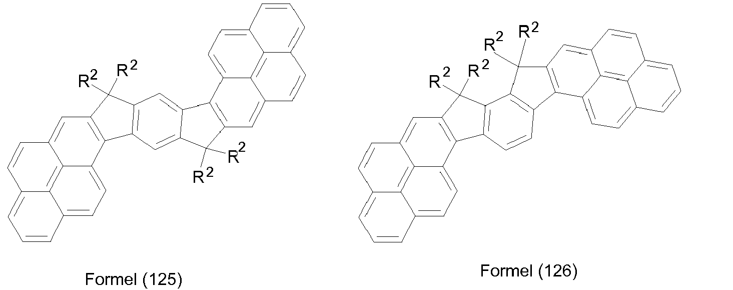

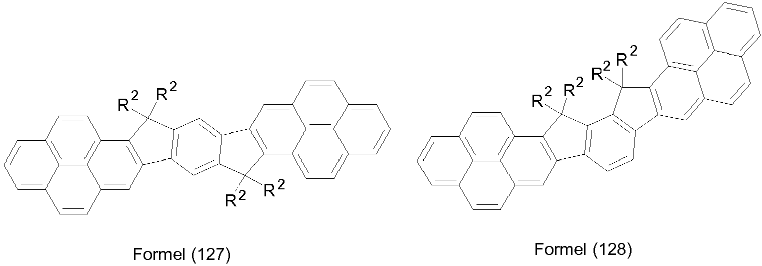

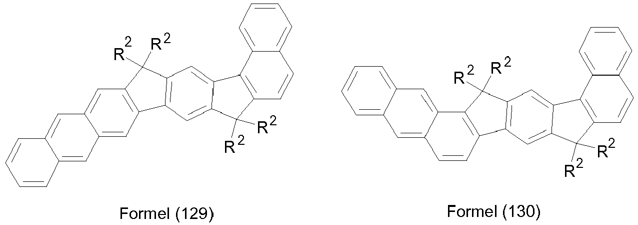

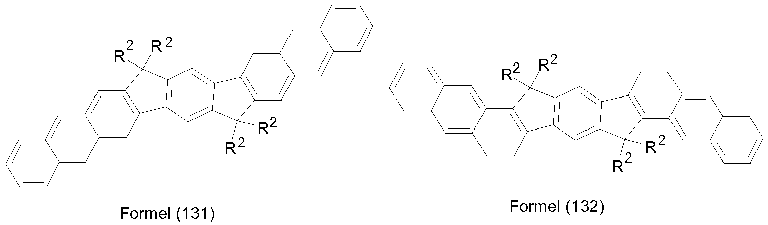

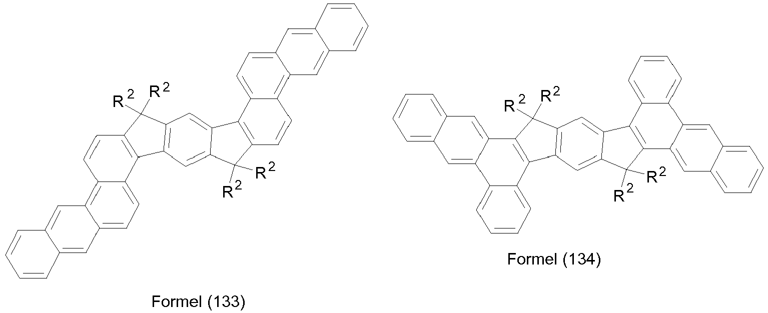

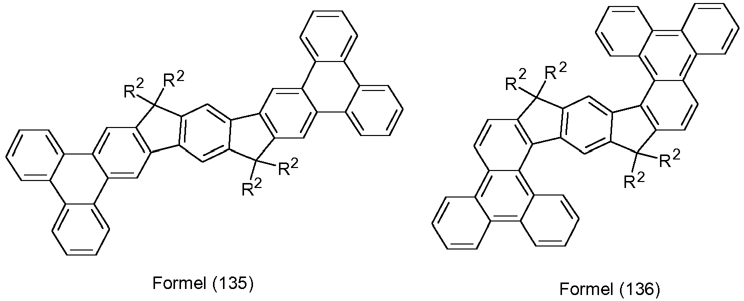

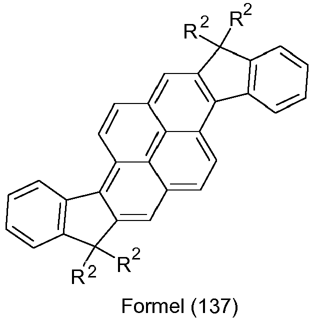

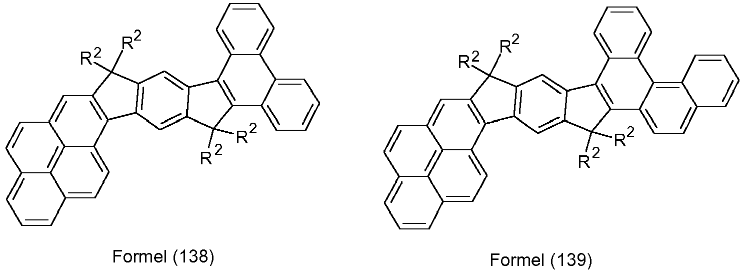

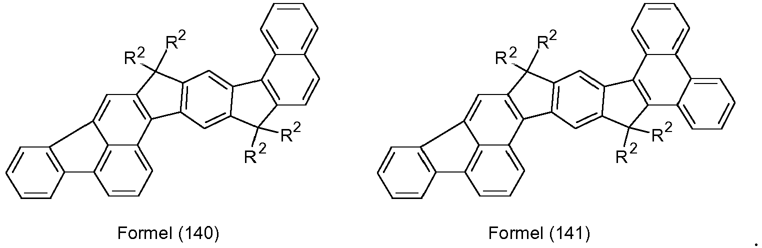

7. Oligomer, Polymer oder Dendrimer enthaltend eine oder mehrere Verbindungen nach einem oder mehreren der Ansprüche 1 bis 6, wobei mindestens einer der Reste R¹ und/oder R² eine Bindung zum Oligomer, Polymer oder Dendrimer darstellt.

8. Verfahren zur Herstellung der Verbindungen nach einem oder mehreren der Ansprüche 1 bis 6, umfassend die Reaktionsschritte:
a) Kupplung von geeignet substituiertem Ar¹, Ar² und Ar³, wobei geeignete Substituenten beispielsweise Carbonestergruppen sein können;und
b) Cyclisierung der Substituenten zur Einführung der Brücken X.

9. Verwendung von Verbindungen nach einem oder mehreren der Ansprüche 1 bis 7 in elektronischen Vorrichtungen, insbesondere in organischen Elektrolumineszenzvorrichtungen (OLEDs, PLEDs), organischen Feld-Effekt-Transistoren (O-FETs), organischen Dünnfilmtransistoren (O-TFTs), organischen lichtemittierenden Transistoren (O-LETs), organischen integrierten Schaltungen (O-ICs), organischen Solarzellen (O-SCs), organischen Feld-Quench-Devices (O-FQDs), lichtemittierenden elektrochemischen Zellen (LECs), organischen Photorezeptoren oder organischen Laserdioden (O-Laser).

10. Organische elektronische Vorrichtung, insbesondere ausgewählt aus organischen Elektrolumineszenzvorrichtungen (OLEDs, PLEDs), organischen Feld-Effekt-Transistoren (O-FETs), organischen Dünnfilmtransistoren (O-TFTs), organischen lichtemittierenden Transistoren (O-LETs), organischen integrierten Schaltungen (O-ICs), organischen Solarzellen (O-SCs), organischen Feld-Quench-Devices (O-FQDs), lichtemittierenden elektrochemischen Zellen (LECs), organischen Photorezeptoren oder organischen Laserdioden (O-Laser), **dadurch gekennzeichnet, dass** mindestens eine organische Schicht mindestens eine Verbindung nach einem oder mehreren der Ansprüche 1 bis 7 enthält.

11. Organische Elektrolumineszenzvorrichtung nach Anspruch 10, **dadurch gekennzeichnet, dass** die Verbindung nach einem oder mehreren der Ansprüche 1 bis 7 als emittierendes Material oder als Hostmaterial in einer emittierenden Schicht oder als Lochtransport- oder Lochinjektionsmaterial in einer Lochtransport- oder einer Lochinjektionsschicht oder als Elektronentransportmaterial in einer Elektronentransportschicht eingesetzt wird.

12. Organische Elektrolumineszenzvorrichtung nach Anspruch 10 oder 11, **dadurch gekennzeichnet, dass** die Verbindung nach einem oder mehreren der Ansprüche 1 bis 7 als emittierendes Material in einer emittierenden Schicht in Kombination mit einem Hostmaterial eingesetzt wird und dass das Hostmaterial ausgewählt ist aus der Gruppe bestehend aus Oligoarylenen, Oligoarylenen enthaltend kondensierte aromatische Gruppen, Anthracenen, Oligoarylenvinylenen, polypodalen Metallkomplexen, lochleitenden Verbindungen, elektronenleitenden Verbindungen, Ketonen, Phosphinoxiden, Sulfoxiden, Boronsäurederivaten, Benzanthracenen oder Verbindungen der Formel (138),
Ar⁴-(Ar⁵)ₚ-Ar⁶ Formel (138)
wobei Ar⁴, Ar⁵, Ar⁶ bei jedem Auftreten gleich oder verschieden eine Aryl- oder Heteroarylgruppe mit 5 bis 30 aromatischen Ringatomen ist, die mit einem oder mehreren Resten R¹ substituiert sein kann, und R¹ dieselbe Bedeutung hat, wie in Anspruch 1 beschrieben, und p gleich 1, 2 oder 3 sein kann; dabei gilt, dass die Summe der π-Elektronen in Ar⁴, Ar⁵ und Ar⁶ mindestens 30 beträgt, wenn p = 1 ist, und mindestens 36 beträgt, wenn p = 2 ist, und mindestens 42 beträgt, wenn p = 3 ist.

13. Lösung oder Formulierung enthaltend mindestens eine Verbindung nach einem oder mehreren der Ansprüche 1 bis 7 und mindestens ein organisches Lösemittel.

## Claims

1. Compound of the formula (1) 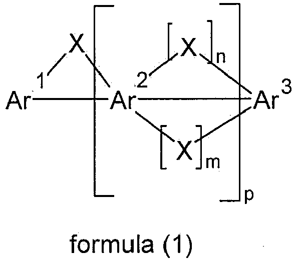 where the following applies to the symbols and indices used:
Ar¹, Ar², Ar³ are on each occurrence, identically or differently, an aryl or heteroaryl group having 5 to 22 aromatic ring atoms, selected from benzene, naphthalene, anthracene, phenanthrene, fluoranthene, naphthacene, benzanthracene, chrysene, pyrene, benzofluoranthene, triphenylene, perylene, dibenzanthracene, benzopyrene, picene, pentacene, pentaphene, pyridine, pyrazine, pyrimidine, pyridazine, quinoline, isoquinoline, phenanthroline and acridine, which may be substituted by one or more radicals R¹, with the proviso that Ar² does not stand for anthracene, naphthacene or pentacene;
X is on each occurrence, identically or differently, a group selected from C(R²)₂;
R¹ is on each occurrence, identically or differently, a group selected from H, D, F, straight-chain alkyl or alkoxy groups having 1 to 10 C atoms or branched or cyclic alkyl or alkoxy groups having 3 to 10 C atoms, which may in each case be substituted by one or more radicals R³, where in each case one or more non-adjacent CH₂ groups may be replaced by R³C=CR³ or O and where one or more H atoms may be replaced by F, or aromatic or heteroaromatic ring systems having 5 to 24 aromatic ring atoms; two or more substituents R¹ may also form a mono- or polycyclic, aliphatic or aromatic ring system with one another;
R² is on each occurrence, identically or differently, a group selected from H, straight-chain alkyl groups having 1 to 10 C atoms or branched or cyclic alkyl groups having 3 to 10 C atoms, where in each case one or more non-adjacent CH₂ groups may be replaced by -R²C=CR²- or -O- and where one or more H atoms may be replaced by F, or a monovalent aryl or heteroaryl group having 5 to 16 aromatic ring atoms, which may be substituted by one or more non-aromatic radicals R²; two radicals R² which are bonded in the same group X may also form a ring system with one another;
R³ is on each occurrence, identically or differently, H or an aliphatic or aromatic hydrocarbon radical having 1 to 20 C atoms;
m, n are 0 or 1, with the proviso that m + n = 1;
p is 1;
Ar¹, Ar² and X together form a five-membered ring or a six-membered ring, and Ar², Ar³ and X together form a five-membered ring or a six-membered ring, with the proviso that either all symbols X in the compound of the formula (1) are bound in a five-membered ring or all symbols X in the compound of the formula (1) are bound in a six-membered ring;
**characterised in that** the sum of all π electrons in groups Ar¹, Ar² and Ar³ is at least 28:
the following compounds are excluded from the invention:

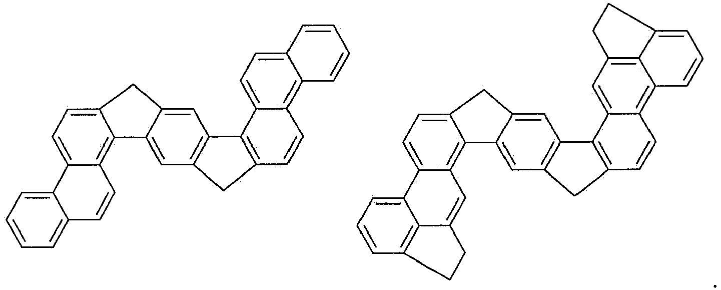

2. Compound according to Claim 1, **characterised in that** the sum of all π electrons in groups Ar¹, Ar² and Ar³ is between 28 and 50, preferably between 28 and 46, particularly preferably between 28 and 42, in particular between 28 and 36.

3. Compound according to Claim 1 or 2, **characterised in that** the groups Ar¹ and Ar³ are selected, independently of one another, from the groups of the formulae (2) to (85), which may in each case be substituted by one or more radicals R¹ and where the symbol * stands for the position of the link from Ar¹ or Ar³ to Ar² and the symbol # stands for the position of the link from Ar¹ or Ar³ to X: 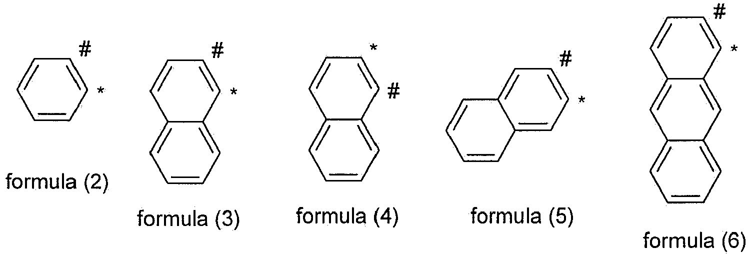 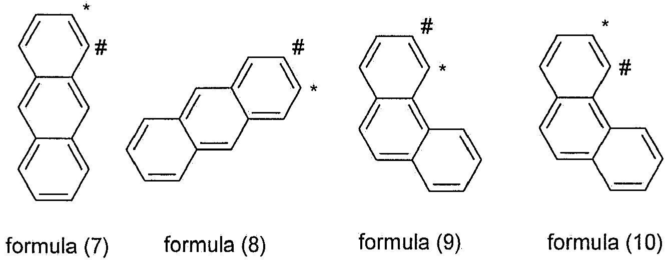 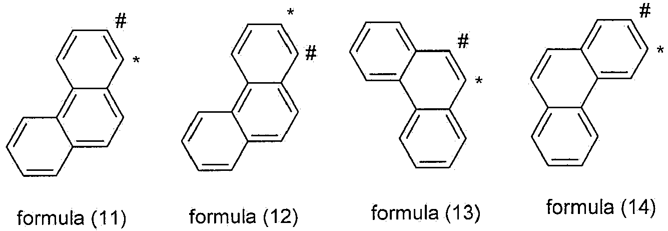 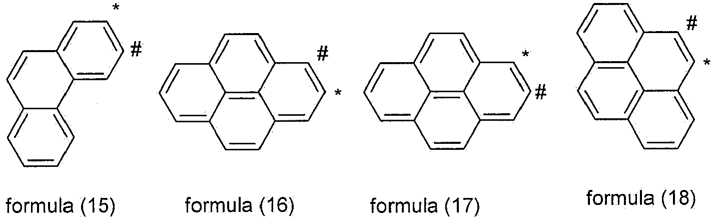 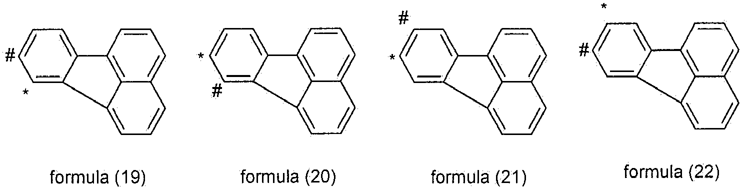 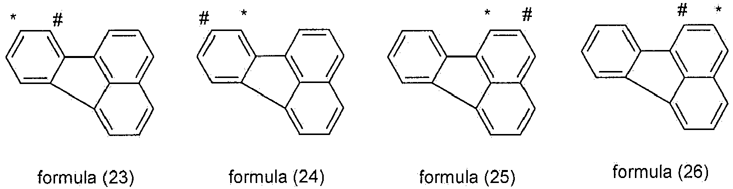 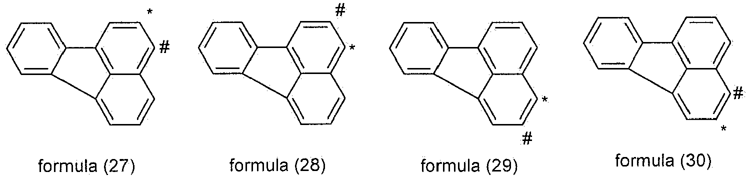 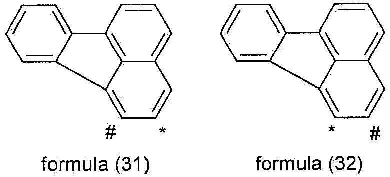 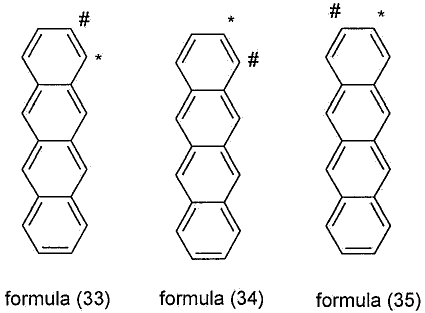 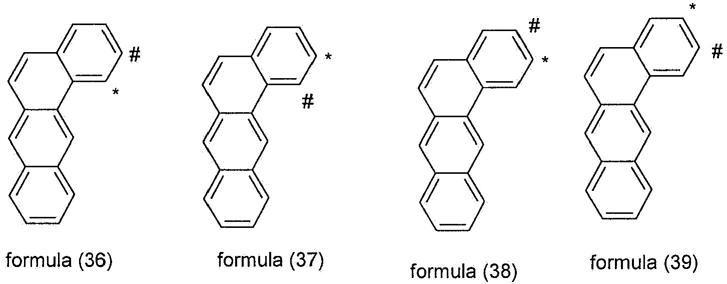 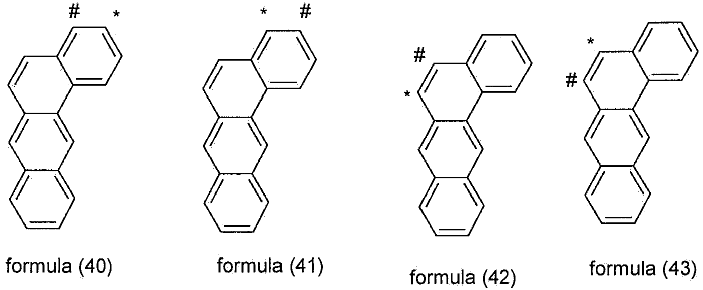 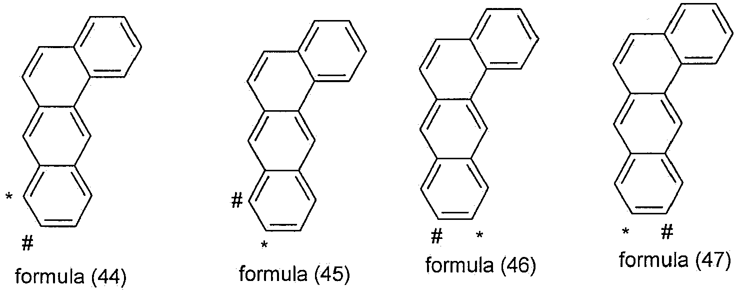 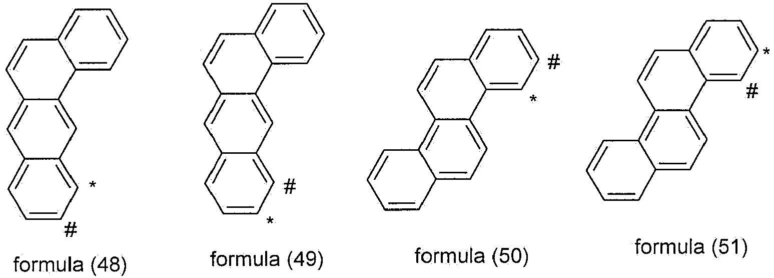 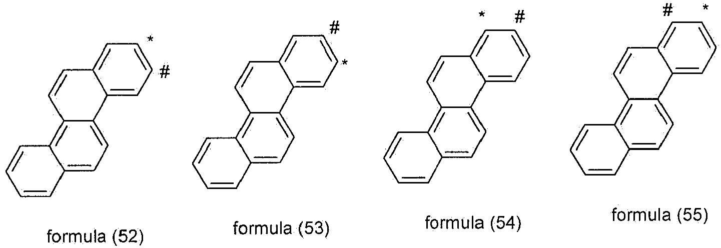 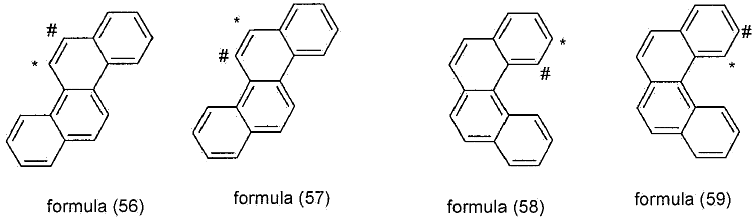 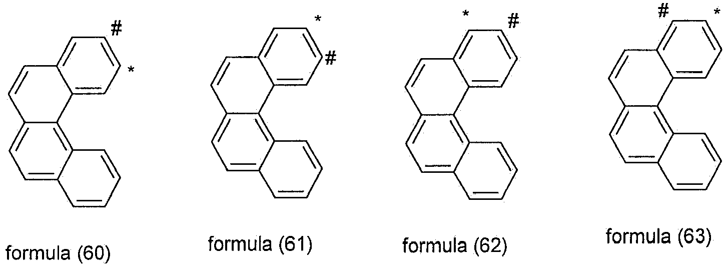 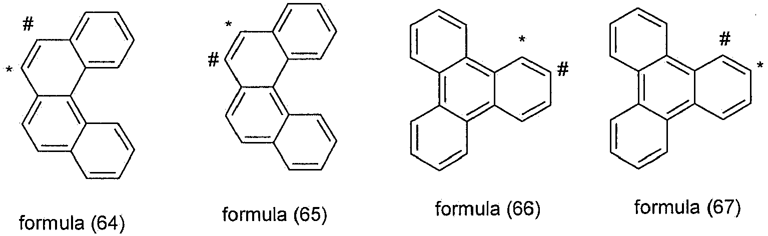 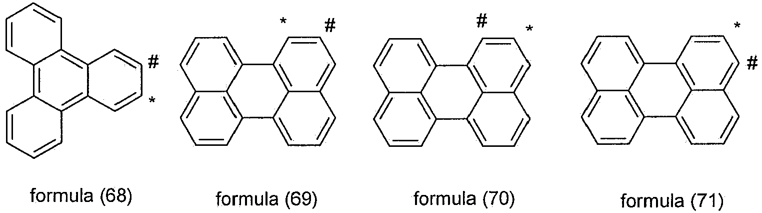 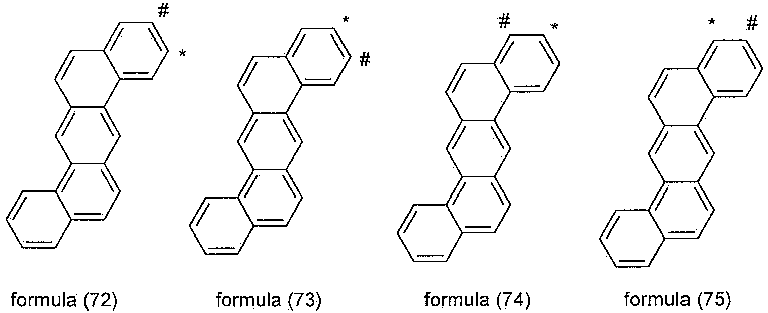 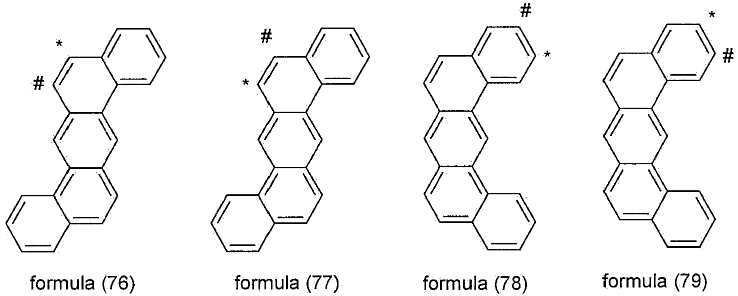 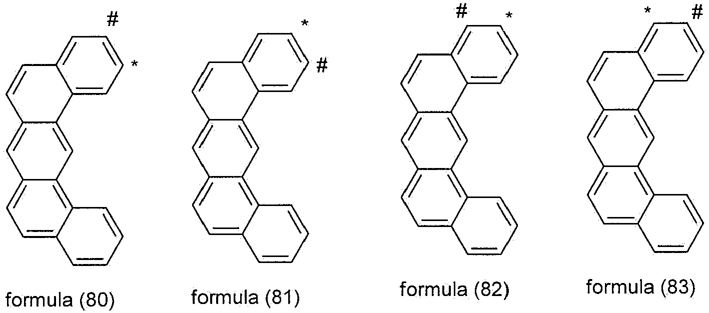 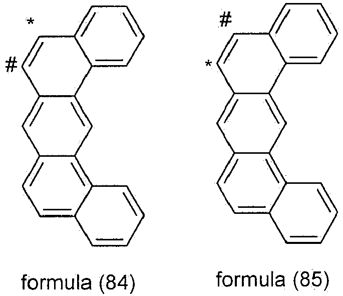 or from corresponding groups which form a six-membered ring together with Ar².

4. Compound according to one or more of Claims 1 to 3, **characterised in that** Ar² is selected, identically or differently on each occurrence, from the groups of the formulae (86) to (106), which may in each case be substituted by one or more radicals R¹ and where the symbol * stands for the position of the link from Ar² to Ar¹ or Ar³ and the symbol # stands for the position of the link from Ar² to X: 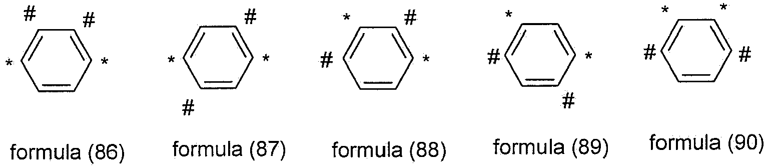 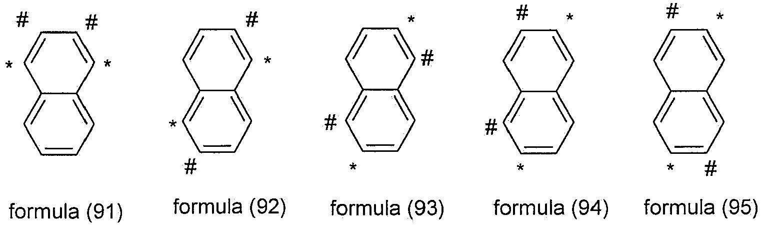 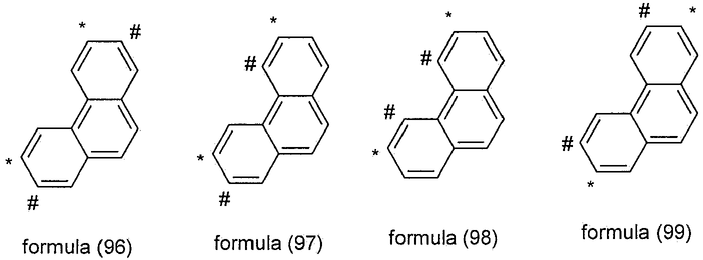 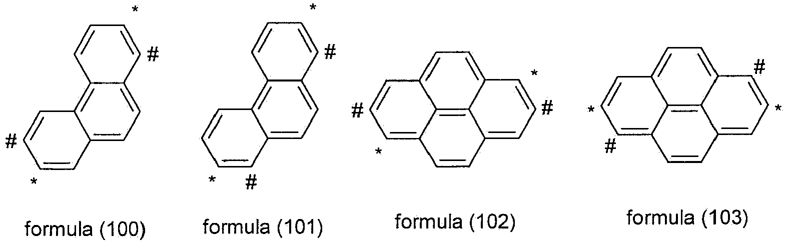 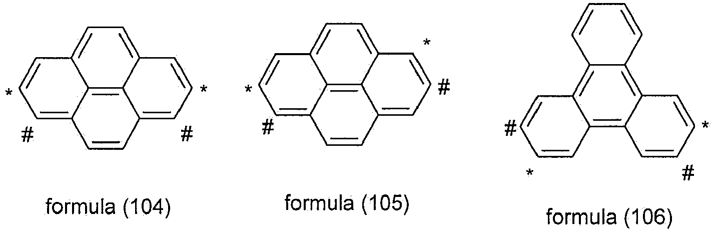 or from corresponding groups which form a six-membered ring together with Ar¹ or Ar³.

5. Compound according to one or more of Claims 1 to 4, **characterised in that** at least one of the groups Ar¹, Ar² and Ar³ has at least 4 condensed rings, i.e. at least 16 π electrons.

6. Compound according to one or more of Claims 1 to 5, **characterised in that** the compounds are selected from the formulae (111) to (141), where the aromatic systems may in each case also be substituted by one or more radicals R¹:

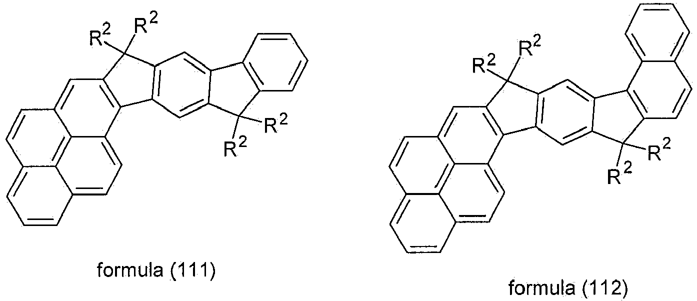

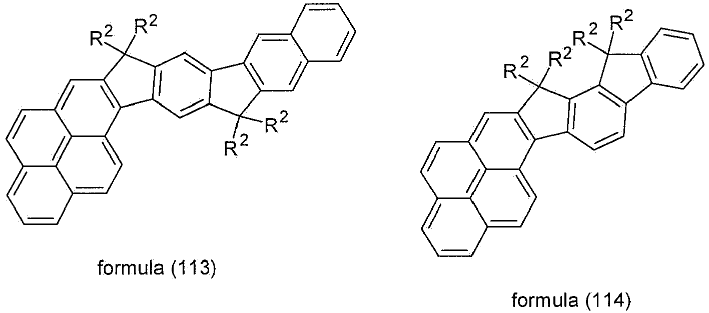

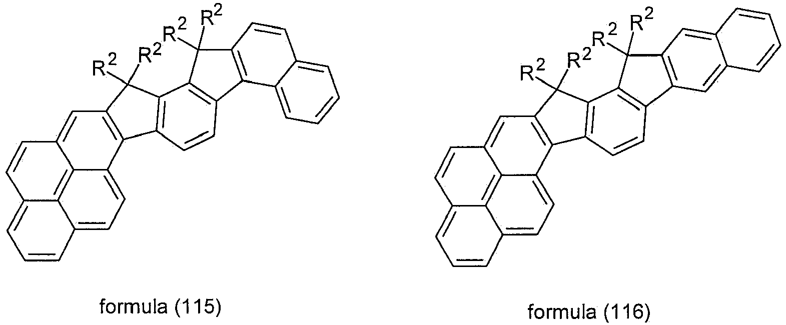

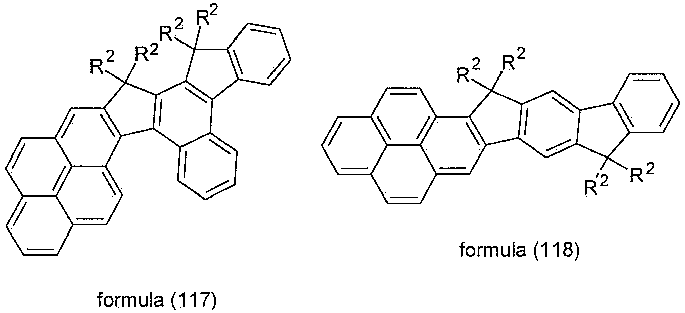

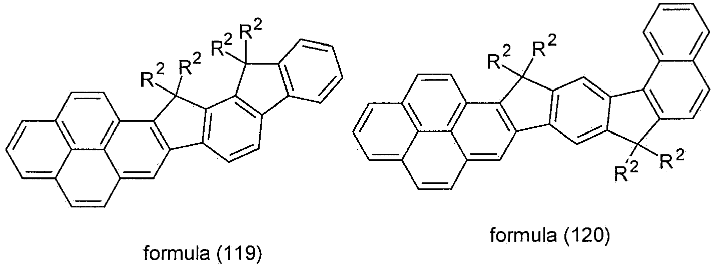

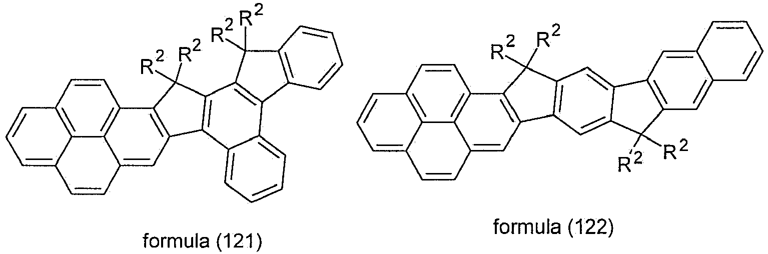

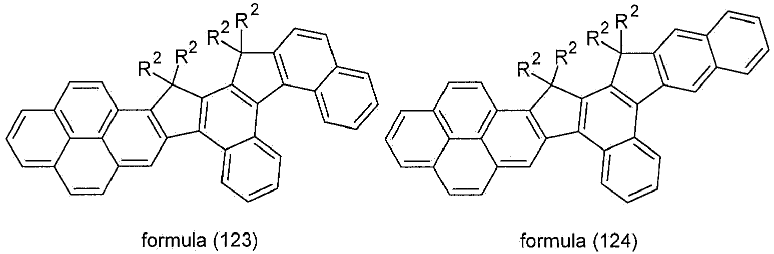

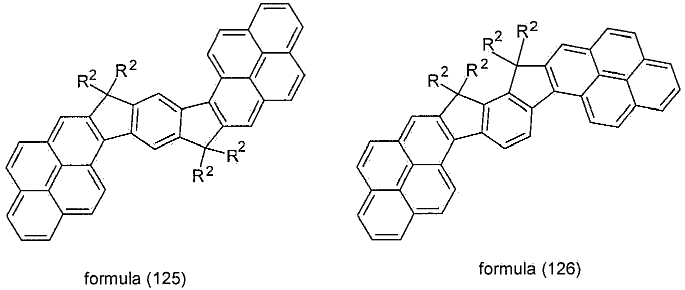

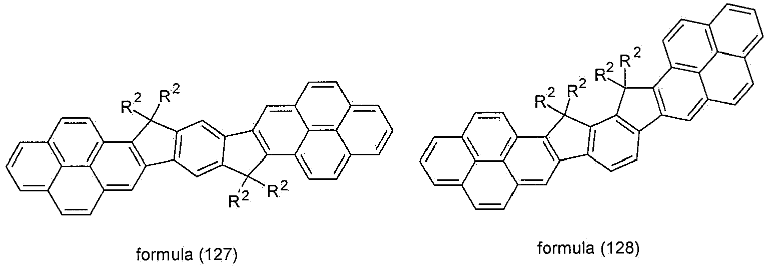

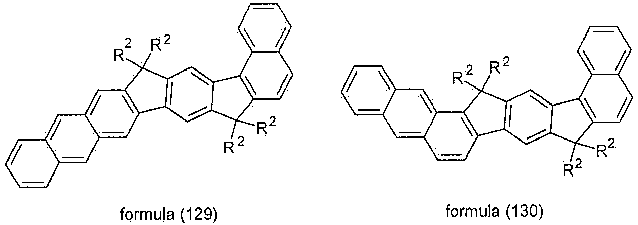

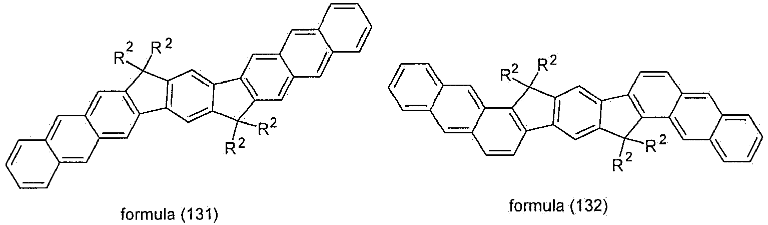

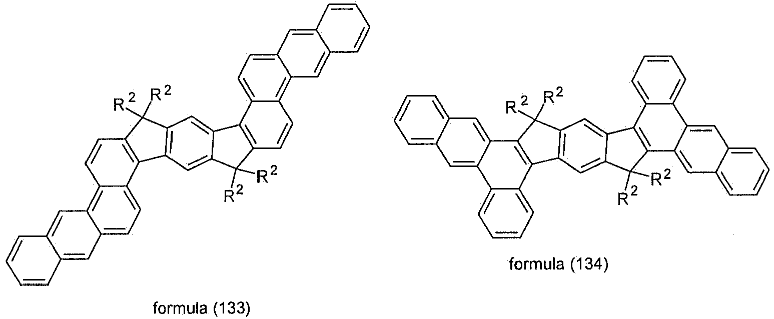

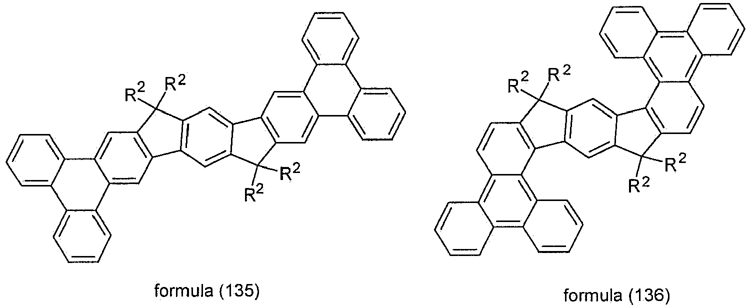

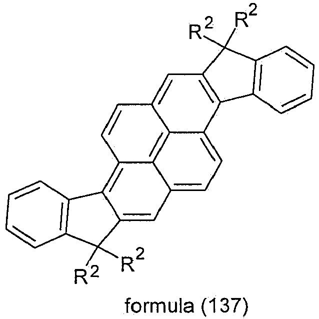

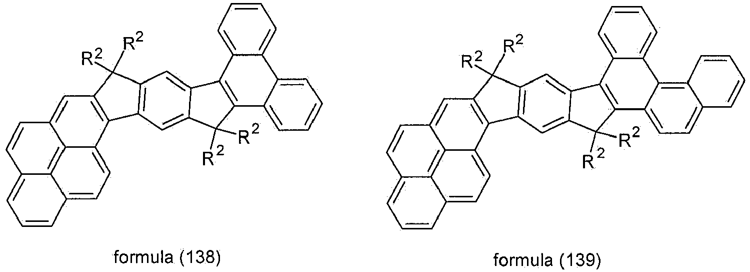

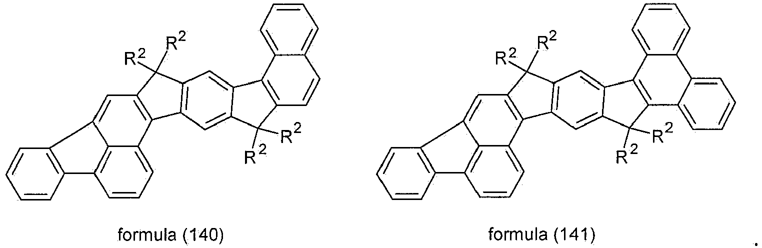

7. Oligomer, polymer or dendrimer comprising one or more compounds according to one or more of Claims 1 to 6, where at least one of the radicals R¹ and/or R² represents a bond to the oligomer, polymer or dendrimer.

8. Process for the preparation of the compounds according to one or more of Claims 1 to 6, comprising the reaction steps:
a) coupling of suitably substituted Ar¹, Ar² and Ar³, where suitable substituents can be, for example, carboxylate groups; and
b) cyclisation of the substituents for introduction of the bridges X.

9. Use of compounds according to one or more of Claims 1 to 7 in electronic devices, in particular in organic electroluminescent devices (OLEDs, PLEDs), organic field-effect transistors (O-FETs), organic thin-film transistors (O-TFTs), organic light-emitting transistors (O-LETs), organic integrated circuits (O-ICs), organic solar cells (O-SCs), organic field-quench devices (O-FQDs), light-emitting electrochemical cells (LECs), organic photoreceptors or organic laser diodes (O-lasers).

10. Organic electronic device, in particular selected from organic electroluminescent devices (OLEDs, PLEDs), organic field-effect transistors (O-FETs), organic thin-film transistors (O-TFTs), organic light-emitting transistors (O-LETs), organic integrated circuits (O-ICs), organic solar cells (O-SCs), organic field-quench devices (O-FQDs), light-emitting electrochemical cells (LECs), organic photoreceptors or organic laser diodes (O-lasers), **characterised in that** at least one organic layer comprises at least one compound according to one or more of Claims 1 to 7.

11. Organic electroluminescent device according to Claim 10, **characterised in that** the compound according to one or more of Claims 1 to 7 is employed as emitting material or as host material in an emitting layer or as hole-transport or hole-injection material in a hole-transport or hole-injection layer or as electron-transport material in an electron-transport layer.

12. Organic electroluminescent device according to Claim 10 or 11, **characterised in that** the compound according to one or more of Claims 1 to 7 is employed as emitting material in an emitting layer in combination with a host material and that the host material is selected from the group consisting of oligoarylenes, oligoarylenes containing condensed aromatic groups, anthracenes, oligoarylenevinylenes, polypodal metal complexes, hole-conducting compounds, electron-conducting compounds, ketones, phosphine oxides, sulfoxides, boronic acid derivatives, benzanthracenes or compounds of the formula (138),
Ar⁴-(Ar⁵)ₚ-Ar⁶ formula (138)
where Ar⁴, Ar⁵, Ar⁶ are on each occurrence, identically or differently, an aryl or heteroaryl group having 5 to 30 aromatic ring atoms, which may be substituted by one or more radicals R¹, and R¹ has the same meaning as described in Claim 1 and p can be equal to 1, 2 or 3; the sum of the π electrons in Ar⁴, Ar⁵ and Ar⁶ is at least 30 if p = 1 and is at least 36 if p = 2 and is at least 42 if p = 3.

13. Solution or formulation comprising at least one compound according to one or more of Claims 1 to 7 and at least one organic solvent.

## Revendications

1. Composé de la formule (1): 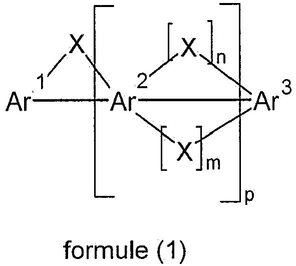 dans laquelle ce qui suit s'applique aux symboles et aux indices qui sont utilisés :
Ar¹, Ar², Ar³ sont pour chaque occurrence, de manière identique ou différente, un groupe aryle ou hétéroaryle qui comporte de 5 à 22 atomes de cycle aromatique, lequel est sélectionné parmi benzène, naphtalène, anthracène, phénanthrène, fluor-anthène, naphtacène, benzanthracène, chrysène, pyrène, benzofluoranthène, triphénylène, pérylène, dibenzanthracène, benzopyrène, picène, pentacène, pentaphène, pyridine, pyrazine, pyrimidine, pyridazine, quinoline, isoquinoline, phénanthroline et acridine, lequel peut être substitué par un radical ou par plusieurs radicaux R¹, étant entendu que Ar² ne représente ni anthracène, ni naphtacène, ni pentacène ;
X est pour chaque occurrence, de manière identique ou différente, un groupe qui est sélectionné parmi C(R²)₂;
R¹ est pour chaque occurrence, de manière identique ou différente, un groupe qui est sélectionné parmi H, D, F, des groupes alkyle ou alcoxy en chaîne droite qui comportent de 1 à 10 atome(s) de C ou des groupes alkyle ou alcoxy qui comportent de 3 à 10 atomes de C, lesquels peuvent dans chaque cas être substitués par un radical ou par plusieurs radicaux R³, où dans chaque cas, un ou plusieurs groupe(s) CH₂ non adjacents peut/peuvent être remplacé(s) par R³C=CR³ ou O et où un ou plusieurs atome(s) de H peut/ peuvent être remplacé(s) par F, ou des systèmes de cycle aromatique ou hétéroaromatique qui comportent de 5 à 24 atomes de cycle aromatique ; deux substituants R¹ ou plus peuvent également former l'un avec l'autre ou les uns avec les autres un système de cycle aliphatique ou aromatique mono- ou polycyclique ;
R² est pour chaque occurrence, de manière identique ou différente, un groupe qui est sélectionné parmi H, des groupes alkyle en chaîne droite qui comportent de 1 à 10 atome(s) de C ou des groupes alkyle ramifiés ou cycliques qui comportent de 3 à 10 atomes de C, où dans chaque cas, un ou plusieurs groupe(s) CH₂ non adjacents peut/peuvent être remplacé(s) par -R²C=CR²- ou -O- et où un ou plusieurs atome(s) de H peut/peuvent être remplacé(s) par F, ou un groupe aryle ou hétéroaryle monovalent qui comportent de 5 à 16 atomes de cycle aromatique, lequel peut être substitué par un radical ou par plusieurs radicaux non aromatique(s) R²; deux radicaux R² qui sont liés au même groupe X peuvent également former un système de cycle l'un avec l'autre ;
R³ est pour chaque occurrence, de manière identique ou différente, H ou un radical hydrocarbone aliphatique ou aromatique qui comporte de 1 à 20 atome(s) de C ;
m, n sont 0 ou 1, étant entendu que m + n = 1 ;
p est 1 ;
Ar¹, Ar² et X forment en association un cycle à cinq éléments ou à six éléments, et Ar², Ar³ et X forment en association un cycle à cinq éléments ou à six éléments, étant entendu que soit tous les symboles X dans le composé de la formule (1) sont liés dans un cycle à cinq éléments, soit tous les symboles X dans le composé de la formule (1) sont liés dans un cycle à six éléments ;
**caractérisé en ce que** la somme de tous les électrons π dans les groupes Ar¹, Ar² et Ar³ est d'au moins 28 ;
les composés qui suivent sont exclus de l'invention :

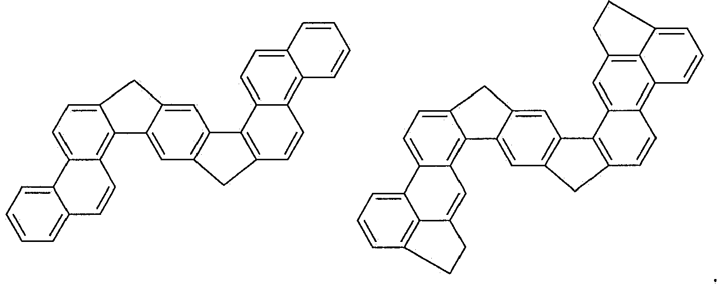

2. Composé selon la revendication 1, **caractérisé en ce que** la somme de tous les électrons π dans les groupes Ar¹, Ar² et Ar³ est entre 28 et 50, de préférence entre 28 et 46, de façon particulièrement préférable entre 28 et 42, en particulier entre 28 et 36.

3. Composé selon la revendication 1 ou 2, **caractérisé en ce que** les groupes Ar¹ et Ar³ sont sélectionnés, de manière indépendante l'un de l'autre, parmi les groupes des formules (2) à (85), lesquels peuvent dans chaque cas être substitués par un radical ou par plusieurs radicaux R¹ et où le symbole * représente la position de la liaison depuis Ar¹ ou Ar³ jusqu'à Ar² et le symbole # représente la position de la liaison depuis Ar¹ ou Ar³ jusqu'à X : 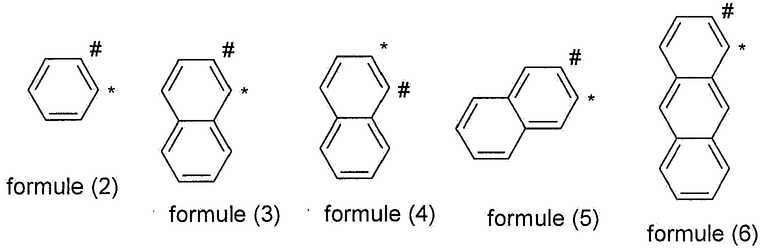 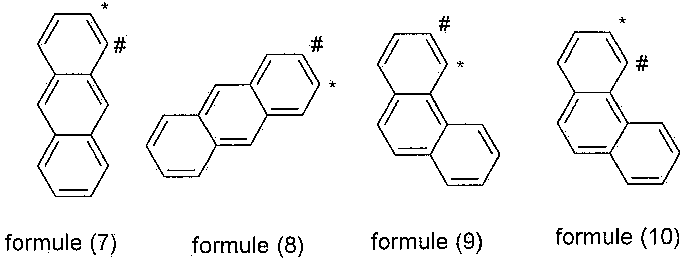 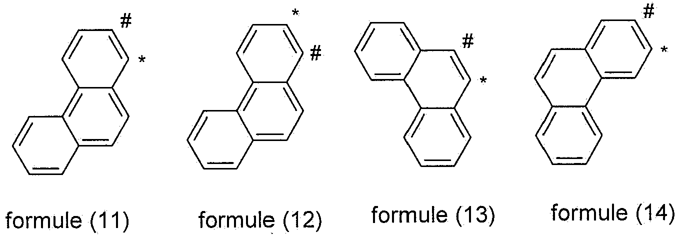 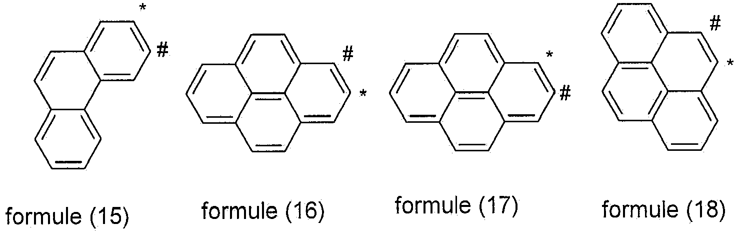 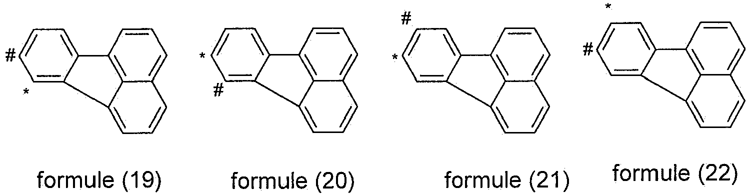 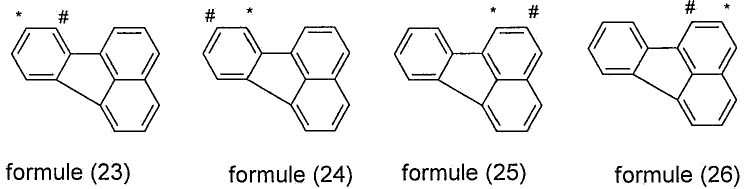 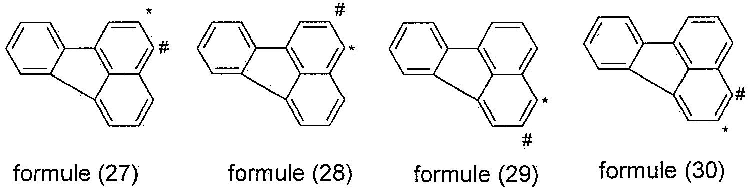 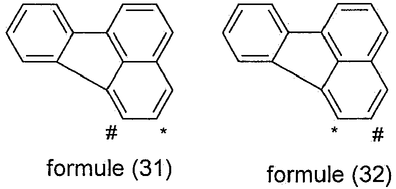 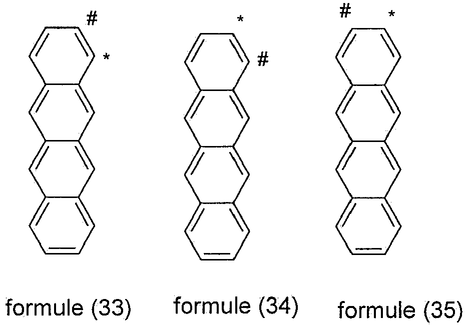 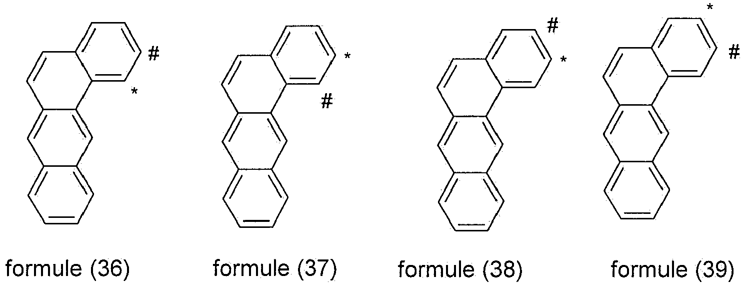 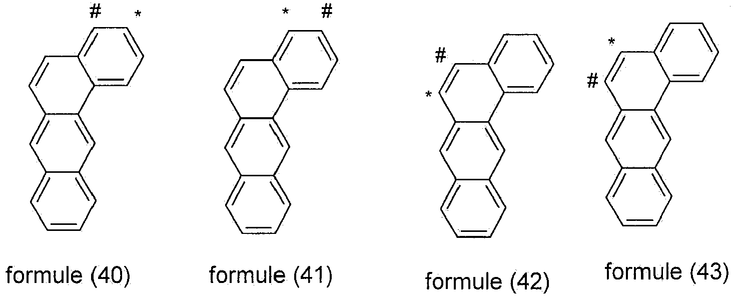 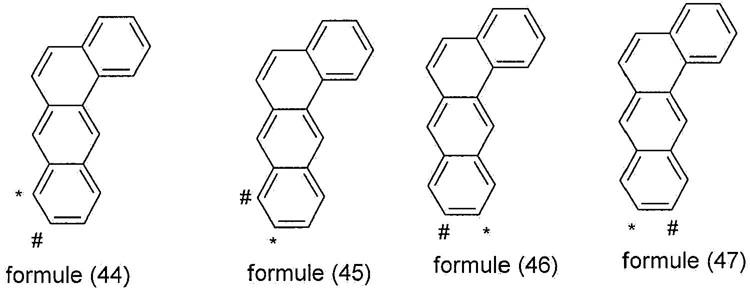 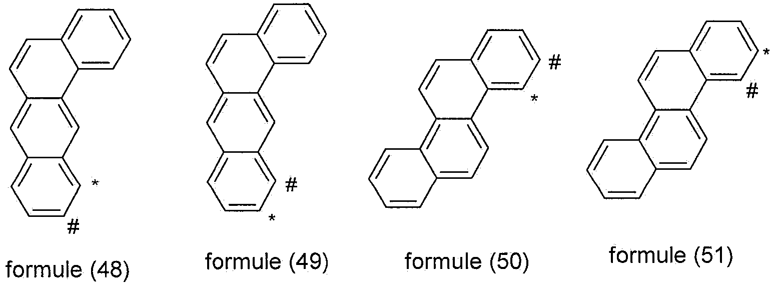 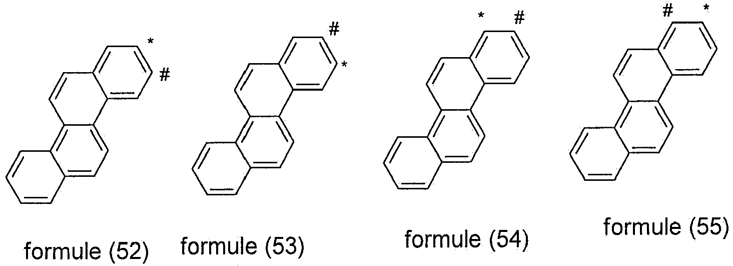 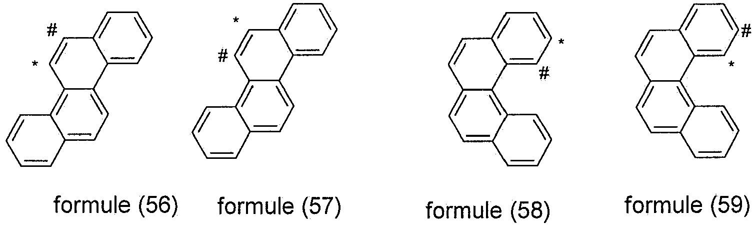 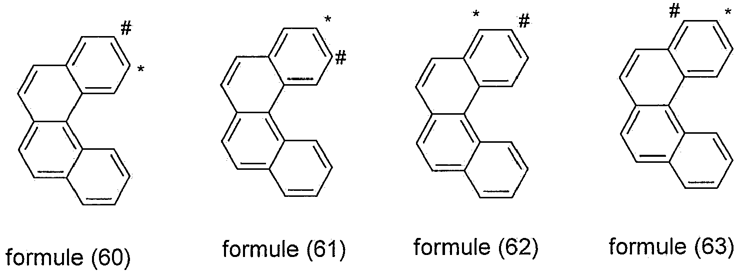 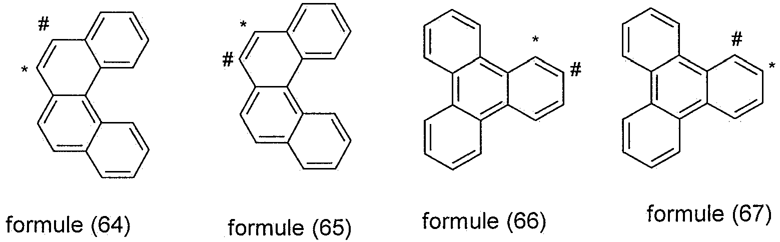 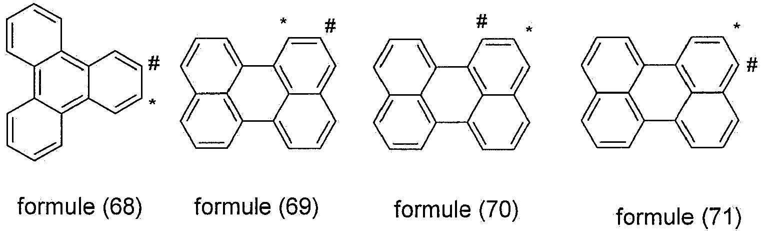 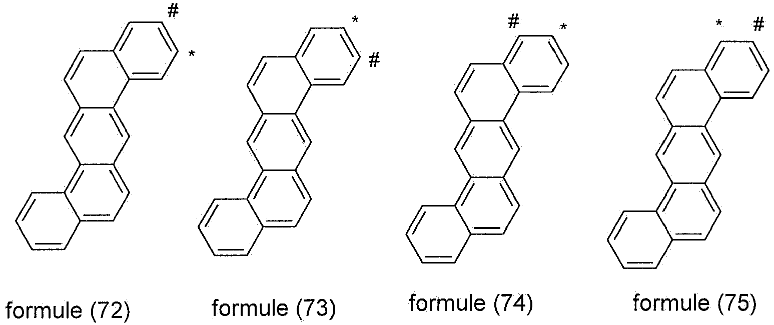 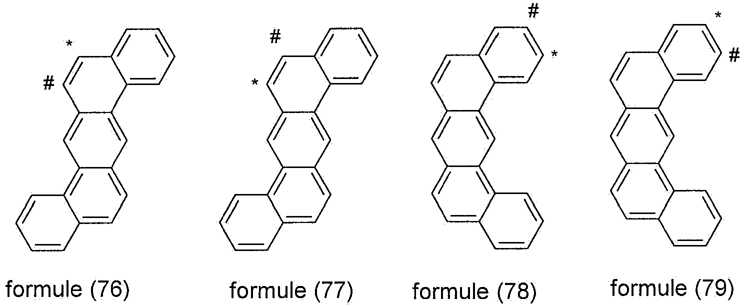 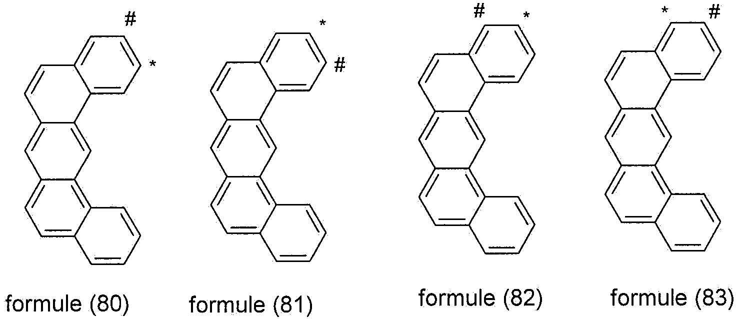 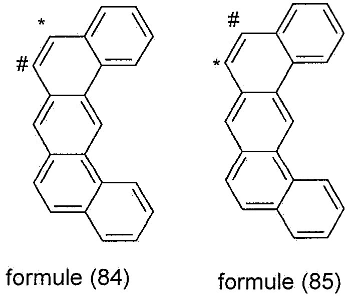 ou depuis des groupes correspondants qui forment un cycle à six éléments en association avec Ar².

4. Composé selon une ou plusieurs des revendications 1 à 3, **caractérisé en ce que** Ar² est sélectionné, de manière identique ou différente pour chaque occurrence, parmi les groupes des formules (86) à (106), lesquels peuvent dans chaque cas être substitués par un radical ou par plusieurs radicaux R¹ et où le symbole * représente la position de la liaison qui va de Ar² jusqu'à Ar¹ ou jusqu'à Ar³ et le symbole # représente la position de la liaison qui va de Ar² jusqu'à X : 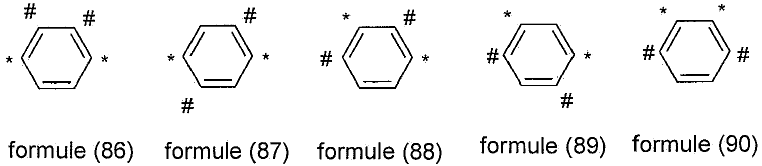 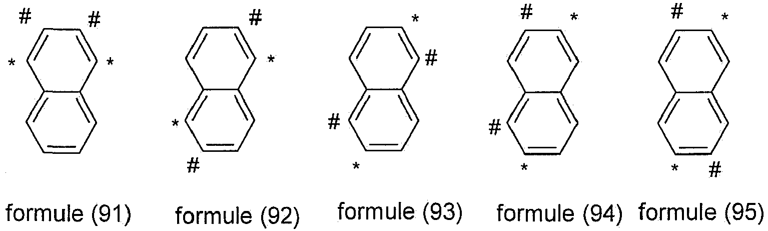 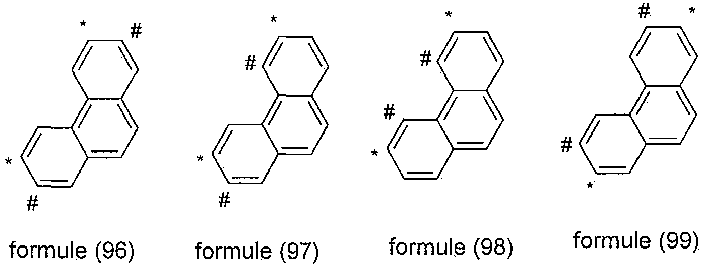 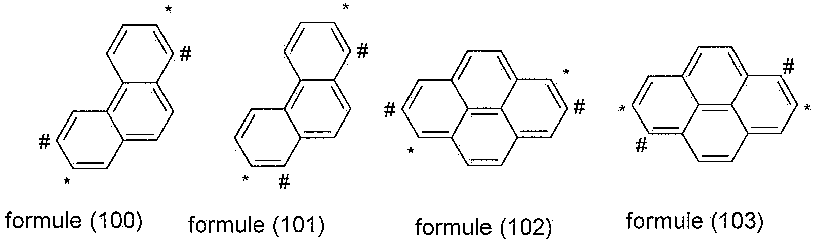 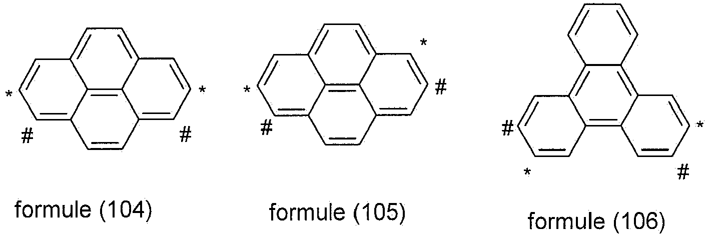 ou depuis des groupes correspondants qui forment un cycle à six éléments en association avec Ar¹ ou Ar³.

5. Composé selon une ou plusieurs des revendications 1 à 4, **caractérisé en ce qu'**au moins l'un des groupes Ar¹, Ar² et Ar³ comporte au moins 4 cycles condensés, i.e. au moins 16 électrons π.

6. Composé selon une ou plusieurs des revendications 1 à 5, **caractérisé en ce que** les composés sont sélectionnés parmi les formules (111) à (141), où les systèmes aromatiques peuvent dans chaque cas également être substitués par un radical ou par plusieurs radi caux R¹:

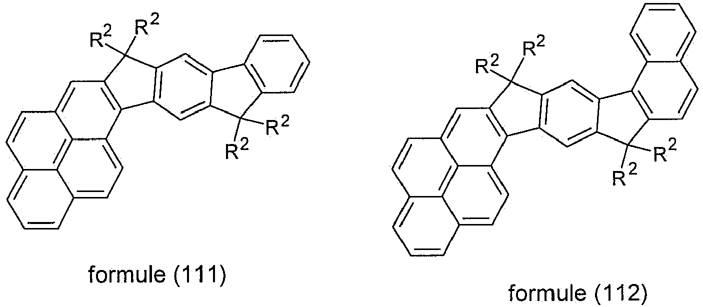

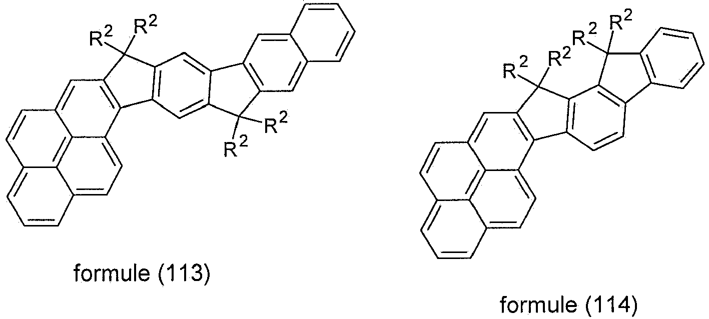

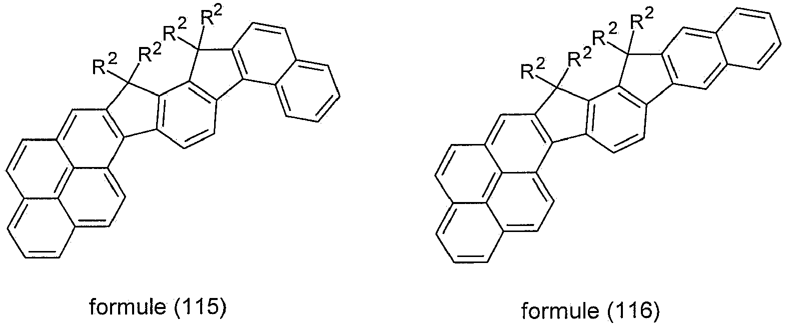

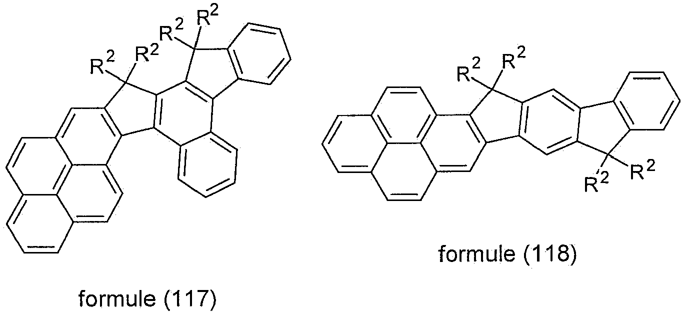

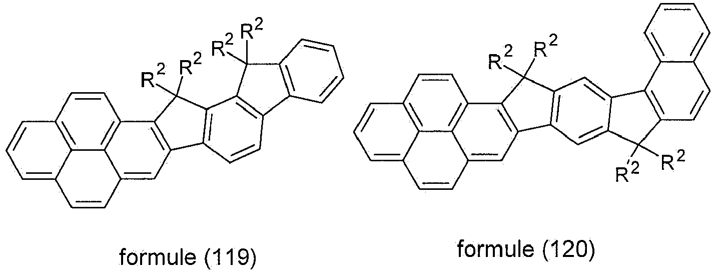

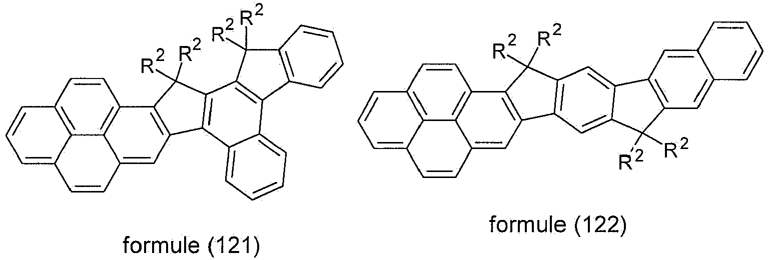

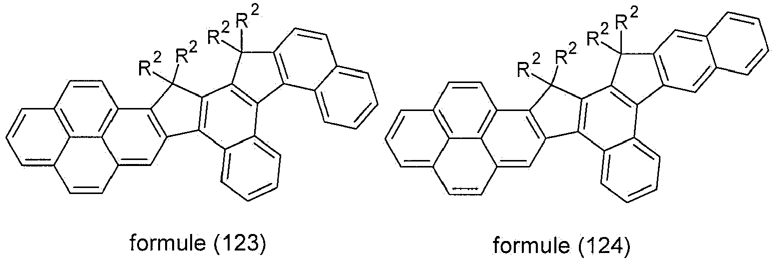

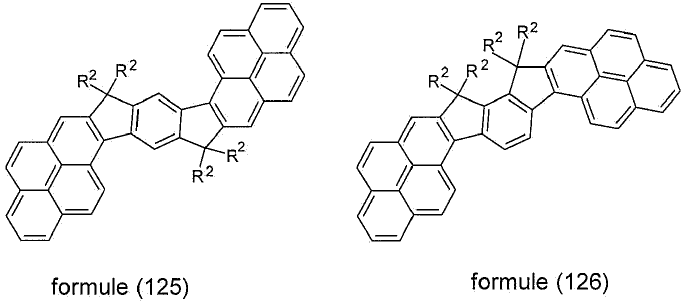

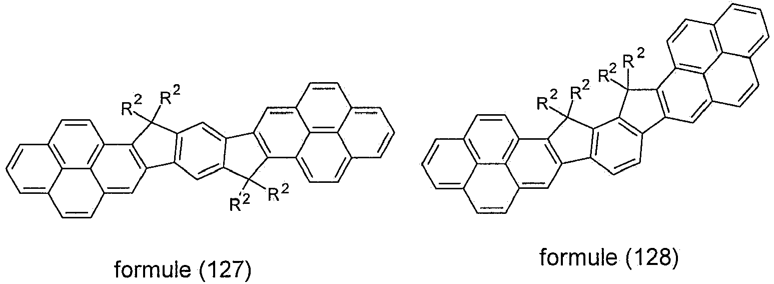

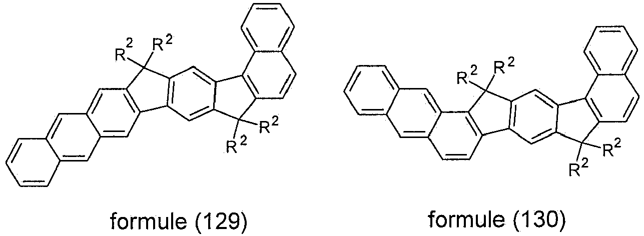

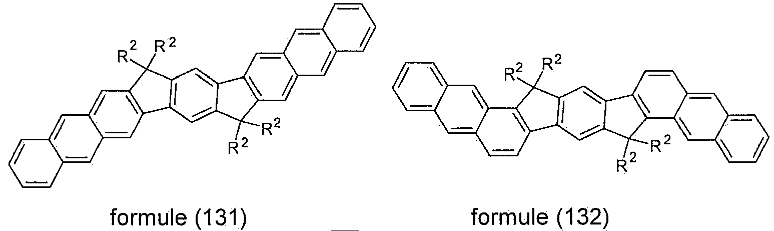

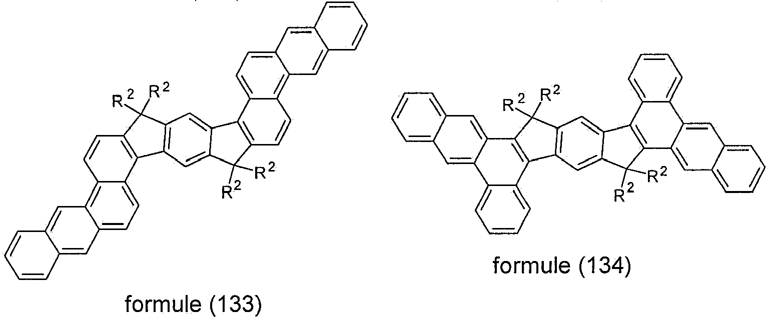

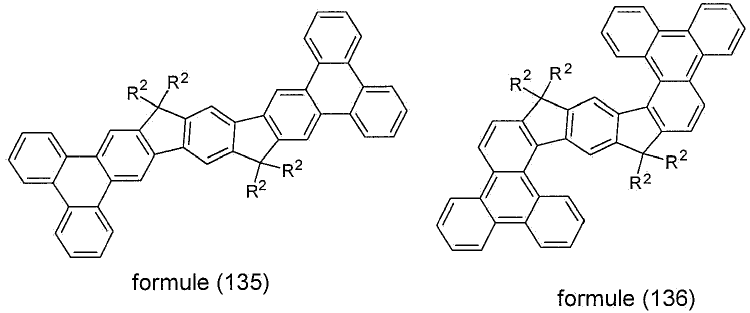

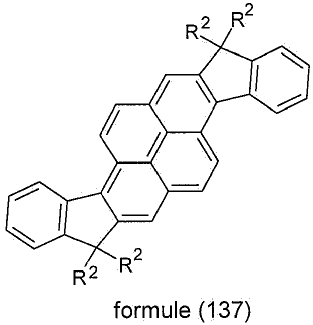

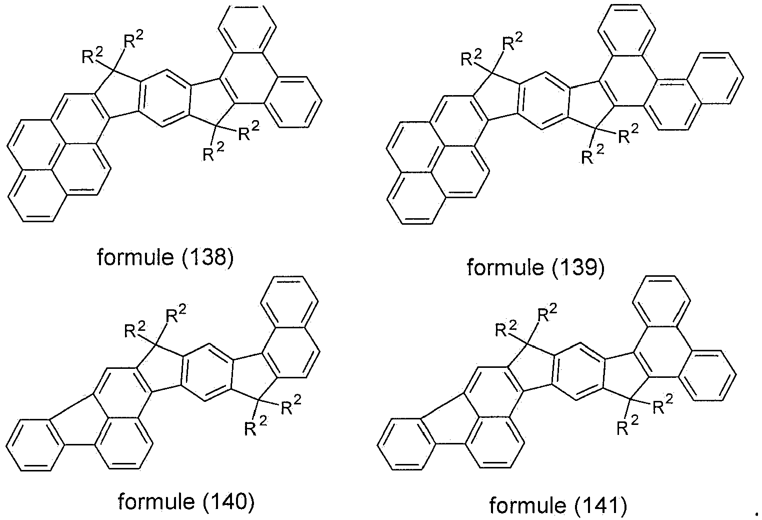

7. Oligomère, polymère ou dendrimère comprenant un ou plusieurs composé(s) selon une ou plusieurs des revendications 1 à 6, où au moins l'un des radicaux R¹ et/ou R² représente une liaison sur le l'oligomère, le polymère ou le dendrimère.

8. Procédé pour la préparation des composés selon une ou plusieurs des revendications 1 à 6, comprenant les étapes de réaction qui suivent :
a) le couplage de Ar¹, Ar² et Ar³ substitués de façon appropriée, où les substituants appropriés peuvent être, par exemple, des groupes carboxylate ; et
b) la cyclisation des substituants pour l'introduction des X.

9. Utilisation d'un composé selon une ou plusieurs des revendications 1 à 7 dans des dispositifs électroniques, en particulier dans des dispositifs électroluminescents organiques (OLED, PLED), des transistors à effet de champ organiques (O-FET), des transistors à film mince organiques (O-TFT), des transistors à émission de lumière organiques (O-LET), des circuits intégrés organiques (O-IC), des cellules solaires organiques (O-SC), des dispositifs à extinction de champ organiques (O-FQD), des cellules électrochimiques à émission de lumière (LEC), des photorécepteurs organiques ou des diodes laser organiques (O-laser).

10. Dispositif électronique organique, en particulier sélectionné parmi les dispositifs électroluminescents organiques (OLED, PLED), les transistors à effet de champ organiques (O-FET), les transistors à film mince organiques (O-TFT), les transistors à émission de lumière organiques (O-LET), les circuits intégrés organiques (O-IC), les cellules solaires organiques (O-SC), les dispositifs à extinction de champ organiques (O-FQD), les cellules électrochimiques à émission de lumière (LEC), les photorécepteurs organiques ou les diodes laser organiques (O-laser), **caractérisé en ce qu'**au moins une couche organique comprend au moins un composé selon une ou plusieurs des revendications 1 à 7.

11. Dispositif électroluminescent organique selon la revendication 10, **caractérisé en ce que** le composé selon une ou plusieurs des revendications 1 à 7 est utilisé en tant que matériau d'émission ou en tant que matériau hôte dans une couche d'émission ou en tant que matériau de transport de trous ou d'injection de trous dans une couche de transport de trous ou d'injection de trous ou en tant que matériau de transport d'électrons dans une couche de transport d'électrons.

12. Dispositif électroluminescent organique selon la revendication 10 ou 11, **caractérisé en ce que** le composé selon une ou plusieurs des revendications 1 à 7 est utilisé en tant que matériau d'émission dans une couche d'émission en combinaison avec un matériau hôte et **en ce que** le matériau hôte est sélectionné parmi le groupe qui est constitué par les oligoarylènes, les oligoarylènes qui contiennent des groupes aromatiques condensés, les anthracènes, les oligoarylène-vinylènes, les complexes de métaux polypodaux, les composés de conduction de trous, les composés de conduction d'électrons, les cétones, les oxydes de phosphine, les sulfoxydes, les dérivés d'acide boronique, les benzanthracènes ou les composés de la formule (138) :
Ar⁴-(Ar⁵)ₚ-Ar⁶ formule (138)
dans laquelle Ar⁴, Ar⁵, Ar⁶ sont pour chaque occurrence, de manière identique ou différente, un groupe aryle ou hétéroaryle qui comporte de 5 à 30 atomes de cycle aromatique, lequel peut être substitué par un radical ou par plusieurs radicaux R¹, et R¹ présente la même signification que celle qui a été décrite selon la revendication 1 et p peut être égal à 1, 2 ou 3 ; la somme des électrons π dans Ar⁴, Ar⁵ et Ar⁶ est d'au moins 30 si p = 1 et est d'au moins 36 si p = 2 et est d'au moins 42 si p = 3.

13. Solution ou formulation comprenant au moins un composé selon une ou plusieurs des revendications 1 à 7 et au moins un solvant organique.
